# EUROPEAN PATENT SPECIFICATION

(11) **EP 0 274 431 B1**
(45) Date of publication and mention of the grant of the patent: **04.05.1994**
(21) Application number: 88300085.3
(22) Date of filing: 07.01.1988
(51) Int. Cl.: A61K 9/50

(54) **Drug delivery compositions and methods**
Mittel zur Arzneistoffabgabe und Verfahren zu deren Herstellung
Compositions pour la libération d'un médicament et leurs procédés de fabrication

(30) Priority: 08.01.1987 US 1814; 26.03.1987 US 31237; 26.05.1987 US 54193; 26.05.1987 US 54194; 15.12.1987 US 130550
(43) Date of publication of application: 13.07.1988
(73) Proprietor: QUIXOTE CORPORATION, Chicago, IL 60601 (US)
(72) Inventor: Ecanow, Bernard, Wilmette Illinois 60091 (US)
(74) Representative: Wain, Christopher Paul

(56) References cited:
- EP-A- 0 083 469
- WO-A-85/05029
- WO-A-85/05035

## Description

The present invention relates to a stable drug delivery composition in oral dosage form or capable of new and unexpected stabilization, solubilization and delivery of stable drug components in parenteral form at the pH of body tissue. More particularly, the present invention is directed to a drug delivery system or composition that allows for rapid dissolution and smooth liberation of any desired drug either orally or parenterally without precipitation, and the method of preparing the drug composition. In accordance with one embodiment of the present invention, liposomes and their contents can be enveloped in a coacervate-based film to prevent disintegration of liposome vehicles.

Drugs now in common use often require formulation compromises in order to prepare the marketed product. Thus, many parenteral compositions must be prepared using the salt of the parent compound and an excessive pH.

The instability of many useful drugs and other useful medical compositions poses other formulation problems. At present, emulsions, microemulsions and liposomes constitute the principal approaches to these problems. While such dosage forms are an advance over older forms, they are often associated with erratic bioavailability and instability of their own.

The herein disclosed invention comprises a method to prepare compositions and compositions which deliver medically useful compositions effectively. The method is based upon the use of a non-toxic aqueous coacervate; said method produces stable microemulsions comprised of particles in which one or more pharmaceutical components have been incorporated. Through the process of this invention particles are enveloped by a coacervate-based film. The compositions of this invention may be administered orally, parenterally or by tissue absorption, as required.

The disclosed method enables the preparation of particles of any desired size and any degree of particle surface film hardness, any number of coacervate-derived films of any desired thickness, or any combination of particle sizes and surface hardnesses. In preferred compositions prepared by the disclosed invention, the particle size is 1 µm (micron) or less. The pharmaceutical component(s) may be any water soluble or water insoluble medically useful composition or combinations of such compositions.

The inventor has previously disclosed compositions based on a coacervate system using albumin and lecithin as principal components of the system. However, by polymerizing one or both of these components, the inventor has not only simplified the method but unexpectedly produced a preparation with vastly improved encapsulating capability. Details of this improvement are presented in the Experiments Section.

While there are apparent similarities to the inventor's prior art, this application differs significantly in several important aspects. Thus, encapsulation is a fundamental aspect of the inventor's prior art and the present invention. However, by polymerizing one or more of the surfactants used in the present invention, and/or by modifying the previously disclosed processes, the proportion of the pharmaceutical component incorporated in the present formulation is approximately two to six times greater than that of formulations disclosed earlier.

The intravenous delivery of many pharmaceutical preparations has been limited to the use of aqueous singular systems, as discussed, based upon the salt of a parent compound and/or the dissolution of the compound by agents such as a propylene glycol or alcohol. Other known systems which function primarily as blood substitutes, however, are able to solubilize and deliver drugs infused therein to a restricted degree. U.S. Patent No. 4,343,797 discloses a method of preparing a synthetic blood substitute based upon a two-phase heterogeneous physico-chemical system which provides for oxygen transport and other physiological functions inherent to whole natural blood. In U.S. Patent No. 4,439,424 a preferred synthetic blood composition comprises albumin, water, sodium chloride, urea and lecithin in the form of a two-phase coacervate system which is rendered isotonic with human blood by the addition of controlled amounts of the sodium chloride. U.S. Patent Nos. 4,558,032, 4,539,204 and 4,596,778 relate to gelatin based synthetic blood substitutes based upon a two-phase coacervate system utilizing respectively, gelatin and acacia; two gelatins; modified fluid gelatins of different isoelectric points; and gelatin or modified gelatin and lecithin. U.S. Patent No. 4,547,490 discloses a further coacervate synthetic blood derived from an aqueous solution containing albumin, sodium chloride and lecithin in a nonpolar or semipolar solvent.

These known products have been found to be capable of carrying drugs, but have been designed to serve as a resuscitative fluid much in the manner of whole natural blood. One of the principal formulation problems successfully addressed by the aforementioned patents is maintenance of the structure and integrity of the microparticles (synthocytes) which comprise the composition.

The hemoglobin component of this composition (i.e., stroma-free hemoglobin, pyridoxilated-polymerized hemoglobin, liposome encapsulated hemoglobin and the like) remains functional and encapsulated within the microparticles (synthocytes) until the particles are metabolized and eliminated from the body. Encapsulation of the hemoglobin component not only preserves functionality but reduces or eliminates endotoxic reactions associated with non-encapsulated hemoglobin. The term "synthocyte" as used in this specification refers to the microparticles of this invention, the particles being 1 µm (micron) or less in any single dimension when administered parenterally, and comprised of a coacervate-based matrix which contains the active component(s); and a coacervate-based film which envelopes the contents of the microparticle and constitutes the outer surface of the particle. The enveloping film may be comprised of one or more layers as required to prepare the desired composition. In this specification the terms "particle", "synthocyte" and "microparticle" will be used interchangeably.

Another example of a coacervate-based system is disclosed in International application PCT/US85/00859, published November 21, 1985, relating to an oral dosage composition and method of preparation to enable the oral administration of insulin. The problems addressed by the disclosed coacervate system, however, include the need for protection of the insulin against degradation by enzymes, and factors such as the acid-base balance and other gastrointestinal conditions and processes. The coacervate-based film envelopes each individual insulin molecule to inhibit the interaction between the insulin component and the degrading conditions.

The prior art describing the liposome technology includes the following: The Ash and Hider U.S. Patent No. 4,448,765 filed July 3, 1979 entitled LIPOSOMES AND THEIR USE IN TREATING HUMAN OR OTHER MAMMALIAN PATIENTS, describes microvesicles which are reportedly stabilized by the incorporation of a polymer having at least six atoms attached to the backbone thereof; said vesicles incorporating a physioplogically-active substance. Kelly U.S. Patent No. 4,356,167 entitled LIPOSOME DRUG DELIVERY SYSTEMS, filed June 22, 1981, describes a liposome medicament delivery system wherein the medicament is in an aliphatic liquid-sterol-water lamellae. The lipid may be a sodium or potassium salt of a C₄ to C₁₈ fatty acid and the sterol may be cholesterol. Kao, Y. and Loo, T., Pharmacological Disposition of Negatively Charged Phospholipid Vesicles in Rats, J. of Pharm. Sci. 59;11, 1980, pp. 1338-1343 contains a report of experiments which investigated the pharmacological disposition of four negatively charged phospholipid vesicles. Gregoriadis, G., the Carrier Potential of Liposomes in Biology and Medicine; N.E. J. of Med. Vol. 295, No. 13, 1976, describes the use of liposomes to carry a large variety of biologically interesting compositions including hormones, drugs, steroids, vitamins, viruses and other compositions such as histamine. Doucet, D., et al; Oxygen Binding of Artificial Erythrocytes, in Proceedings of Int. Soc. of Artif. Organs, 5, (Supp) 1981, pp. 392-395, describes the use of liposomes to incorporate a concentrated hemoglobin solution. Gaber, B., et al; Encapsulation of Hemoglobin in Phospholipid Vesicles, FEBS Letters, Vol. 153, 2, 1983, pp. 285-287, describes a method to encapsulate hemoglobin in phospholipid vesicles. Gruner, S., Novel Multilayered Lipid Vesicles: Comparison of Physical Characteristics of Multilamellar Liposomes and Stable Plurilamellar Vesicles, Biochemistry, 24, 1985, 2833-2842, discusses the effects of osmotic compression on the production of various liposome compositions; Szebeni, J. et al.; Encapsulation of Hemoglobin in Phospholipid Liposome; Characterization and Stability; Biochemistry; 24, 1985, 2827-2832, reports the encapsulation of hemoglobin in liposomes of different lipid composition. Blood Policy and Technology; Office of Technology Assessment, U.S. Congress, U.S. Government Printing Office, 1985, pp. 146-151, reports the methods of formulation and the difficulties encountered in using liposome based blood substitutes.

It is important to emphasize that liposomes differ from the coacervate preparations previously discussed and herein disclosed in mode of manufacture, and in physical and structural properties.

Coacervates are used in the preparation of the compositions of this invention.

The Ecanow et al United States Patent No. 4738952, filed December 20, 1985, describes a synthetic whole blood wherein the coacervate system is produced from lecithin dispersed in an aqueous solution containing albumin and sodium chloride; said system enabling a more effective use of the incorporated pyridoxilated polymerized hemoglobin.

The Ecanow United States Patent No. 4849405, describes an oral dosage form of insulin based on the use of a coacervate system comprised of lecithin, albumin, and insulin.

The Ecanow United States Patent No. 4794000, describes an oral drug delivery system based on a dextranpolyethylene glycol coacervate.

There are fundamental differences between this invention and the inventor's prior art. In some instances, e.g., the blood substitute formulations, are based on precisely opposite theoretical and functional characteristics. Thus, the blood substitute formulations are based squarely on a gas exchange system. In this instance, oxygen is absorbed by the hemoglobin component of the synthocyte and released from the hemoglobin to the tissues according to oxygen tensions. This phenomena is represented in whole blood and some blood substitutes by a sigmoid curve. In contrast, in this invention, the drug is released from encapsulation; the release can be represented by a straight line function or by an exponential rate of release.

Further, the inventor's blood substitutes are specifically formulated to maintain the hemoglobin component in an encapsulated state up to the time the synthocyte and its contents are metabolized. In the present invention, the formulations are designed to release the drug from the synthocyte promptly after administration. The present invention provides for a variety of routes of administration; the inventor's blood substitutes are restricted to intravenous injection.

### SUMMARY OF THE INVENTION

The method of this invention enables the preparation of carrier vehicles useful for the delivery of medically useful compositions for man and animals. The finished products of this invention range from a red blood cell substitute (resuscitation fluid) to aqueous based formulations of water insoluble, water soluble and water sensitive drugs.

The fact that in the method of this invention, aqueous formulations successfully incorporate and deliver water insoluble and water sensitive drugs is evidence that this invention constitutes a significant advance in the state of the art.

The aqueous based compositions of this invention comprise one or more of a large variety of surface active agents, that is, molecules which are non toxic and endogenous or exogenous to the body and can include one or more monomeric, polymerized or polymerizable surface active agents, particularly a polymerized form of a protein such as albumin and/or a polymerized form of a phospholipid such as lecithin and may include one or more surface active agents either in polymeric or monomeric form, including lecithin, albumin, gelatin, modified liquid gelatin, acacia gel and combinations thereof. Finished products of this invention may be administered by oral, parenteral, transdermal, transmucosal or inhalation routes, or by combinations of these routes.

Formulations prepared according to the method of this invention are stable, pharmacologically-active and will not precipitate in body tissue or fluid. Parenteral drugs now prepared to an excessive pH can be reformulated using the method of this invention. The resulting product will have improved bioavailability and absorption; said reformulation will reduce or eliminate pain and tissue damage associated with injection of drugs with an excessive pH. The physiologically-active compounds, such as a drug, may be in a concentrated form, or may be dissolved or suspended in a carrier, e.g., a liquid carrier, such as an oil, glyceride, lipid, coacervate phase(s) or within a film carrier such as a liposome. In one embodiment, a liposome is encapsulated in a coacervate-based matrix or coacervate phase and/or coacervate-based film to prevent the liposome from unravelling prematurely.

Either prompt or sustained release characteristics or mixtures thereof, can be manufactured by the method of the present invention. into the circulatory system without being absorbed through the lining of the GI tract. The formulations are stable, pharmacologically active and will not precipitate in body tissue or fluid. The drug delivery formulations of this invention include emulsions, microemulsions and coacervate phase encased liposomes. In those delivery formulations which are prepared to an excessive pH and are administered parenterally, the herein disclosed formulations will improve dissolution, be free of the pharmacokinetic distortions associated with current drug vehicles and reduce or eliminate pain and tissue damage. Use of this invention for orally administered drugs or other medically useful compositions significantly improves their bioavailability and absorption. In brief, this invention comprises a coacervate system containing at least one monomeric or polymerized surface active compound which can be processed to result in a composition including one or more physiologically-active compounds coated or encased with a coacervate phase film and may be in the form of a microemulsion to stabilize and improve the dosage forms specified above. The physiologically-active compounds, such as a drug, may be in a concentrated form, or may be dissolved or suspended in a carrier, e.g., a liquid carrier, such as an oil or glyceride, or within a film carrier such as a liposome. In one embodiment, a liposome is encapsulated in a coacervate based matrix or coacervate phase and/or coacervate based film to prevent the liposome from unravelling.

The method of this invention enables the preparation of carrier vehicles useful for the delivery of medically useful compositions for man and animals. Thus, the disclosed method can be used to produce a composition which comprises a red blood cell substitute (resuscitation fluid); if desired, by modifying the method, it will produce an effective plasma expander. The method of the present invention using a polymerized or polymerizable surface active agent constitutes an improvement in incorporating ability ranging from two to six times greater than that of the inventor's previously disclosed methods.

The delivery systems of this invention encompass emulsions, microemulsions, encapsulated liposomes, suspensions, gels and microsuspensions. The size of the coacervate-based encapsulated particles which comprise the finished products of this invention extend from the nanogram range to about one micron when administered parenterally. Larger size particles may be made by the disclosed method if required. Filtering procedures may be used to insure appropriate particle size. This invention envisages compositions in which particles 1 µm (micron) or less are mixed with particles 2 µm (microns) or more.

In this invention, the pharmacologically-active component is bound to or embedded in the coacervate matrix comprising the pharmacologically-active compounds held in a coacervate matrix of the equilibrium water phase and/or the coacervate phase forming a particle; and the particle, including the pharmacologically-active component and equilibrium water and/or coacervate phase (forming the coacervate matrix) is then encapsulated by a coacervate-based film. The coacervate-based film can contain one or more pharmacologically-active component(s), and layered films can contain no, or different, pharmacologically-active components. The film can be prepared to any degree of structure (hardness) ranging from soft (semi-gel like) to rigid. The method provides for a mixture of particle sizes if desired, and for any degree of surface film hardness, any number of surface films with or without an active compound, or combinations thereof.

In accordance with one embodiment of the present invention, a perhalogenated compound and a two-phase aqueous coacervate system are combined to yield a useful composition that can be used to solubilize and stabilize physiologically-active compounds. The resulting products can be administered orally or parenterally, preferably intravenously, to perform such useful functions as acting as a barrier to food absorption to assist in weight reduction programs; as an oxygen-transporting contrast medium; and/or as a resuscitation fluid. The method and composition of the present invention also can be used to solubilize and stabilize non-polar physiologically-active pharmaceutical compounds, such as drugs, enzymes, biologicals, peptides, antimicrobials, anesthetic agents, or combinations thereof. Surprisingly and unexpectedly, according to the present invention, the anesthetic agents, including gases, liquids and liquifiable gases, can be incorporated into the composition of the present invention. In general, the composition and method of the present invention facilitates the manufacture of medically-useful products and facilitates the introduction of these medically-useful products into the body.

In accordance with another embodiment, the physiologically-active compound is a heme, hemoprotein, and/or a heme-hemoprotein complex, such as stroma-free hemoglobin; polymerized hemoglobin; and/or pyridoxilated, polymerized hemoglobin; and/or an oxygen solvent to augment the oxygen transport capability of the body, to treat for several of the anemias and/or to act as an oxygen-carrying plasma extender.

### OBJECTS OF THE PRESENT INVENTION

Accordingly, an object of the present invention is to provide a composition and a method of making the composition wherein one or more physiologically-active compounds are encapsulated by an aqueous coacervate-based film containing at least one monomeric or polymerized surface active compound to provide unexpected stability to the composition.

Another object of the present invention is to provide a composition and a method of making the composition wherein one or more physiologically-active compounds is dissolved or dispersed in a liquid carrier and the carrier and compound are encased in an aqueous coacervate-based matrix containing at least one monomeric or polymerized surface active compound, the composition including an aqueous colloid-rich phase or an aqueous colloid-poor phase or a combination of both phases from a two-phase coacervate system.

Another object of the present invention is to provide a composition and a method of making the composition wherein one or more physiologically-active compounds is dissolved or dispersed in a liquid carrier, such as a glyceride, and the carrier and compound are encased in an aqueous coacervate-based matrix containing at least one surface active compound including an aqueous colloid-rich phase or an aqueous colloid-poor phase or a combination of both phases from a two-phase coacervate system, thereby preventing the compound from diffusing out of the composition prematurely, and to prevent degradation of the compound by body fluids.

Another object of the present invention is to provide a composition which includes one or more physiologically-active compounds in concentrated form or in a solid or liquid carrier, encased in an aqueous coacervate-based matrix containing at least one surface active compound, particularly a film containing an aqueous colloid-rich phase.

Still another object of the present invention is to provide a composition and a method of making the composition wherein previously encapsulated physiologically-active compounds, such as compounds encapsulated in a liposome, are further stabilized by encapsulation in an aqueous coacervate-based matrix containing at least one surface active compound to prevent the compound from leaking by preventing the liposome from unravelling prematurely.

A further object of the present invention is to provide a composition and a method of making the composition wherein one or more physiologically-active compounds are encapsulated in an aqueous coacervate-based matrix containing at least one polymerized surface active compound, the matrix including a colloid-rich or a colloid-poor phase, or a combination of a colloid-rich and a colloid-poor phase to stabilize the physiologically-active compound and prevent the compound from prematurely leaking out of the coacervate-based film, and to prevent compound-degenerative materials, such as enzymes, or other digestive fluids, from reaching and degenerating the compound.

It is a further object of the present invention to provide a non-toxic, two-phase aqueous coacervate-based composition, including a non-toxic perhalogenated compound, such as a perhalogenated hydrocarbon and/or a perhalogenated amine, to solubilize and stabilize physiologically-active compounds, such as drugs, enzymes, biologicals, peptides, antimicrobials, anesthetic agents, oxygen carriers, contrast media and mixtures thereof, and to facilitate the oral or parenteral introduction of the physiologically-active compound into the body.

Another object of the present invention is to provide a composition and method of making the composition wherein one or more physiologically-active compounds are encapsulated by an aqueous coacervate-based matrix containing a polymer of lecithin to substantially and unexpectedly increase the yield or amount of physiologically-active compound capable of being carried in the composition.

Another object of the present invention is to provide an aqueous coacervate-based composition, incorporating heme, hemoproteins, or a heme-hemoprotein complex, that can be used to increase the oxygen-transport and iron-transport capabilities of the body, to treat various anemias and/or to extend plasma volume.

Another object of the present invention is to provide a coacervate matrix enveloping one or more physiologically-active compounds wherein the coacervate matrix includes a plurality of surface active compounds one of which is polymerized albumin or polymerized lecithin.

Another object of the present invention is to provide a composition and method of making the compound wherein a three-layer coacervate system is formed using a physiologically-acceptable alcohol, and the middle layer, comprising an aqueous colloid-rich phase, is separated from the other two layers and then formed into a second, two-phase coacervate system with one or both phases or layers suitable for encapsulating a physiologically-active compound for oral, tissue-absorptive, or parenteral administration.

Another object of the present invention is to provide a composition and a method of making the composition wherein one or more physiologically-active compounds is dissolved or dispersed in a liquid carrier, such as a glyceride, and the carrier and compound are encased in an aqueous coacervate-based film including an aqueous colloid-rich phase or an aqueous colloid-poor phase or a combination of both phases from a two-phase coacervate system, thereby preventing the compound from diffusing out of the composition prematurely, and to prevent degradation of the compound by body fluids.

The above and other objects and advantages of the present invention will become apparent from the following detailed description of the preferred embodiments.

### DETAILED DESCRIPTION OF THE PREFERRED EMBODIMENTS

The present invention provides for a stable drug delivery composition useful for the oral administration or tissue lining absorption of drugs and other medically useful compositions and for the stabilization, solubilization and delivery of pharmacologically active substances as parenterally administrable drugs at the pH of body tissue. The compositions of the present invention are derived from a non-toxic two-phase aqueous coacervate system forming a matrix containing at least one polymerized surface active compound, one phase of which is colloid-rich, semipolar to nonpolar in character and capable of solubilizing oil-soluble and water-insoluble components; and the second phase of the coacervate composition is colloid-poor, semipolar to polar in character and capable of solubilizing water-soluble and, to a lesser degree, water-insoluble components. The polar phase of a coacervate composition is made of strong, dipolar from 0.1D to 0.8D. The nonpolar phase is made up of molecules have little or no dipole character, generally in the range of 0 to 0.1D. See Remington's Pharmaceutical Sciences, Mack Publishing Company, 1973, pp. 241-242. To a limited degree, however, the colloid-poor phase may be able to solubilize some apparently water-insoluble compounds, the colloid-rich phase being insoluble and in equilibrium with said colloid-poor phase. In this invention, a drug is incorporated in the coacervate phase based matrix and encapsulated comprises a medically useful, surprisingly stable composition suitable for oral, parenteral, transdermal, transmucosal or inhalation administration or combinations of such modes of administration.

As used in this invention the term, "transmucosal" means that a formulation of this invention is applied directly to any of the accessible mucosal membraneous tissues of the body. The term, "transdermal" is used to mean a formulation applied directly to the surface of the skin. "Tissue absorptive" includes both transdermal and transmucosal. The terms, "oral", "parenteral" and "inhalation" are used as per conventional definitions. In this invention, the oral route means that any oral dosage form can be accommodated, i.e., tablets, capsules, liquids, particles, and the like.

The term "drug" is defined by the Federal Food, Drug and Cosmetic Act, and as used in the present disclosure, is "articles recognized in the official United States Pharmacopoeaia, offical Homeopathic Pharmacopoeaia, or official National Formulary or any supplement to any of them". Further clarification of this definition is supported by Remington's Pharmaceutical Sciences, which states "drug" to mean "any article contained in the above-cited references which is used in the process of diagnosis, cure, treatment, mitigation or prevention of disease in man and animals". Remington's Pharmaceutal Sciences, (Easton, Pa.: Mack Publishing Co., 1975), p. 1843. All drugs as encompassed by any of these definitions can be incorporated into the coacervate compositions of the present invention for oral or parenteral administration to mammals.

It is known that the active components of many parenteral drugs such as antibiotics, anesthetics, anticancer agents, hypertensive compounds, and the like, are insoluble or only slightly soluble at the pH of plasma or tissue fluids and as such, the procedures commonly employed to deal with this problem include use of the salt of the parent compound and/or use of propylene glycol and/or alcohol as a solubilizing agent(s) for the drug. Physician's Desk Reference, (oradell, N.J.: Medical Economic Co., 1986).

U.S. Pharmacopoeaia, indicates that of the injectable drugs used in the direct medical treatment of diseased states, approximately 200 are prepared from the salt of the parent compound and administered at a pH which differs significantly from that of body tissue. It follows from accepted pharmaceutic principles that when a drug based upon the salt of a parent compound is injected, the intracellular and extracellular water at the site of injection will buffer the drug sufficiently to alter its pH and cause a quantity of it to precipitate out of solution and into the injected site.

The following list illustrates the extensive use of the salt of the parent compound in injectable drugs as well as the frequency of an associated excessive pH. As is evident, virtually all classes of drugs are represented, and, in accordance with the principles of the present invention, are suitable for formulation in all embodiments of the coacervate compositions of the present invention for oral or parenteral administration or for tissue absorption.

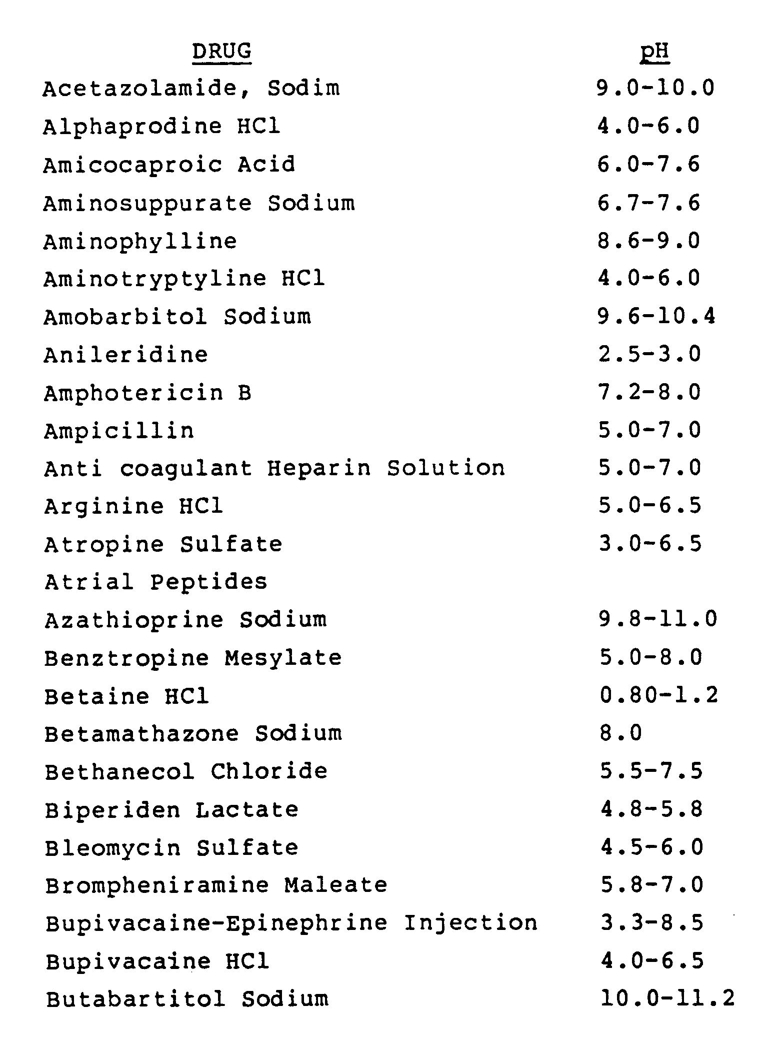

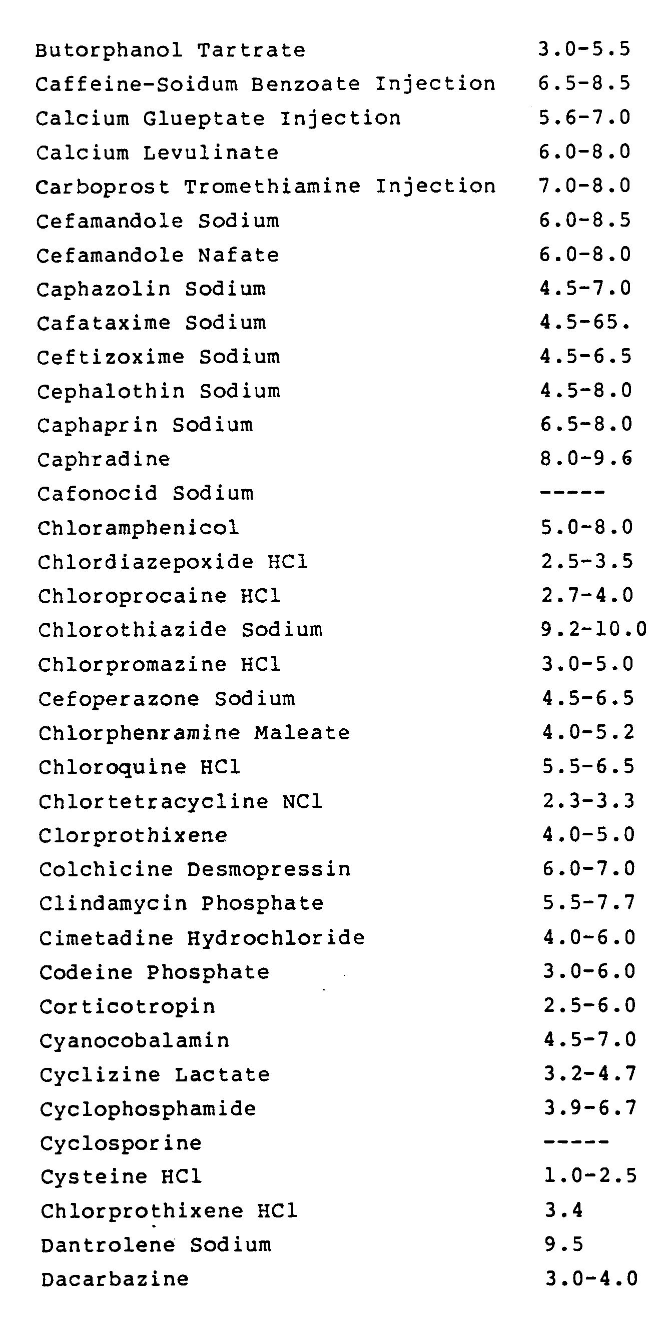

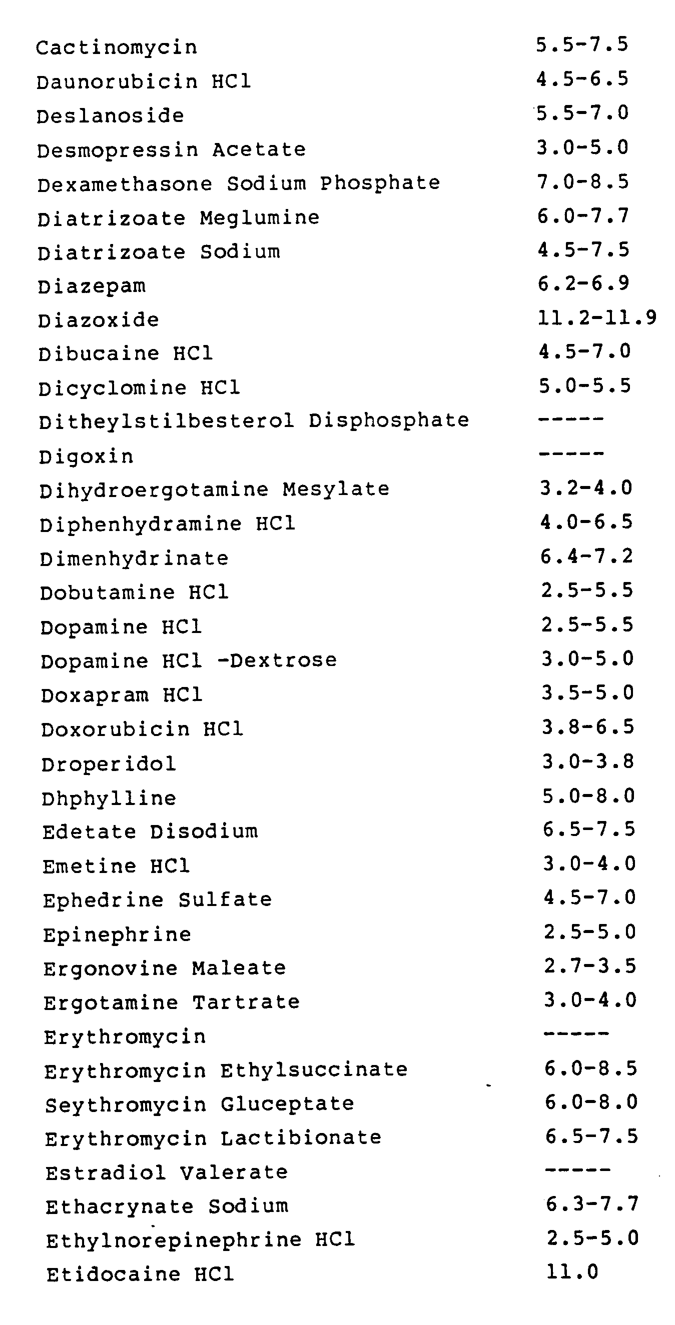

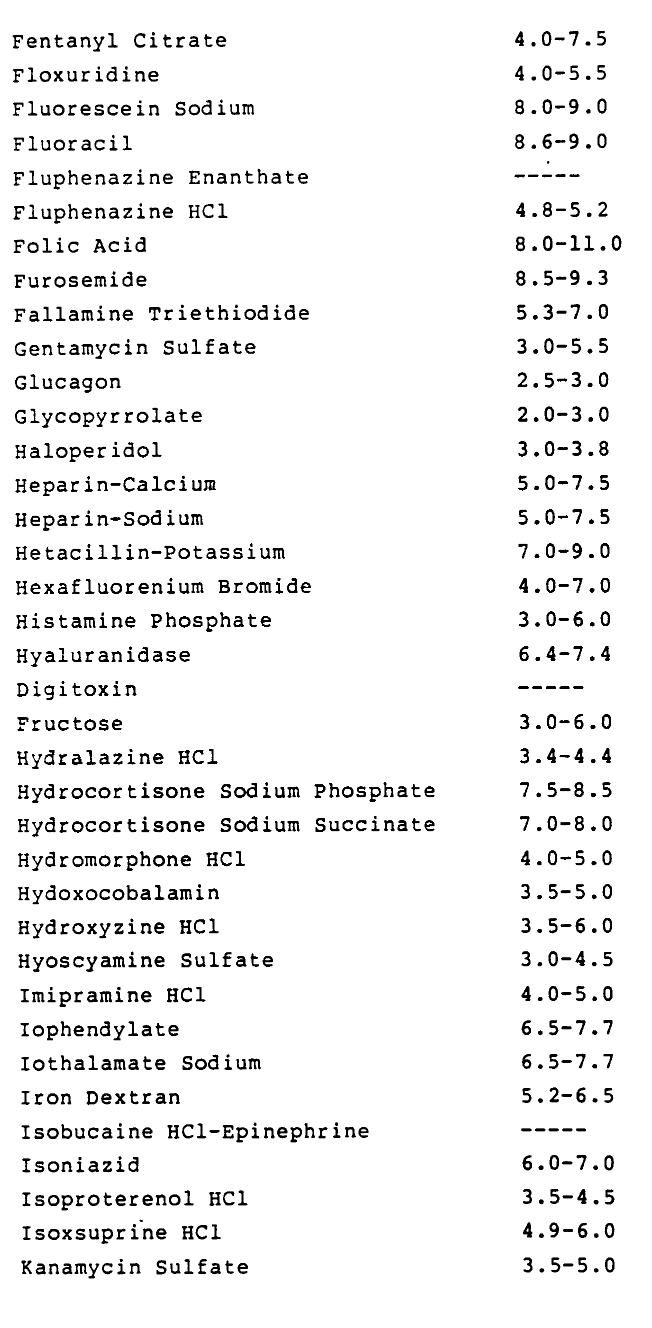

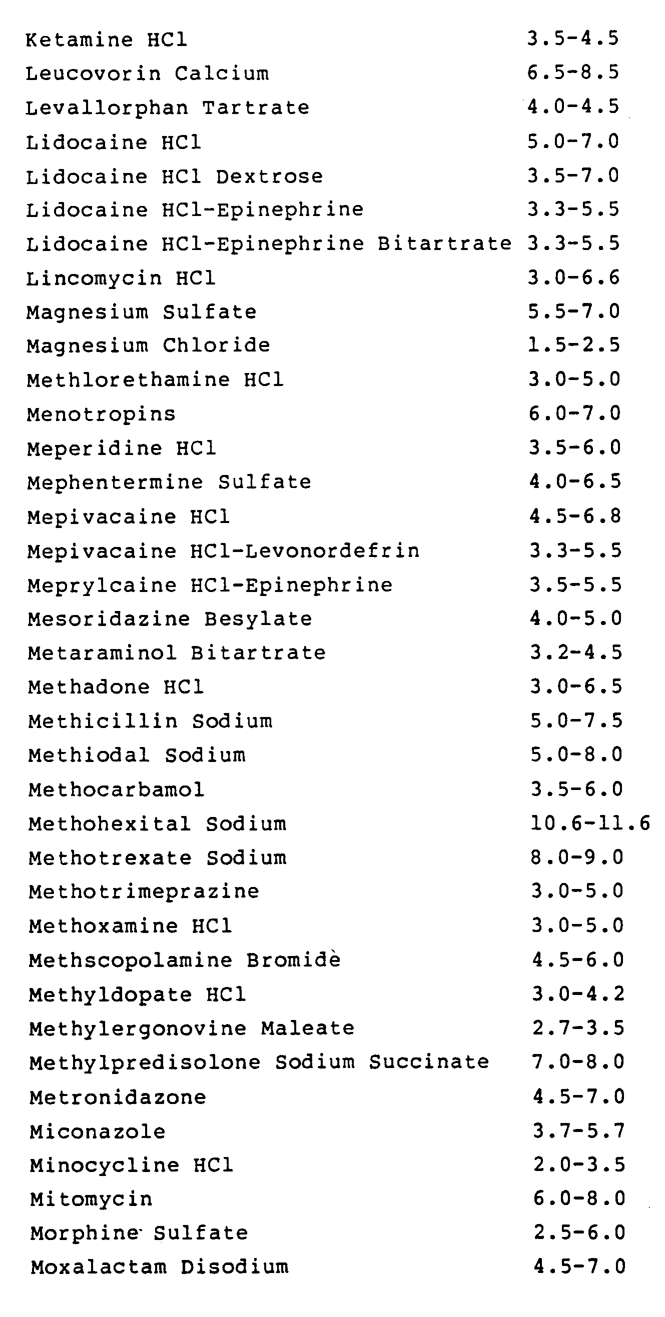

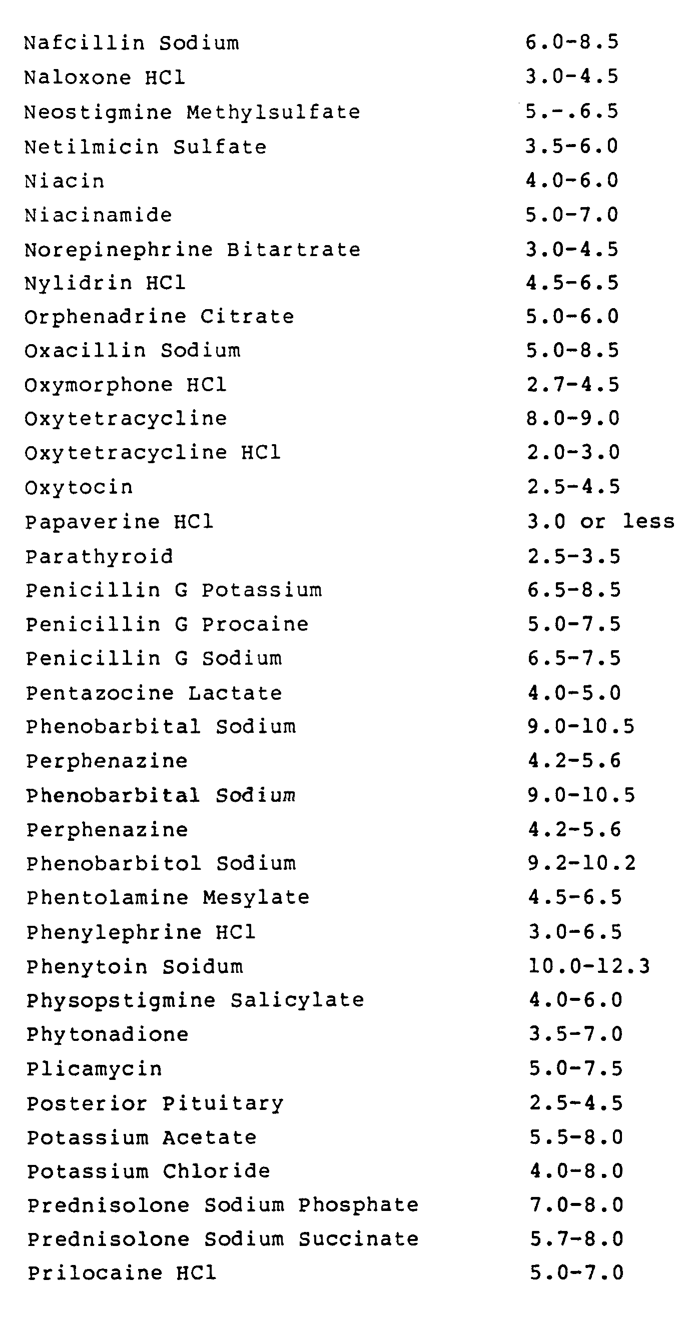

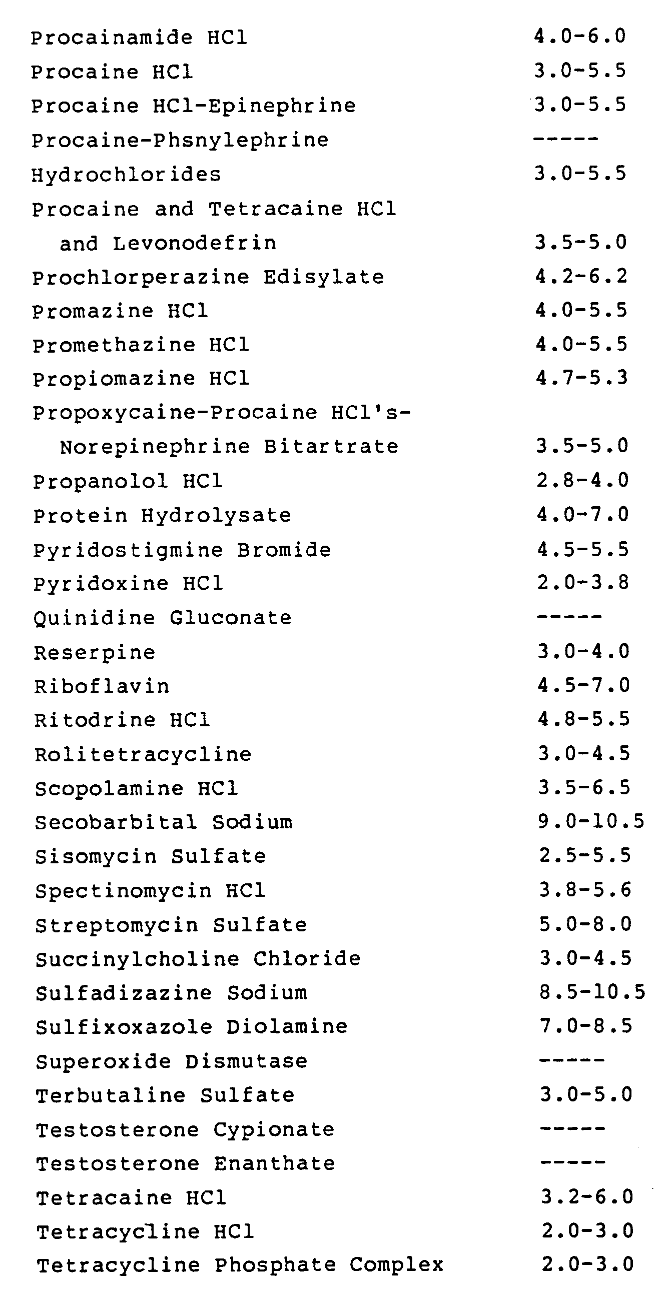

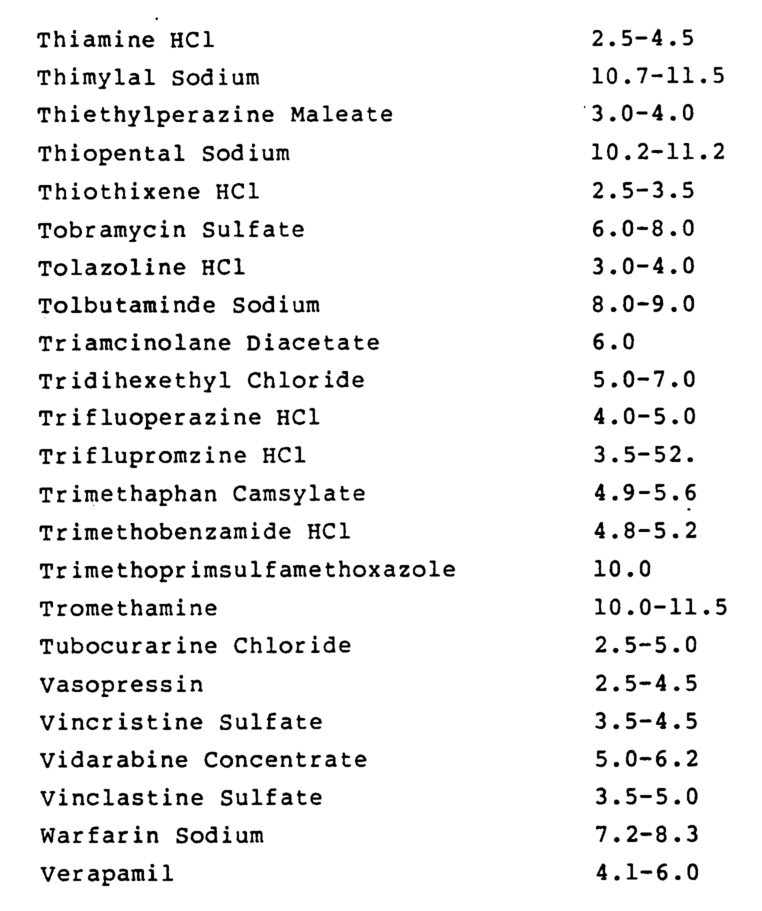

It is to be understood that the above list of drugs is for purposes of illustration and is not provided as an all inclusive list of all the drugs which may be beneficially formulated or reformulated using the oral or parenteral drug, tissue absorptive, transmucosal, or inhalation delivery compositions of the present invention. Other physiologically-active compounds that can be encapsulated in the coacervate compositions of the present invention include biologically-active compounds, such as proteins, enzymes, anti-enzymes, peptides, catecholamines, anti-histamines, analgesics, and the like. For the purposes of the present invention "biological" is defined to mean any medically useful composition derived from a biological source and/or a synthetic pharmacological equivalent thereof such as insulin, heme, hemoglobin, and hormones; "enzyme" or "enzyme system" is defined to mean any protein or conjugated protein produced biologically or synthetically and which functions as a biocatalyst; "peptide" refers to peptides and polypeptides derived from either endogenous, exogenous, or synthetic sources and is used to mean polymers of amino acids linked together by an amide type linkage known as a peptide bond. Anti-enzymes are chemical or biological entities specific to a given enzyme which act to interfere with or terminate the biological activity of the enzyme. Other medically useful compositions known to those skilled in the art, for example, globulin, one or more glycoproteins, such as erythropoietin, also may be incorporated in the compositions of the present invention.

Drug compounds dissolved in any non-toxic, physiologically-acceptable solvent such as a glycol, for example, propylene glycol, and/or alcohol also may be used in the practice of this invention. The procedure and quantity of drug incorporated when using such compositions are any desired quantity, such as that described.

The following are examples of standard doses which can be incorporated into the coacervate-based matrix-enveloped compositions or products of this invention:

| | |
|---|---|
| Cimetidine HCl | 150 mg/ml |
| Diazepam | 5 mg/ml |
| 5-Fluorouracil | 500 mg/10ml |
| Erythromycin Lactobionate | 1 mg/ml |
| Flosuridine | 500 mg/5ml |
| Amthoteracin D | 0.1 mg/ml |
| Fluphenazine HCl | 2.5 mg/ml |
| Heparing Sodium | 1,00-20,000 units/ml |
| Haloperidol lactate | 5 mg/ml |
| Insulin | 40 units |
| Ketamine HCl | 10 mg/ml |
| Labeltol HCl | 5 mg/ml |
| Lipocaine HCl | 10 mg/ml |
| Miconozole | 10 mg/ml |
| Morphine Sulfate | 0.5-1.0 mg/ml |
| Dropendal | 2.5 mg/ml |
| Imipramine HCl | 25 mg/2ml |
| Phenytoin | 100 mg/ml |
| Pentobartital Sodium | 50 mg/ml |
| Tetracycline HCl | 250 mg/100ml |
| Thiopental Sodium | 0.2 mg/2ml |
| Verapamil HCl | 2.5 mg/ml |
| Vincristine Sulfate | 1.0 mg/ml |
| Fentanyl citrate | 0.05 mg/ml |
| Methylprednisolone Sodium | ----- |
| Succinate | 40 mg/ml |

Once the drug is solubilized in (held within the matrix of) the colloid-rich phase of the composition of the present invention and adjustments are made to the pH, if necessary, e.g., to 7.3-7.4 by the addition, for example, of either hydrochloric acid or sodium bicarbonate, the preparation may be administered or stored in the standard or desired dose in appropriate containers such as ampules, vials, and the like. Alternatively, following solubilization of the drug in the coacervate phase, the previously separated colloid-poor phase can be added and the resulting mixture emulsified using techniques known to produce microemulsions. Following adjustment of the pH to, for example, 7.3-7.4, by adding either HCl or sodium bicarbonate as required, the preparation is now ready for use or storage. In some instances, as noted previously, the colloid-poor phase may be used to solubilize and prepare formulations of polar and semipolar drug compositions.

Other medically useful compositions encompassed by the definition of drugs herein are also delivered more effectively orally, parenterally or by other means of administration that eventuate in tissue absorption of the drug(s) by incorporation into the compositions of the present invention since such incorporation improves such parameters as stability, solubility, delivery and the like. Other medically useful compounds capable of being incorporated in the compositions of all embodiments of the present invention include vitamins, for example, vitamin B complex, vitamins A, D, E and K, vitamin B₁₂, folic acid, and the like. Additionally, enzymes such as glucagon, lipase, a-amylase, superoxide dismutase, and the like, and nutrients such as fats, proteins, amino acids and carbohydrates may be incorporated into the drug delivery compositions of the present invention. Nutritional formulations using the compositions of the present invention may include both water-soluble and water-insoluble compounds, for example, vitamins, polyunsaturated corn oil, soy oil, triglycerides, amino acids, soy protein isolate, soy lecithin, corn syrup, sucrose and other nutritional entities known to be useful when normal food intake is precluded or otherwise interfered with. Peptide and/or polypeptide compositions, particularly dipeptides and tripeptides, also can be included in the compositions of the present invention. Additional active compounds includeable into the coacervate compositions of the present invention, particularly for oral administration include Azothioprine; Cyclosprine; Monensin; low molecular weight Heparin, Amrinone and other ionotropics; Superoxide Dismutase; Protaglandins; Interferons; Urokinase; hGH (human growth hormone); Aminoglycoside antibiotics; Estrogen; Cephalosporins; Androgens; Anti-androgens; Renin Inhibitors; Lipoxygenase Inhibitors; Hypothalmic and Pituitary Hormones; Cardiac Glyscosides; Anti-inflamatory steroids; Non-steroid Inflamatory drugs; Vancomycin; Tissue Plasminogen Activators; Enzymes such as adenosine deaminase; and blood factors, such as Factor VIII and Factor IX complex.

Compositions of one embodiment of the present invention comprise one or more non-toxic monomeric or polymeric surface active agents which may be derived from endogenous, exogenous or synthetic sources. For purposes of this invention, they may be selected from any of the classes of surfactants described by Zographi (Ref: Remington's Pharmaceutical Sciences, pp. 295-296, Mack Publishing Co. 1976). The polymers are obtained from the following monomers: anionic, cationic, amphoteric, and non-ionic surfactants. Surface active agents in the anionic classification include di-(2-ethylhexyl) sodium sulfosuccinate; non-ionic compositions include polyethylene glycol (PEG) and polymerized esters; and amphoteric surface active agents include (1) compounds classified as simple, conjugated, derived, and secondary proteins such as the albumins; gelatins; modified fluid gelatins; lipoproteins; alpha, beta and gamma globulins and glycoproteins, and (2) compounds referred to as phospholipids. The amine salts and the quaternary ammonium salts within the cationic group also comprise useful surfactants. Other surfactant compounds useful to form coacervates in accordance with the principles of the present invention include compositions within the groups known as the polysaccarides and their derivatives, such as chitosan; dextran; the mucopolysaccarides; and the polysorbates and their derivatives. Synthetic polymers that may also be used include compositions such as polyethylene glycol and polypropylene glycol. Other suitable surface active agents that can be used to prepare coacervate compositions useful in accordance with the principles of the present invention include pectin, glycoprotein, glycolipid, galactose, and modified fluid gelatin. Acceptable coacervate compositions can be made from single surface active agents listed above or through the use of appropriate combinations thereof.

Compounds that are not intrinsically surface active may also be used to prepare coacervates provided they can be made surface active by chemical or other means. Thus, fatty acids are not considered to be surface active compounds; however, when they are reacted with an alkaline chemical entity, the resulting reaction products will include one that has surface active properties. For instance, mixing stearic acid with sodium hydroxide will produce a salt of stearic acid that has surfactant characteristics.

Virtually all surfactants useful to this invention can by polymerized (e.g., modified fluid gelatin) and used in the method and compositions of the present invention.

As noted above, known synthetic polymers, e.g., polyethylene glycol dextran, a polysaccaride, and the like, may be used in this invention.

To achieve the full advantage of the present invention, the coacervate matrix includes at least one polymerized surfactant. The polymerized surfactant or a combination of polymerized and monomeric surfactants have produced delivery systems that are exceptional in their ability to entrap hemoglobin and other pharmaceutical components.

Surfactants useful to this invention can be polymerized by using, as an example, any of the customary aldehydes (e.g., gluteraldehyde) and following known procedures associated with the use of the aldehydes to produce the polymer form.

To achieve the full advantage of the present invention, the combination of the surface active compounds include polymerized albumin and polymerized lecithin; polymerized albumin and monomeric lecithin; monomeric albumin and polymerized lecithin; casein and polymerized lecithin; gelatin and polymerized lecithin; pectin and polymerized albumin. It should be noted, however, that coacervate compositions useful in accordance with the principles of the present invention can be made from selected single surface active agents such as polymerized albumin or polymerized lecithin.

To achieve the full advantage of the present invention, one of the surface active agents should be a phospholipid, such as lecithin or polymerized lecithin, in the composition of the present invention. It may be derived from any suitable source. Egg lecithin is preferred. Other phospholipids such as cephalin, isolecithin, sphingomyelin, phosphatidyl serine, phosphatidic acid, phosphatidyl inositol and phosphatidyl choline, and combinations thereof, may be used in place of or in addition to lecithin, particularly where the other surfactant is polymerized albumin.

Further, to achieve the full advantage of the present invention, a suitable surface-active protein, such as albumin or polymerized albumin, comprises another component of the compositions of the present invention. Albumin derived from any acceptable source, such as human, is acceptable. Other suitable surface active proteins include alpha-, beta- and gamma-globulins, gelatin, modified fluid gelatin, lipoproteins, polypeptides and mucopolysaccarides. While albumin is preferred, any suitable, non-toxic protein, i.e., simple, conjugated or derived proteins or mixtures thereof, including the globulins may be used in place of albumin. When globulin is used in the process described in this specification, the finished product not only comprises a coacervate but a composition in which the medical usefulness and the activity of the gamma globulin is prolonged.

The use of an aqueous based coacervate composition as the starting composition for the microemulsions of the finished products of the present invention distinguishes the compositions of the disclosed invention from known microemulsions that are comprised of a lipid (liquid or solid) or oil phase and a water phase. It should be noted that stabilization of known microemulsions by encasing the microemulsions in a coacervate phase matrix and/or film, in accordance with the principles of the present invention, is an important feature of the present invention.

According to another embodiment of the method and composition of the present invention any non-toxic molecule capable of carrying, and releasing oxygen to the bloodstream of a patient, and/or an oxygen solvent, preferably a molecule that contains iron, can be used as the physiologically-active component of the composition. Further, it has been found that, a heme, and/or a hemoprotein and/or a heme-hemoprotein complex can be used as the physiologically-active component. Since the heme, hemoprotein and heme-hemoprotein complexes contain iron, these preferred compounds possess an important advantage because sources for the physiologically-active component include endogenous, exogenous and synthetic compositions.

As used above and hereinafter, heme is defined to comprise the prosthetic group of hemoglobin. However, according to the composition and method of the present invention, prosthetic groups of other compositions, such as myoglobins, catalases, cytochromes b and some peroxidases can also can be used to form the heme-hemoprotein complexes. The preferred heme molecule for use in the present invention is a complex of iron and protoporphyrin 9, type III. Similarly, hemoproteins that can be used above or in the heme-hemoprotein complex include hemoglobin, ovoglobulin, ovocanalbumin, Cytochrome c, Cytochrome P₄₅₀, polymerized hemoglobin, pyridoxilated-polymerized hemoglobin, ovomucin and lactalbumin. To achieve the full advantage of the present invention, when using a hemoprotein or the heme-hemoprotein complex combination, hemoglobin of human, bovine, synthetic or genetic engineering sources, in stroma-free form, is utilized. as the hemoprotein.

In accordance with an important embodiment of the present invention, all of the components of the present invention, and especially the heme, the hemoprotein and/or the heme-hemoprotein complex should be be pure and stable. Therefore, either prior to or after incorporating the heme, hemoprotein and/or heme-hemoprotein complex into the composition of the present invention, a solution of the heme, hemoprotein and/or the heme-hemoprotein complex is treated with a chemical agent that forms stable iron complexes with the heme, hemoprotein or heme-hemoprotein complex, such as a carbon monoxide or an inorganic cyanide, particularly cyanide salts or cyanide complexes, such as sodium cyanide hydrogen cyanide. The chemical agent can be removed readily from the heme, hemoprotein and/or heme-hemoprotein complex when an excess of oxygen is introduced into the solution. For example, carbon monoxide is bubbled through the heme, hemoprotein and/heme-protein solution until all oxygen is removed from the solution. In a subsequent processing step, the complexed carbon monoxide is removed by introducing an excess of oxygen to the heme, hemoprotein and/or heme-hemoprotein complex. In accordance with a new and unexpected feature of the present invention, the intimate contact of the solution with carbon monoxide stabilizes the heme, hemoprotein and/or heme-hemoprotein complex during the manufacturing process, thereby preventing oxidation of the heme, hemoprotein and/or heme-hemoprotein complex.

Further, the composition prepared according to the method of the present invention provides iron that is absorbed into the body of humans and other mammals from the heme, hemoprotein and/or the heme-hemoprotein molecules as intact heme molecules. This absorption duplicates the natural source and natural mode of iron absorption and, therefore, provides a significant improvement over iron compounds now in commercial use. For example, usually the iron that is present in such commercial compositions first must be converted to ferrous iron in the stomach and duodenum before any absorption occurs.

In accordance with an important feature of the present invention, the heme, hemoprotein and/or heme-hemoprotein complex present in the composition after oral administration is protected from degradation in the digestive tract by the coacervate phase of the coacervate system. A film of coacervate-phase water surrounds and completely coats each heme, hemoprotein and/or heme-hemoprotein complex to protect the heme, hemoprotein and/or heme-hemoprotein complex from gastrointestinal degradation. The coacervate-phase water differs in physical structure from the bulk water which is present in the stomach, thereby slowing the diffusion of digestive enzymes in the bulk water. Accordingly, the digestive enzymes are unable to penetrate the film of coacervate-phase water that covers each heme, hemoprotein and/or heme-hemoprotein complex molecule, thereby reducing the rate of interaction of the enzymes with the heme, hemoprotein and/or heme-hemoprotein complex. This slow rate of interaction allows physiologically useful quantities of heme, hemoprotein and/or heme-hemoprotein complex to avoid degradation and, therefore, to pass through the walls of the small intestine and into the circulatory system of the recipient. The protective feature afforded by the coacervate phase also operates to protect the oxygen-carrying physiologically-active component(s) against degradation effects of pH, acid-base balance and other conditions and processes of the gastrointestinal tract.

In accordance with another important embodiment of the present invention, liposomes and their contents are enveloped with a coacervate-based film -the film being derived from any of the two-phase liquid aqueous non-toxic coacervate compositions described herein. Liposomes of any acceptable composition and of any lamellar structure, i.e., unilamellar, multilamellar, or plurilamellar, or any combination may be used in accordance with the principles of this embodiment of the present invention. Further, the liposome may contain any of the following physiologically-active components: any oxygen carrying molecule or oxygen solvent(s), any drugs, hormones, biologicals, steroids, peptides, tissue plasminogen activators, enzymes, anti-enzymes and/or any other compositions or combinations thereof which are of biological or medical interest. Suitable oxygen solvents include perfluorocarbons; the coacervate phase of a lecithin-water coacervate solution; the coacervate phase of a lecithin-albumin-water solution, and the like. The source of the physiologically-active compound(s) can be either endogenous, exogenous or synthetic or any acceptable, non-toxic combination thereof.

The liposome embodiment of the present invention comprises a liposome component, the pharmaceutic and/or pharmacologic entity(s), or combinations thereof, contained within the liposomes, and a coacervate-based matrix and/or film encapsulating the liposome component and its contents. In this embodiment of the present invention, liposomes containing the physiologically-active molecule are mixed into a surfactant solution such as a solution of polymerized albumin. To achieve the full advantage of this embodiment of the present invention, the surfactant (polymerized albumin) solution also contains polymerized lecithin and is converted to a coacervate phase to form a coacervate matrix and/or film enveloping the liposome composition.

Further emulsification of this mixture produces a composition enveloping the liposome in a coacervate-based matrix and/or film. The emulsified composition including the liposome enveloped by a coacervate-based matrix and/or film then is microemulsified to produce a microemulsion having particles of 1 micron or less in size. As an option, microemulsion(s) can be processed further to produce sustained release compositions; the methods include application of heat; use of a cross-linking agent, or repeating specific process steps.

As a further option, the composition may be dried, such as by treatment with heat, or freeze dried to produce a lyophilized product, which can be produced to any desired particle size and reconstituted with any physiologically appropriate fluid. When reconstituted, such compositions include a resuscitation fluid and a delivery system to introduce compositions of biological or medical interest into the body.

The specific compositions of this invention can be administered either parenterally, transdermally, orally, absorbed through the tissue lining of other body sites, or via inhalation, or combinations thereof, as appropriate.

When the reconstituted composition is intended for intravenous use, isotonicity and pH should be adjusted by known procedures to normal body values before administration. A single layer of coacervate matrix and/or film encapsulates the composition. If desired, in accordance with a preferred method, the finished product can be subjected to film encapsulation procedures repeatedly to produce concentric films of any desired thickness. Each film can contain no additional physiologically-active component or each can contain a desired concentration of any physiologically-active component, same or different.

While it is preferred that the microparticles of the finished products of this invention be 1 µm (micron) or less, particularly when administered parenterally, there are conditions, such as for oral administration, when microparticles of this invention are 2 µm (microns) or more in size.

In accordance with one embodiment of the present invention, any appropriate non-toxic coacervate composition containing one or more polymerized surface active agents, particularly polymerized albumin and/or polymerized lecithin can be used in the manufacture of the drug delivery compositions of the present invention. The coacervate-enveloped compositions of the present invention incorporate non-toxic endogenous or exogenous surface-active agents, derivatives thereof and/or combinations of surface-active agents or derivatives.

In the preferred formulation, polymerized albumin comprises one component. The lecithin that is used in this method may be monomeric or polymerized. One method by which lecithin can be polymerized is as follows: Remove one or both of the fatty acids of lecithin by hydrolysis. This step results in a free hydroxyl group on each lecithin molecule. This in turn produces a lecithin composition that lends itself to polymerization by customary techniques.

To achieve the full advantage of the present invention, when polymerized albumin is used, the polymerized albumin should contain from two to six albumin molecules. Longer molecule chains may be used but are not preferred. To achieve the full advantage of the present invention, the weight average molecular weight of the polymerized albumin, when used in this invention should range from about 200,000 to about 300,000, preferably from about 200,000 to about 280,000. Compositions of greater or lesser molecular weight may also be used. The albumin component can be derived from any acceptable, endogenous, exogenous, or synthetic source including genetically engineered sources. The lecithin may be derived from any suitable non-toxic source. In the practice of this invention, any acceptable phospholipid can be substituted for lecithin. In such case the phospholipid may be monomeric or polymerized. To achieve the full advantage of the present invention, when polymerized lecithin used in the method and compositions of the present invention, the polymerized lecithin should contain three to four linked lecithin molecules and has a weight average molecular weight of about 1600 to about 3200, preferably about 2500. Shorter or longer chain phospholipids also may be used.

In preparing the red blood cell substitutes (resuscitation fluids), it is preferred that a reducing agent be added to the product in the process of manufacture.

Depending upon the finished product to be produced, the pharmaceutically-active component may be an oxygen carrying molecule, an oxygen solvent molecule, hormone(s), water soluble drugs) water insoluble drug(s), biological(s), a water sensitive drug(s), a peptide(s), an enzyme(s), and the like, either singly or in combination. The active component is incorporated in the composition during the manufacturing steps in that quantity that will provide the desired clinical amount. Thus, for example, in the case of drugs, this will be the dose given in the Physician's Desk Reference plus five to fifteen percent. If desired, greater or lesser amounts of any given drug may be used to meet specific medical requirements. In the case of the inclusion of hemoglobin, the preferred quantity is that amount that will produce fourteen percent hemoglobin in the finished product. Greater or lesser quantities of hemoglobin may also be used depending upon the end use of the finished product. If desired, a sterol, such as cholesterol, and/or urea preferably 0.1% to 3% may be included in the composition to formulate red blood cell substitutes.

Any composition of this invention to be administered orally can be formulated to any dosage, e.g., capsules, tablets, syrups, emulsions, suspensions, microemulsions, and the like.

When preparing the intravenous red blood cell substitute of this invention, the basic method is followed. Additional steps are used, however, to adjust the pH, viscosity, and isotonicity of the preparation, when necessary, to approximate the values of whole human blood. In the method of this invention that prepares parenteral products, the pH is adjusted to approximately 7.4. The oral drug compositions of this invention require no adjustment of pH, viscosity or isotonicity. pH adjustment, however, is preferred. Manufacture of all preparations of this invention are preferably made under sterile conditions.

In accordance with another embodiment of the present invention, a composition comprising a non-toxic perhalogenated compound and a non-toxic two-phase coacervate aqueous system is utilized to prepare medically-useful products for oral or parenteral introduction into the body. The coacervate-based composition can be used to solubilize and stabilize physiologically-active compounds, in order to facilitate delivery of medically-useful products, such as a resuscitation fluid, an oxygen-carrying contrast medium, or a pharmaceutical, into medical patients.

The perhalogenated compounds that are useful in the present invention include organic compounds such as hydrocarbons, ethers and/or amines, having essentially all of the hydrogen atoms of the organic compound replaced by a halogen atom, such as fluorine, iodine, chlorine or bromine. Of particular interest are the perfluorinated and perfluoroiodinated compounds, especially the perfluorohydrocarbons and perfluoroamines, or hydrocarbons and amines having essentially all of the hydrogen atoms replaced by fluorine atoms.

To prepare the red blood cell substitute, the following method is used: to 100 mls of a 30% solution of monomeric lecithin, add 10 gms of stroma-free hemoglobin and, if desired, also add 0.1% cholesterol. If cholesterol is used first it should be dissolved into the surfactant, e.g., lecithin, such as with the aid of a solvent, e.g., n-butyl alcohol. The quantity of the hemoglobin component can vary from 1% w/v to 20% w/v. If desired, modified hemoglobin may be substituted in the same quantities for stroma-free hemoglobin. In addition, hemoglobin encapsulated within liposomes may be substituted for the preferred stroma-free hemoglobin. It is preferred that a reducing agent and/or an antioxidant such as reduced nicatinamide adenine dinucleotide phosphate (NADPH), or reduced nicatinamide adenine dinucleotide phosphate (NADH), or glutathione, or dextrose, or ascorbic acid or other known non-toxic antioxidants be added as the next step. Appropriate combinations of reducing agents also may be used, if desired. If added, such compounds are added in such quantity as will prevent the oxidation of the hemoglobin component; generally trace amounts up to 3 molar percent based upon the molar amount of hemoglobin. To achieve the full advantage of the present invention, the reducing agent should be included in an amount of from trace amounts to 6 millimoles per mole of hemoglobin. Acceptable compositions may be prepared without the use of reducing agents.

Following this step add 20 gms of either monomeric or polymerized albumin. The polymerized form is preferred. The resulting composition then is processed by means of a colloid mill and/or a microfluidizer for 10 minutes. During this processing step add a polymerization initiator, e.g., a solution of 1-ethyl-3-dimethyl-aminopropyl-carbodiimide of about 10% added during colloid milling and/or microfluidizing for in-situ polymerization of the albumin during processing of the composition. The quantity of polymerization initiator may range from 0.1% to 20% based on the total weight of the composition. Following this processing step the product is stored from 4 to 12 hours at a temperature that may range from 4° C to 10° C. The internal phase (particles) are separated from the aqueous system by known filtering or separation means and washed with sterile saline solution. The particles then are dispersed in physiological saline solution.

The pH of the preparation is adjusted, if necessary, to approximate that of whole human blood. Addition of HCl or NaOH may be used for this purpose. Isotonicity of the product is adjusted, if necessary, to approximate that of whole human blood. This may be accomplished by the addition of appropriate amounts of any of the following: electrolytes present in human plasma, normal physiological saline solution, Ringer's solution, or any other suitable composition.

Next, adjust the viscosity of the product so that it approximates the viscosity of whole human blood, for example, by the addition of water to decrease the viscosity, or by adding any non-toxic, physiologically-acceptable hydrophillic colloid, such as albumin or pectin, to increase the viscosity. On completion of this step the product comprises a microemulsion that consists of microencapsulated particles containing the hemoglobin component. It should be emphasized that this method encapsulates hemoglobin more efficiently than that of the inventor's previously disclosed encapsulating techniques.

At this stage of manufacture the product may be used as a red blood cell substitute (resuscitation fluid) provided that it is first filtered to remove all extraneous matter and all particles that exceed 1 micron in size in any dimension.

It is preferred, however, that the filtered product be processed further using either a chemical process based on a crosslinking agent, such as gluteraldehyde, or a physical processing step such as heat. The heating step is preferred and consists of heating the preparation for 2 minutes at a temperature that may range from 50° C to 70° C. The duration of the heating step may be more than 2 minutes or less than 2 minutes depending upon the desired degee of particle(s) surface film structuring. While temperatures less than 50 degrees Centigrade are feasible for this purpose, they are not preferred. A combination of crosslinking and heating for film hardening also can be used.

Depending upon the time and temperature used in the the heating step, the encapsulating surface film produced by this step will range from gel-like to rigid in character. Following this step the resulting composition is filtered to remove all extraneous matter and any microencapsulated particles that exceed 1 µm (micron) in size in any dimension. The finished product of this invention can consist of particles with a single degree of surface film hardness or, alternatively, can consist of particles of mixed surface film hardnesses. The desired quantity of said particles can be added to any appropriate physiological fluid. As such it will then comprise a product suitable for intravenous administration as a red blood cell substitute (resuscitation fluid). If desired, the microencapsulated particles may be used in much the same manner as packed red blood cells. If desired, the product may be lyophilized or otherwise dried using conventional techniques. The dried composition may be reconstituted as needed using an appropriate physiological fluid either as a blood substitute or as a perfusion fluid.

This invention provides for several alternative methods of preparing an intravenous red blood cell substitute. One alternative consists of the following steps: prepare polymerized albumin by adding 1 ethyl-3-dimethyl-aminopropyl-carbodiimide (EDC) to a 10% solution of monomeric albumin in an amount sufficient to produce a concentration of 5.0% w/v EDC in the product. Process in a colloid mill and/or a microfluidizer for about 10 minutes. Next, store the product at 4° C for 10 hours. Next, mix a 10% w/v solution of pyridoxilated polymerized hemoglobin in an amount sufficient to achieve a hemoglobin concentration of 10% by weight of the surfactant-water composition and, if desired, add 2 millimoles of NADPH into the product and process in a colloid mill and/or a microfluidizer for about 10 minutes; following this step, mix a solution of polymerized lecithin into the solution in an amount sufficient to provide a polymerized lecithin concentration of 8% by weight of the solution and process again in a colloid mill and/or a microfluidizer for 10 minutes. Next, adjust the pH of the product to that of whole blood using HCl or NaOH as necessary. Following this step, adjust the isotonicity using plasma electrolytes in such concentration as will give the product the isotonicity of whole human blood. Adjust the viscosity as the next step so that the viscosity approximates that of whole blood. At this stage of manufacture the product may be used as a red blood cell substitute (resuscitation fluid) provided that it is first filtered to remove all extraneous matter and all particles that exceed 1 µm (micron) in size in any dimension. It is preferred, however, that the filtered product be processed further using either a chemical process based on a non-toxic crosslinking agent, such as gluteraldehyde, or a physical processing step, such as heat. The heating step is preferred and consists of heating the preparation for 2 minutes at a temperature that may range from 50° C to 70° C producing a film that can range from gel-like to rigid in character. Following this step, the resulting composition is filtered to remove all extraneous matter and any microencapsulated particles that exceed 1 µm (micron) in size in any dimension. The finished product of this invention can consist of particles with a minimal degree of surface film hardness or, alternatively, it can consist of particles of mixed surface film hardnesses. The desired quantity of said particles can be added to any appropriate physiological fluid. As such it will then comprise a product suitable for intravenous administration as a red blood cell substitute (resuscitation fluid). If desired, the microencapsulated particles may be used in much the same manner as packed red blood cells. If desired, the product may be lyophilized or otherwise dried and reconstituted with any appropriate physiological fluid.

In accordance with another important embodiment of the present invention, the coacervate-based, hemoglobin-containing compositions of the present invention can be used as a perfusion fluid for preventing degeneration of an organ or tissue used for transplantation or replacement of the organ or tissue for a mammal. When used as a perfusion fluid, the hemoglobin-containing active compound, e.g., hemoglobin, stroma-free hemoglobin, pyridoxilated polymerized hemoglobin and/or modified hemoglobin, should be present in the coacervate composition in an amount of 0.5% to 30% by weight of the coacervate composition, preferably 1% to 20% by weight of the coacervate composition. The coacervate-based perfusion fluids of the present invention are useful with or without a hemoglobin component and can be used in ambient air or may be oxygenated for additional oxygen transfer to the tissue or organs. The coacervate-based perfusion fluid is capable of maintaining tissue and organs viable for up to at least 15 days and sometimes up to 30 days whereas prior art perfusion fluids generally are capable of sustaining tissue viability for only about 8 to 10 hours.

Another alternative coacervate-based intravenous red blood cell substitute using the methods of this invention comprise the following steps:
Mix 10% w/v of stroma-free hemoglobin into a solution of polymerized lecithin of about 20% concentration. Process the product in a colloid mill and/or a microfluidizer for about 2 minutes. Mix ascorbic acid of at least 0.1 gm per 100 ml of solution and an amount equal to 10% w/v (100 units/cc. in the finished product) of polymerized albumin into the product and process the resulting product in a colloid mill and/or a microfluidizer for about 5 minutes. Next, store the product for about 10 hours at 4° C. Adjust the pH of the preparation after the period of storage to about 7.4 using HCl or NaOH as necessary. Following this step, add electrolytes present in human plasma in such amount as will give the product the isotonicity of whole human blood. Next, adjust the viscosity of the product so that it approximates the viscosity of whole human blood. On completion of this step, the product comprises a microemulsion that consists of microencapsulated particles containing the hemoglobin component. At this stage of manufacture the product may be used as a red blood cell substitute (resuscitation fluid) provided that it is first filtered to remove all extraneous matter and all particles that exceed 1 µm (micron) in size in any dimension. It is preferred, however, that the filtered product be heated for 2 minutes at 70° C. The product then is filtered to remove all extraneous matter and all microencapsulated particles which exceed 1 µm (micron) in any single dimension. After this step, the product may be used as an intravenous blood substitute or stored preferably at 4° C until needed.

In accordance with another important embodiment of the present invention, the coacervate-based, hemoglobin-containing compositions of the present invention can be used as a perfusion fluid for preventing degeneration of an organ or tissue used for transplantation or replacement of the organ or tissue for a mammal. When used as a perfusion fluid, the hemoglobin-containing active compound, e.g., hemoglobin, stroma-free hemoglobin, pyridoxilated polymerized hemoglobin and/or modified hemoglobin, should be present in the coacervate composition in an amount of 0.5% to 30% by weight of the coacervate composition, preferably 1% to 20% by weight of the coacervate composition. The coacervate-based perfusion fluid is capable of maintaining tissue and organs viable for up to at least 15 days and sometimes up to 30 days whereas prior art perfusion fluids generally are capable of sustaining tissue viability for only 8 to 10 hours.

While the processes described above use hemoglobin as the active component and yield intravenous red blood cell substitutes, this invention, as noted previously, enables the use of any medically useful composition as the active component to prepare compositions that can be administered via parenteral, oral, transdermal, transmucosal and inhalation routes.

Also, while the size of the particles of the injectable or parenteral red blood cell substitute should be 1 micron or less in size in any diameter, there are other embodiments of this invention in which the particle size comprising the composition can be more than 1 µm (micron) and further, in specific instances of this invention desirable compositions will be comprised of a combination(s) of particles with differing sizes; i.e., a given percentage of the particles will be 1 micron or less in size and the remaining percentage of particles will be 2 µm (microns) or more. Percentages and particle sizes will vary depending upon the specific formulation and the specific drug or combination of drugs used in the formulation.

In the preferred embodiment of an orally administered composition of this invention, the following process is used.

More specifically, warm a mixture of 7.5 gms of monoglyceride and 7.5 gms of diglyceride at from 65-70° C until a clear solution of the mixture is produced.

Unequal proportions of these glycerides may be used, but are not preferred. Equal or unequal proportions of mono-, di- and triglycerides also may be used. However, this mixture is also not preferred. When the warming step is completed the pharmaceutical component is mixed into the solution. The product of this step is subjected to a spray drying step while the product is gradually cooled. The product resulting from this step will be a powder comprising particles containing the pharmaceutical component.

The next step is designed to coat the particles with a coacervate film. The procedure is as follows. Make a suspension of the powder referred to above in 100 mls of water containing 10 gms of polymerized albumin and 10 gms of monomeric lecithin. Stir the composition for about two minutes. Add an appropriate alcohol dropwise, n-butyl alcohol is preferred, until a coacervate forms and coats the particles with a coacervate film, stirring during the addition of alcohol and afterward to insure that coacervate encapsulation of the particles continues for from 1/2 to 3 hours. The product then is stored for from 12 to 24 hours at 4° C. The composition can be further microfluidized after encapsulation to further reduce the particle size, regardless of the number of films applied over the particles.

Following the storage step, the particles are separated from its liquid vehicle by filtration or other known appropriate methods and dried using any of the conventional drying techniques. This method yields a finished oral medically useful product comprised of a coacervate encapsulated pharmaceutically-active component.

If desired, the product referred to above can be processed further to produce a sustained release composition. This may be accomplished by any of several methods including application of heat, use of a cross-linking agent, such as gluteraldehyde, or covering the encapsulated product with one or more additional layers of coacervate film. The additional layer(s) of coacervate film is preferred. The additional layers can contain the same or different physiologically-active compound in a concentration of 0.5% to 50% based on the weight of the film, or may contain no additional physiologically-active component.

The procedure is as follows. Make a suspension of the powder referred to above in 100 mls of water containing 10 gms of polymerized albumin and 10 gms of monomeric lecithin. Stir the composition for about 2 minutes. Add n-butyl alcohol dropwise until a coacervate forms and coats the particles with a coacervate film, stirring during the addition of alcohol and afterward to insure that coacervate-encapsulation of the particles continues for from 1/2 to 3 hours. The product is then stored for from 12 to 24 hours at about 4° C.

Following the storage step, the particles are separated from their liquid vehicle by filtration, sieve or other known appropriate methods and washed and dried following any of the conventional drying techniques. Repeating this procedure will add an additional coacervate layer (film) containing no additional physiologically-active compound to the composition. The additional layers (films) can include one or more pharmaceutically-active component together with the coacervate-based encapsulating film if added to the coacervate composition, or may contain no additional physiologically-active component, as desired. This process may be repeated as often as required to produce one or more additional layers (films) with corresponding sustained release effects and/or containing any combination of active components. The particular active ingredient(s) and quantities of the active ingredient(s) within each layer can be varied to meet dosage requirements.

If desired, the structure of the surface of any of the layers referred to above can be hardened by applying heat to the product at a temperature and duration that will not adversely effect the activity of the pharmaceutic component. Any non toxic denaturing or polymerizing agent can be used in place of heat. If the heating step is incorporated in the described method, the release of the active component will be extended over a greater time period. It should be noted that in preparing the sustained release products of this invention, any single procedure or combination of procedures may be used provided that such procedure does not compromise the activity of the pharmaceutical component.

The particle size of the oral form of the composition of this invention can be any size suitable for the optimal delivery of the incorporated pharmaceutical component(s). The herein described oral formulation can be used in any of the known dosage forms such as tablets, capsules, syrups, caplets, powders, and the like.

An alternative formulation for an orally administered medical composition uses the following method. In this method, any non polar volatile, organic solvent may be used. In this instance carbon tetrachloride is the solvent. However, it must be emphasized that in the use of such solvents, all traces of this component must be removed in one of the manufacturing steps before proceeding to produce the finished product. The process is as follows: mix 10 gms of lecithin in 10cc of carbon tetrachloride; this will result in a clear solution. Add the pharmaceutical component into the solution (e.g., 5 gms of erythromycin) and mix thoroughly. Using any appropriate method, i.e., spray drying, distillation or the like, process the product to remove the carbon tetrachloride. Spray drying is preferred. This process will yield powder-like particles comprised of lecithin and the pharmaceutical component encased in the lecithin matrix.

The next step is designed to coat the particles with a coacervate film. The procedure is as follows. Make a suspension of the powder referred to above in 100 mls of water containing 10 gms of polymerized albumin and 10 gms of monomeric lecithin. Stir the composition for about two minutes. Add an appropriate alcohol dropwise, n-butyl alcohol is preferred until a coacervate forms and coats the particles with a coacervate film, stirring during the addition of alcohol and afterward to insure that coacervate encapsulation of the particles continues for from 1/2 to 3 hours. If desired, the same or a different pharmacologically-active component can be added to the albumin-lecithin solution for incorporation into each additional film layer. The product then is stored for from 12 to 24 hours at about 4° C.

Following the storage step, the particles are separated from its liquid vehicle by filtration or other appropriate means and dried using any conventional method. This yields a finished oral product of the pharmaceutically-active component coated with a coacervate-based film. The product can be used to prepare capsules or reconstituted with an appropriate liquid(s), e.g., syrup, to produce a product with the desired clinical dose.

If desired, the product referred to above can be processed further to produce a sustained release composition. This may be accomplished by any of several methods including application of heat, use of a cross-linking agent such as gluteraldehyde, or covering the encapsulated product with one or more additional layers of coacervate film. The additional layer of film is preferred, and any of the additional layers can be hardened for sustained release. It is preferred that the final layer not be hardened for prompt release of any active component contained therein.

The procedure is as follows. Make a suspension of the powder referred to above in 100 mls of water containing 10 gms of polymerized albumin and 10 gms of monomeric lecithin with or without 0.5% to 50% w/v of any physiologically-active compound. Stir the composition for about 2 minutes. Add n-butyl alcohol dropwise until a coacervate forms and coats the particles with a coacervate film, stirring during the addition of alcohol and afterward to insure that coacervate-encapsulation of the particles continues for from 1/2 to 3 hours. The product is then stored for from 12 to 24 hours at about 4° C. Longer contact of the particles with the coacervate, while stirring, provides thicker films for control of film thickness and control of the amount of the active compound, if any, contained therein.

Following the storage step, the particles are separated from the liquid vehicle by filtration, sieve or other known appropriate methods and dried using any of the conventional drying techniques. This procedure will add a coacervate layer (film) to the composition; said layer (film) comprising the pharmaceutically-active component, if any, and the coacervate-based encapsulating film. This process may be repeated as often as required to produce one or more additional layers (films) with corresponding sustained release effects. The quantities of the active ingredient(s) within each layer can be varied to meet dosage requirements.

If desired, the structure of the surface of any of the layers referred to above can be hardened by applying heat to the product at a temperature and duration that will not adversely effect the activity of the pharmaceutic component. Any non toxic denaturing or polymerizing agent can be used in place of heat. If the heating step is incorporated in the described method, the release of the active component will be extended over a greater time period. It should be noted that in preparing the sustained release products of this invention, any single procedure or combination of procedures may be used provided that such procedure does not compromise the activity of the pharmaceutical component.

The particle size of the oral form of this alternative can be any size suitable for the optimal delivery of the incorporated pharmaceutical component(s). The herein described oral formulation can be used in any of the known dosage forms such as tablets, capsules, syrups, caplets, powders, and the like.

It is preferred that both the matrix containing the physiologically-active component(s) and the film that encapsulates it be coacervate derived or based. However, if desired, the matrix and/or film may be comprised of a non-coacervate composition. For example, the film may be comprised of such materials as glycerides, carnuba wax or sugar, and the like. If a sugar coating is to encapsulate the matrix, the particles comprised principally of matrix containing the active compound are dispersed in thick sugar syrup and the composition is mixed rigorously for from 30 seconds to 5 minutes or more depending on the desired film thickness. Following this step, the particles are separated from the vehicle by filtering if a consistent size particle is desired or centrifuged or by any other acceptable conventional process. If a non-coacervate based matrix is desired, the following method is used (See method for inhalation composition.) The film in such case will be coacervate based.

As noted previously, this invention provides for modifications of the basic formulation. Such modifications produce medically useful compositions which can be used either transdermally or transmucosally. These formulations involve processing the preferred oral composition to produce a gel.

When applied transmucosally, (i.e., intranasally or rectally), the gel composition of this invention adsorbs onto the mucous or rectal membranes and is absorbed therein through the mucosal tissue to the blood stream. The active component is released from the gel and is absorbed by the membranes, passing into the blood stream by way of the capillaries. The gel composition can be prepared in forms that provide for immediate release, sustained release or any combination thereof. The quantity of drug incorporated can be varied according to medical requirements. Only those active components which are intrinsically irritating to the nasal or rectal membranes are not desirable for incorporation in the gel.

Preparation of the gel can be accomplished, as an example, by mixing additional albumin, or pectin or combinations thereof into the oral formulations. Accordingly, hydrocolloids, particularly bioadhesive hydrocolloids, such as albumin and pectin, are added after the oral formulation has been completed to formulate the gel.

The gel composition can incorporate non polar drugs. These may be further subclassified as drugs or other physiologically-active compound(s) which are heat sensitive and drugs or other physiologically-active compound(s) which are heat stable. Thus, erythromycin is an example of a heat stable composition; insulin is regarded as a heat sensitive composition. In the process of this invention, heat stable drug preparations can be "fixed", i.e., the encapsulating film can be hardened or structured in the manner described. On the other hand, heat sensitive drug preparations can be structured by the application of physical techniques such as radiation or physicochemical means, such as n-butyl alcohol which form a coacervate and then an encapsulating film of the composition.

The process to prepare the gel is based on any of the oral preparations described above. It is preferred however, to use the above-described example method incorporating the mono and di glyceride components. More specifically, warm a mixture of 7.5 gms of monoglyceride and 7.5 gms of diglyceride until a clear solution of the mixture is produced.

Unequal proportions of these glycerides may be used, but are not preferred. Equal or unequal proportions of mono-, di- and triglycerides also may be used. However, this mixture is also not preferred. When the warming step is completed the pharmaceutical component is mixed into the solution. The solution then is subjected to a spray drying step while the product is gradually cooled. The product resulting from this step will be a powder comprising particles containing the pharmaceutical component.

The next step is designed to coat the particles with a coacervate film. The procedure is as follows. Make a suspension of the powder referred to above in 100 mls of water containing 10 gms of polymerized albumin and 10 gms of monomeric lecithin. Stir the composition for about two minutes. Add an appropriate alcohol dropwise, n-butyl alcohol is preferred, until a coacervate forms and coats the particles with a coacervate film, stirring during the addition of alcohol and afterward to insure that coacervate encapsulation of the particles continues for from 1/2 to 3 hours. The product is then stored for from 12 to 24 hours at 4° C.

Following this step, the composition is processed through centrifugation, sieving or other appropriate separatory techniques to separate the coacervate drug particles from its vehicle and to retain particles having a gel-like surface. Following this step, a bioadhesive hydrocolloid, such as albumin or pectin or any appropriate combination thereof, is mixed into the composition. The quantity of albumin that may be used ranges from 2% w/v to 20% w/v; the range of pectin that may be used also ranges from 2% w/v to 20% w/v. 10% w/v of pectin is preferred. The pH of the preparation is adjusted to 7.4 by the addition of HCl or NaOH, as required.

In the case of heat unstable drugs, and the like, the process described above is modified. Following the coating step the composition is stored at room temperature for about 24 to 72 hours. After this period, the composition may be used or the composition may be heated at from 40 to 50° C until the particles assume a soft gel-like character. It is preferred that following the coating steps the preparation can be processed by the dropwise addition of a suitable protein denaturing agent, such as ethyl alcohol, n-butyl alcohol, isopropyl alcohol, and the like, until the structure of the interfaces of the particles range from soft gel-like to semi-solid. N-butyl alcohol is preferred.

If desired, a sustained release effect for this composition is achieved by subjecting portions of the product to heat, a cross-linking agent or radiation or combinations thereof. Heat is preferred. The following procedure is provided as an example. 20% of the composition is not heat treated. 20% is heated at 70° C for 40 seconds; 20% is heated at 65° C for 60 seconds and the remaining 20% is heated at 60° C for 90 seconds. The point to be made is that the exact combination of heat and time depends upon the specific drug being used and the desired schedule of drug release.

Microparticles comprising the transmucosal products can range from less than 1 µm (micron) to 6 µm (microns) or more in size. Particles less than 1 µm (micron) in size are preferred. The quantity of drug or other active agent to be incorporated into the composition is determined by medical considerations and can range from 1/2 of the convention oral or intravenous dose to four times the conventional dose.

After the composition is produced, e.g., normal or sustained release or combinations of normal and sustained release, the composition is placed in any dosage form suitable for nasal or rectal administration.

The process used to produce the transmucosal gel is used to produce the transdermal gel except that the adjustment of the pH is not required. In this embodiment, the drug or combination of drugs is sequestered or otherwise incorporated in a coacervate-based composition comprised of microencapsulated particles which range from semi-liquid to gel-like in structure. The composition(s) may be applied directly to the skin in such forms as ointments or gels and incorporated in a cellulose based patch or a suitable container. In the preferred mode, the composition is placed in a container and used in conjunction with an appropriate sonifier. By this technology an unexpected degree of drug absorption by the skin is achieved. Moreover, the transdermal gel and sonifier permits any scheduled administration or emergency administration as required.

While transdermal methods of drug administration are known, the literature does not reveal a technique in which a coacervate-based microencapsulated composition is used in conjunction with a sonifier. In this embodiment of the invention, use of a sonifier produces a local cavitational effect in the skin and in underlying tissue fluids, the net effect of which is to make the skin more permeable to the drug(s) being administered and to make the drug more available to the underlying tissues, including the capillaries and microcirculation. The cavitational effect is induced when sonification is applied and is ended when sonification is terminated. The application of the sonifier permits intermittent increases in skin permeability to administered drugs as required.

In the preferred practice of this invention, the finished product, e.g., the coacervate-based gel containing the medication required for either single or multiple doses, is placed in a suitable, leak proof container approximately the size of a wrist watch. The bottom of the container should be made of a semi-porous or microporous support layer, e.g., a cellulose layer. The sides and top of the container should be made of plastic or other suitable non toxic material. The top of the container should have an opening large enough to contain the head of a hand held sonifier such as NOVOPHON (manufacturer: Elredge Resources). Any suitable sonifier presently used for physical therapy can be used in accordance with this embodiment of the present invention. The container should be constructed such that a thin layer of either aluminum foil or plastic or other composition suitable to transmit the sonifier effects to the medication and the skin is placed between the medication and the sonifier head. The medication is administered transdermally, as required by placing the head of the sonifier in the container and activating the sonifier for the time necessary to administer the prescribed dose of medication.

Presently available transdermal patches are characterized by a continuous straight line release of the drug. This has been shown to be problematic with drugs such as nitroglycerin. In addition, such release is inconsistent with the body's normal release of hormones, enzymes and the like. Thus, by way of illustration, the body normally secretes very small quantities of insulin throughout the day. Upon ingestion of food, however, a surge of insulin is released. This invention permits a virtual duplication of such release phenomena not only for endogenous compositions but for medically useful exogenous compounds as well. In addition, this invention enables transdermal administration of medication according to any medically prescribed schedule.

This invention differs importantly from Zeffarone (U.S. patent No. 4,557,723) in that in his techniques a rate controlling membrane is used and is limited to drugs which show natural diffusive flux. No such features and limitations are present in this embodiment of the present invention. Ariura (U.S. patent No. 4,474,570) uses iontophoresis as the driving force. However, the Ariura device produces an unacceptable degree of heat in its operation. The present invention does not generate unacceptable heat; its driving force not only differs but its effect upon the skin differs from that of iontophoresis. The U.S. patent of Sibalis (4,557,723) describes an electrophoretic technology but also identifies the problems with known transdermal delivery devices. The Sibalis technology is fundamentally unrelated to the transdermal-sonifier embodiment of the present invention. Sibalis relies upon battery supplied electrical current which battery must be changed according to different flow requirements. Sibalis simply refers to a medicament. In the Ecanow patent, medications are encapsulated in coacervate-based films. The herein described use of localized sound waves as opposed to electrical current is an unobvious modification in that in the herein disclosed use, sound waves produce an effect on the skin and tissue fluids of the site that renders the site more permeable to drugs within the coacervate compositions. Sibalis and others who have described transdermal inventions do not disclose such properties.

Embodiments of the disclosed transdermal composition may be comprised of a matrix of hydrated hydrophilic colloids of which coacervate compositions comprise one class of such colloids. Gels constitute another class. Other compositions that could be used to provide the substrate for the medicament in this invention include any gelled silicon media, petrolatum and the like. In this invention, a gel based upon any acceptable coacervate formulation is preferred. Any of the described formulations which eventuate as oral or parenteral products of this disclosure can constitute the basis for preparing the transdermal product. Medications that can be incorporated in the gel for transdermal administration include enzymes, biologicals, drugs and other useful medicaments. In short, any medically useful molecule capable of penetrating the skin may be used in this invention. Use of the sonifier in conjunction with this composition acts in an unexpected manner to increase the rate of transdermal passage.

The method to produce a coacervate-based inhalant composition, as another embodiment of the present invention for administration of any suitable medication is as follows:

A method to prepare an inhalant medication is as follows:
Heat a mixture of 7.5 gms of monoglyceride and 7.5 gms of diglyceride at a temperature of from 70 to 75° C until a clear solution of the mixture is produced. Mix the desired amount of the pharmaceutical component into the solution. Next, process the product by means of a spray drying step to produce a powder form of the product. Next, make a suspension of the powder in 100 mls of water containing 10 gms of polymerized albumin and 10 gms of monomeric lecithin. Stir the composition for about two minutes. Add an appropriate alcohol dropwise, n-butyl alcohol is preferred. During the addition of alcohol the product should be stirred gently until a coacervate forms and coats the particles with a coacervate film, to insure that coacervate encapsulation of the particles continues for from 1/2 to 3 hours. After this step, adjust the pH to 7.4 and if desired, and store for from 12 to 24 hours at 4° C.

Following the storage step, the particles are separated from the liquid vehicle by filtration or other appropriate means. The particles are processed using a colloid mill and/or a microfluidizer and sieving or other filtering procedures to produce particles which are 1 µm (micron) or less in size. After this step, the quantity of particles necessary to produce the required dose is mixed into any appropriate fluid, such as the liquid vehicle separated from the particles. The product comprises an inhalant medication and is administered by nebulizer or other mist spraying apparatus.

The examples which follow are representative embodiments. They do not constitute the entire range of medications that can be used in this invention.

### EXAMPLE 1

To 100 mls of a 10% w/v solution of polymerized albumin add 10 gms of stroma-free hemoglobin from any appropriate source. Next, add 2 millimoles of glutathione and 8 gms of monomeric lecithin to the composition. Using a colloid mill and/or a microfluidizer, process the product for approximately 2 minutes. During this processing step add 10% of 1 ethyl-3 dimethyl aminopropyl carbodiimide (EDC). Following this processing step store the composition for 12 hours at 4° C. The pH of the composition then is adjusted through the use of HCl or NaOH to approximate the pH of normal whole blood. This adjustment is made prior to the step of colloid mill and/or a microfluidizer processing and after the period of storage. Isotonicity also is adjusted by adding plasma electrolytes in such concentration as will approximate the isotonicity of whole human blood. This step is taken following the period of storage. Adjustment of viscosity to approximate that of whole human blood is made after the period of storage. At this point, the product may be used as a red blood cell substitute, provided that it is filtered to eliminate all extraneous matter and all particles larger than 1 µm (micron) in size. It is preferred that the product be processed further using a heating step designed to harden the coacervate-based film that envelopes the particle(s) containing the hemoglobin component. In the heating step, the composition is heated for 2 minutes at 70° C. Following this step the composition is filtered to eliminate all extraneous matter and all particles larger than 1 µm (micron) in size. Particles of 1 µm (micron) or less then can be put into an appropriate physiological fluid and transfused or stored at 4° C and reconstituted as a red blood cell substitute (resuscitation fluid) using an appropriate physiological fluid.

### EXAMPLE 2

The procedure of Example 1 is followed except that polymerized lecithin is used in place of monomeric lecithin.

### EXAMPLE 3

The procedure of Examine 2 is followed except that monomeric albumin is used in place of polymerized albumin.

### EXAMPLE 4

The procedure of Example 1 is followed but, in addition, the encapsulated microparticles are subjected to a lyophilizing process to produce a powder form of the composition that can be mixed into an appropriate physiologic fluid as needed to produce a red blood cell substitute.

### EXAMPLE 5

The procedure of Example 1 is followed except that 10% w/v of modified hemoglobin is used in place of stroma-free hemoglobin and 0.1% urea is added.

### EXAMPLE 6

The procedure of Example 1 is followed except that the heating step consists of heating the composition at 50° C for 1 minute.

### EXAMPLE 7

The procedure of Example 1 is followed except that stroma-free hemoglobin encapsulated in liposomes comprises the hemoglobin component.

### EXAMPLE 8

Heat a mixture of 7.5 gms of monoglycerize and 7.5 gms of diglyceride at a temperature of from 70 to 75° C until a clear solution of the mixture is produced. Mix 15 gms of erythromycin into the glyceride solution thoroughly. The objective of the next step is to convert the composition to a powder form. This may be accomplished by any conventional form such as spray drying. In this instance the warm product comprised of the glyceride components and the erythromycin is gradually cooled to give a powder. The particle size of the product following the spray drying will range from about 2 µm (microns) to 500 µm (microns). The finished product may consist of a single size or combination of sizes.

The next step is designed to coat the particles with a coacervate film. The procedure is as follows. Make a suspension of the powder referred to above in 100 mls of water containing 10 gms of polymerized albumin and 10 gms of monomeric lecithin. Stir the composition for two minutes. Add an appropriate alcohol dropwise, n-butyl alcohol is preferred, until a coacervate forms and coats the particles with a coacervate film, stirring during the addition of alcohol and afterward to insure that coacervate encapsulation of the particles continues for from 1/2 to 3 hours. The product is then stored for from 12 to 24 hours at 4° C.

Following the storage step, the particles are separated from the liquid vehicle by filtration or other appropriate means and dried using any conventional method. This yields a finished oral product of a coacervate coated pharmaceutically-active component, coated with the coacervate-based film. The product can be used to prepare capsules or reconstituted with an appropriate liquid(s), e.g., syrup, to produce a product with the desired clinical dose.

### EXAMPLE 9

The procedure of Example 8 is followed, but in addition, the encapsulated microparticles are subjected to a lyophilizing process before prepared as capsules, tablets, or liquid preparations.

### EXAMPLE 10

The process of Example 9 is followed except the product is divided into four portions. Portion 1 is heated at 50° C for 2 minutes; portion 2 is heated at 60° C for 2 minutes; portion 3 is heated at 65° C for 2 minutes; portion 4 is heated at 70° C for 1-1/2 minutes. After the heating step, the products are combined thereby forming a sustained release composition.

### EXAMPLE 11

The process of Example 9 is followed except that the finished product of Example 9 is processed further by adding an additional coacervate (film) coat to the product as per the process of Example 9. The resulting composition comprises a sustained release composition with two release rates.

### EXAMPLE 12

The process of Example 11 is followed except that 20% of the finished product is processed to receive an additional coacervate (film) coat, two additional coats (films) are applied to 30% of the product, and 50% of the product comprises the original finished product of Example 11. The products are mixed together thereby comprising a composition with sustained release characteristics, i.e., three release rates.

### EXAMPLE 13

The procedure of Example 8 is followed except that regular insulin is used in place of erythromycin and in a quantity that will yield a finished oral composition containing 40 units of insulin per dose.

### EXAMPLE 14

The procedure of Example 13 is followed except that regular insulin incorporated in liposomes is used in as the active component.

### EXAMPLE 15

The procedure of Example 8 is followed, except that urokinase in an amount that will give unit dose of 50,000 IU is used in place of erythromycin and the encapsulated microparticles are subjected to a lyophilizing process to produce a powder which can be placed in capsules for oral use or used to reconstitute a liquid dosage form by using an appropriate liquid.

### EXAMPLE 16

The procedure of Example 8 is followed except that 15 gms of erythromycin encapsulated in liposomes is used..

### EXAMPLE 17

The procedure of Example 8 is followed except that lidocaine is used in place of erythromycin and in a quantity that will yield a finished composition containing 1% lidocaine/5 ml per unit dose.

### EXAMPLE 18

The procedure of Example 8 is followed except that 500 mgs of flourourocil encapsulated in liposomes is used as the active component.

### EXAMPLE 19

The procedure of Example 11 is followed except that following the application of the additional coacervate coating to the product the composition then is heated at 70° C for 60 seconds.

### EXAMPLE 20

The procedure of Example 8 is followed except that erythromycin is replaced with urokinase in an amount that will yield 50,000 IU of urokinase per unit dose.

### EXAMPLE 21

The procedure of Example 20 is followed except that the urokinase component is encapsulated in liposomes.

### EXAMPLE 22

This process consists of the following steps: prepare polymerized albumin by adding 1 ethyl-3-dimethyl-aminopropyl-carbodiimide to a 10% solution of monomeric albumin in an amount sufficient to produce a concentration of 5.0% w/v EDC in the product. Process in a colloid mill and/or a microfluidizer for about 2 minutes. Next, store the product at about 4° C for about 10 hours. Mix into the product a solution of stroma-free hemoglobin to a concentration of about 10% by weight of the composition and about 2 millimoles of NADPH into the product and process in a colloid mill and/or a microfluidizer for about 5 minutes; following this step, mix into the solution polymerized lecithin in an amount sufficient to provide the solution with a concentration of polymerized lecithin of about 8% by weight into the product, and process again in a colloid mill and/or a microfluidizer for about 3 to 5 minutes. Next, adjust the pH of the product to that of whole blood using HCl or NaOH as necessary. Following this step, adjust the isotonicity using plasma electrolytes in such concentration as will give the product the isotonicity of whole human blood. Adjust the viscosity as the next step so that the viscosity approximates that of whole blood. At this stage of manufacture the product may be used as a red blood cell substitute (resuscitation fluid) provided that it is first filtered to remove all extraneous matter and all particles that exceed 1 micron in size in any dimension. It is preferred, however, that the filtered product be processed further using either a chemical process based on a non-toxic crosslinking agent, such as gluteraldehyde, or a physical processing step, such as heat. The heating step is preferred and consists of heating the preparation for 2 minutes at a temperature that may range from 50° C to 70° C producing a film that can range from gel-like to rigid in character. Following this step, the resulting composition is filtered to remove all extraneous matter and any microencapsulated particles that exceed 1 micron in size in any dimension. The finished product of this invention can consist of particles of 1 µm (micron) or less in size with a minimal degree of surface film hardness or, alternatively, it can consist of particles of mixed surface film hardnesses. The desired quantity of the particles can be added to any appropriate physiological fluid. As such, it will then comprise a product suitable for intravenous administration as a red blood cell substitute (resuscitation fluid). If desired, the microencapsulated particles may be used in much the same manner as packed red blood cells. If desired, the product may be lyophilized or otherwise dried and reconstituted as a resuscitation fluid with any appropriate physiological fluid.

### EXAMPLE 23

The procedure of Example 22 is followed except that polymerized lecithin is used in place of monomeric lecithin.

### EXAMPLE 24

The procedure of Example 22 is followed except that stroma-free hemoglobin incorporated in liposomes is used in place of stroma-free hemoglobin.

### EXAMPLE 25

The procedure of Example 22 is followed but in addition, the encapsulated microparticles are subjected to a lyophilizing process to produce a powder form of the composition that can be mixed into an appropriate physiological fluid to produce a red blood cell substitute.

### EXAMPLE 26

The procedure of Example 22 is followed except that 10% w/v of modified hemoglobin is used in place of stroma-free hemoglobin.

### EXAMPLE 27

The procedure of Example 22 is followed except that the heating step consists of heating the composition at 50° C for 2 minutes.

### EXAMPLE 28

The procedure of Example 8 is followed, except that erythromycin is replaced with heparin in an amount that will give 1000 USP units/ml in the finished product; the composition is subjected to a lyphilizing process to produce a powder form of the composition which can be prepared as tablets or capsules or reconstituted using any appropriate pharmaceutical fluid thereby comprising a useful oral medication.

### EXAMPLE 29

The procedure of Example 1 is followed except that (1) 5 gms of erythromycin replaces the hemoglobin and (2) the steps involving glutathione and the addition of 1-ethyl-3 dimethyl aminopropyl-carbodiimide are omitted. The step to adjust the pH of the composition to approximately 7.4 is made after the storage period. After the heating and filtering steps, the microparticles are dispersed in any appropriate physiological fluid. The product then is ready for parenteral administration or stored, preferably under refrigeration, until needed.

### EXAMPLE 30

The procedure of Example 29 is followed except that urokinase is used in place of erythromycin in a quantity that will yield a finished composition containing 50,000 IU of urokinase per unit dose.

### EXAMPLE 31

The procedure of Example 29 is followed except that 500 mgs of flourouracil is substituted for erythromycin and the sizes of the microparticles of the finished product range from less than 1 µm (micron) to 4 µm (microns).

### EXAMPLE 32

The procedure of Example 1 is followed except that 10% w/v of erythropoietin is used in place of hemoglobin, and the steps involving the use of glutathione and 1 ethyl-3-dimethyl aminopropyl carbodiimide are omitted. The finished product comprises a useful intravenous composition of erythropoietin. The product can be lyophilized to produce a powder form of the composition and stored under refrigeration or reconstituted with an appropriate physiological fluid for intravenous administration.

### EXAMPLE 33

The procedure of Example 22 is followed except that 10% w/v of modified hemoglobin is used in place of stroma-free hemoglobin.

### EXAMPLE 34

The procedure of Example 31 is followed except that the flourouracil is encapsulated in microparticles of 2 to 4 µm (microns) in size. This product is combined with the erythromycin product of Example 29; the mixture comprising an intravenous composition composed of two active components with differing particle sizes, eryrhtomycin 1 µm (micron) or less; flourouracil 2-4 µm (microns).

### EXAMPLE 35

The procedure of Example 22 is followed, except that the stroma-free hemoglobin encapsulated in liposomes is used as the starting hemoglobin component.

### EXAMPLE 36

The procedure of Example 22 is followed but, in addition, the encapsulated microparticles are subjected to a lyophilizing process to produce a powder form of the composition that can be stored at 4° C or reconstituted with an appropriate physiologic fluid to produce a red blood cell substitute (intravenous).

### EXAMPLE 37

The procedure of Example 31 is followed except that the erythromycin and flourourocil incorporated in liposomes are used as the active components.

### EXAMPLE 38

The procedure of Example 22 is followed except that 275 mgs of erythromycin is used in place of the hemoglobin component and the steps involving the use of NADPH and 1 ethyl-3-dimethyl-aminopropyl carbodiimide are omitted. The step in which the pH is adjusted to approximately 7.4. is made after the storage period. After the heating and filtering steps, the microparticles are dispersed in any appropriate physiological fluid. The product is then ready for intravenous administration or it may be stored, preferably at 4° C.

### EXAMPLE 39

The procedure of Example 38 is followed except that leuprolide in a quantity that will yield leuprolide mg/0.2 ml in the finished product is substituted for erythromycin.

### EXAMPLE 40

The procedure of Example 39 is followed, except that urokinase in that quantity as will give 50,000 IU activity per dose in the finished product is used in place of leuprolide and the product is lyophilized. The product can be reconstituted with an appropriate physiological fluid for intravenous use.

### EXAMPLE 41

The procedure of Example 40 is followed except that 60,000 IU of urokinase incorporated in liposomes, is used as the active component.

### EXAMPLE 42

The procedure of Example 40 is followed except that 500 mgs of flourouracil replaces urokinase and is processed so that the particle size of the particles ranges from 2-4 µm (microns) and the product of Example 38 is mixed with the product of this Example. This comprises an Example of a combination of two compositions, each with a different particle size.

### EXAMPLE 43

Mix to a concentration of 10% w/v stroma-free hemoglobin into a solution of polymerized lecithin of about 20% concentration. Process the product in a colloid mill and/or a microfluidizer for 2-3 minutes. Mix ascorbic acid of at least 0.1 gm per 100 ml of solution and an amount equal to 10% w/v (100 units/cc. in the finished product) of polymerized albumin into the product and process the resulting product in a colloid mill and/or a microfluidizer for about 5-7 minutes. Next, store the product for 10 hours at 4° C. Adjust the pH of the preparation after the period of storage to 7.4 using HCl or NaOH as necessary. Following this step, add electrolytes present in human plasma in such amount as will give the product the isotonicity of whole human blood. Next, adjust the viscosity of the product so that it approximates the viscosity of whole human blood. On completion of this step, the product comprises a microemulsion that consists of microencapsulated particles containing the hemoglobin component. At this stage of manufacture the product may be used as a red blood cell substitute (resuscitation fluid) provided that it is first filtered to remove all extraneous matter and all particles that exceed 1 µm (micron) in size in any dimension. It is preferred, however, that the filtered product be heated for 2 minutes at 70° C. The product then is filtered to remove all extraneous matter and all microencapsulated particles which exceed 1 micron in any single dimension. After this step, the product may be used as an intravenous blood substitute or stored, preferably at 4° C, until needed.

### EXAMPLE 44

The procedure of Example 43 is followed except that stroma-free hemoglobin incorporated in liposomes is used in place of stroma-free hemoglobin.

### EXAMPLE 45

The procedure of Example 43 is followed but in addition, the encapsulated microparticles are subjected to a lyophilizing process to produce a powder form of the composition that can be mixed into an appropriate physiological fluid to produce a red blood cell substitute.

### EXAMPLE 46

The procedure of Example 43 is followed except that 10% w/v of modified hemoglobin is used in place of stroma-free hemoglobin.

### EXAMPLE 47

The procedure of Example 43 is followed except that the heating step consists of heating the composition at 50° C for 2 minutes.

### EXAMPLE 48

The procedure of Example 43 is followed except that (1) 275 mgs of erythromycin replaces the hemoglobin component. The step in which the pH is adjusted to approximately 7.4. is made after the storage period. After the heating and filtering steps, the resulting microparticles are dispersed in any appropriate physiological fluid. The product then is ready for intravenous administration or storage at 4° C.

### EXAMPLE 49

The procedure of Example 43 is followed, but in addition, the composition is lyophilized to produce a powder form of the product which can be reconstituted with any appropriate physiological fluid.

### EXAMPLE 50

The procedure of Example 48 is followed, except that 500 mgs of flourouracil is used in place of erythromycin and the sizes of the microparticles are in the range of 2 to 4 microns.

### EXAMPLE 51

The procedure of Example 50 is followed except that the flourouracil is encapsulated in liposomes before being encapsulated within the coacervate composition.

### EXAMPLE 52

The procedure of Example 48 is followed except that leuprolide in a quantity that will yield leuprolide mg/0.2 ml in the finished product is substituted for erythromycin.

### EXAMPLE 53

The procedure of Example 8 is followed except that 200 mgs. of ibuprofen is used in place of erythromycin.

### EXAMPLE 54

The procedure of Example 43 is followed except that 10% w/v of erythropoietin is used in place of hemoglobin and the step involving the use of ascorbic acid is omitted. The finished product comprises a useful intravenous composition of erythropoietin.

### EXAMPLE 55

The product of Example 48 is mixed with the product of Example 50 to produce a combination of two compositions with differing particle sizes.

### EXAMPLE 56

The procedure of Example 48 is followed except that leuprolide is used in place of erythromycin, and in an amount that will give 4 mgs/0.2 ml of leuprolide in the finished product.

### EXAMPLE 57

The procedure of Example 48 is followed except that erythromycin is replaced with urokinase in an amount that will give 50,000 IU per dose in the finished product.

### EXAMPLE 58

The procedure of Example 56 is followed, except that the leuprolide component is encapsulated in liposomes.

### EXAMPLE 59

The procedure of Example 8 is followed except that after the addition of n-butyl alcohol and the stirring step, 10% w/v of pectin is mixed into the product now comprised of the microparticles separated from the vehicle. After the step in which pectin is added, the pH of the composition is also adjusted using either HCl or NaOH as required to 7.4. The product comprises a composition that can be administered transmucosally and/or transdermally.

### EXAMPLE 60

The process of Example 59 is followed except that 5% nitroglycerin is used to replace erythromycin and 5% w/v of albumin is used in place of pectin. The finished product comprises a transmucosal medication.

### EXAMPLE 61

The process of Example 59 is followed except that 5 mg/ml of lidocaine replaces the nitroglycerin.

### EXAMPLE 62

The procedure of Example 59 is followed except that a heating step of about 50° C for 40 seconds replaces the step using n-butyl alcohol. Following heating, the product is processed to separate the microparticles from the vehicle. 10% w/v pectin is mixed into the resulting microparticle composition. During the step in which the pectin is added, the pH of the product is adjusted to about 7.4 using either HCl or NaOH as required.

### EXAMPLE 63

The procedure of Example 59 is followed except that 5% nitroglycerin is used in place of erythromycin; storage of the composition at room temperature for about 24 hours replaces the heating step.

### EXAMPLE 64

The procedure of Example 59 is followed except that 5% w/v albumin replaces pectin and 10% nitroglycerin is used.

### EXAMPLE 65

The process of Example 59 is followed except that 5% nitroglycerin is used to replace erythromycin and 5% w/v of albumin is used in place of pectin. The finished product comprises a transmucosal medication.

### EXAMPLE 66

The process of Example 65 is followed except that 5 mg/ml of lidocaine replaces the nitroglycerin.

### EXAMPLE 67

The process of Example 66 is used except that 100 units of regular insulin is used instead of lidocaine.

### EXAMPLE 68

The process of Example 59 is followed except that 0.1% ascorbic acid is added at the same time pectin is added.

### EXAMPLE 69

The process of Example 62 is used except that 100 units of insulin is used instead of erythromycin.

### EXAMPLE 70

The process of Example 62 is used except that 0.2% ascorbic acid is added when pectin is added.

### EXAMPLE 71

The procedure of Example 62 is followed except that 40 units of insulin is used in place of erythromycin; storage of the composition at room temperature for about 24 hours replaces the heating step.

### EXAMPLE 72

Mix 10 gms of lecithin into 10 cc of carbon tetrachloride. Add 5 gms of erythromycin to the solution. Spray dry the product until all traces of carbon tetrachloride are removed. The resulting product will be comprised of powder-like particles comprised of erythromycin and covered by a film of lecithin. Next, make a suspension of the powder in 100 mls of water containing 10 gms of polymerized albumin and 10 gms of monomeric lecithin. Stir the composition for about two minutes. Add an appropriate alcohol dropwise, n-butyl alcohol is preferred. During the addition of alcohol the product should be stirred gently until a coacervate forms and coats the particles with a coacervate film. Stirring continues for from 1/2 to 3 hours to insure coacervate encapsulation of the particles. After this step, adjust the pH to 7.4 and if desired, store for from 12 to 24 hours at 4° C.

Following the storage step, the particles are separated from the liquid vehicle by filtration or other appropriate means and dried using any conventional method. This yields a finished oral product comprised of coaceravate coated erythromycin. The product can be used to prepare tablets, capsules or reconstituted with an appropriate liquid(s), e.g., syrup, to produce a product with the desired clinical dose.

### EXAMPLE 73

Mix 10 gms of lecithin into 10 cc of carbon tetrachloride. Add 3 gms of erythromycin into the solution. Distill the product until all traces of the carbon tetrachloride are removed and the product, comprised of lecithin and erythromycin, is in the form of a viscous residue. Mix 50 mls of a 10% solution of albumin into the product. Add 100 mls of distilled water and centrifuge the composition for 10 minutes at 2500 rpm. The particles now separated from their vehicle are collected. They comprise an oral erythromycin coated by a coacervate film.

### EXAMPLE 74

The method of Example 73 is followed except that the steps that begin with the addition of the albumin component are repeated.

### EXAMPLE 75

The process of Example 72 is followed except that the product of Example 72 is converted to a sustained release preparation using the following method. Make a suspension of the powder referred to above in 100 mls of water containing 10 gms of polymerized albumin and 10 gms of monomeric lecithin. Stir the composition for two minutes. Add n-butyl alcohol dropwise until a coacervate forms and coats the particles with a coacervate film, stirring continues for from 1/2 to 3 hours during the addition of alcohol and afterward to insure coacervate encapsulation of the particles. The product is then stored for from 12 to 24 hours at 4° C.

Following the storage step, the particles are separated from the liquid vehicle by filtration and dried. The resulting composition comprises a sustained release medication useful for oral administration.

### EXAMPLE 76

The process of Example 74 is followed except that the product of Example 74 is converted to a sustained release preparation using the following method. Make a suspension of the powder referred to above in 100 mls of water containing 10 gms of polymerized albumin and 10 gms of monomeric lecithin. Stir the composition for two minutes. Add n-butyl alcohol dropwise until a coacervate forms and coats the particles with a coacervate film, stirring continues for from 1/2 to 3 hours during the addition of alcohol and afterward to insure coacervate encapsulation of the particles. The product is then stored for from 12 to 24 hours at 4° C.

Following the storage step, the particles are separated from its liquid vehicle by filtration and dried. The resulting composition comprises a sustained release medication useful for oral administration.

### EXAMPLE 77

Mix 6 gms of lecithin in 200 mls of water; add about 24 mls of an appropriate alcohol such as n-butyl alcohol dropwise to the mixture. Next mix a solution comprised of 3 gms of NaCl and 60 mls of water into the lecithin-alcohol composition and allow to stand until a two phase coacervate is produced. Separate the two phases and add 20% acetylcysteine to the coacervate phase. Mix thoroughly. The pH is adjusted to 8.0 after which the composition containing the medication may be administered by nebulizer which sprays a mist of the particles having a particle size of 1 µm (micron) or less.

### EXAMPLE 78

The process of Example 77 is followed except that 5 gms of lecithin is added to the coacervate phase in place of acetylcysteine and the pH is adjusted to 7.4 using HCl or HaOH as required.

### EXAMPLE 79

The process of 77 is followed except that the lecithin-coacervate phase, per se, is adjusted to a pH of 7.4 and used as an inhalant medication.

### EXAMPLE 80

Heat a mixture of 7.5 gms of monoglyceride and 7.5 gms of diglyceride at a temperature of from 70 to 75° C until a clear solution of the mixture is produced. Mix 15 gms of erythromycin into the glyceride solution thoroughly. Next, process the product by means of a spray drying step to produce a powder form of the product. Next, Make a suspension of the powder in 100 mls of water containing 10 gms of polymerized albumin and 10 gms of monomeric lecithin. Stir the composition for about two minutes. Add n-butyl alcohol dropwise until a coacervate forms and coats the particles with a coacervate film, stirring for from 1/2 to 3 hours during the addition of alcohol and afterward to insure coacervate encapsulation of the particles. The product is then stored for from 12 to 24 hours at 4° C.

Following the storage step, the particles are separated from the liquid vehicle by filtration or other appropriate means. The particles are processed using a colloid mill and/or a microfluidizer and sieving procedures to produce particles which are 1 µm (micron) or less in size. After this step, the quantity of particles necessary to produce the required dose is mixed into any appropriate fluid including the liquid vehicle referred to above. The product comprises an inhalant medication and is administered by nebulizer or other mist spraying apparatus.

### EXAMPLE 81

The method of Example 77 is followed except that 15 gms of erythromycin is used in place of acetylcysteine and the pH adjustment is made to 7.4.

### EXAMPLE 82

The procedure of Example 1 is followed except that (1) 275 mgs of erythromycin is dispersed in oleic acid of 10% w/v as the first step of the process and the step using the reducing agent is omitted. The step to adjust the pH of the composition to approximately 7.4. is made after the storage period. After the heating and filtering steps, the microparticles are dispersed in any appropriate physiological fluid. The product is then ready for oral administration or stored, preferably under refrigeration, until needed. The composition may be administered orally, if desired.

### EXAMPLE 83

The procedure of Example 82 is followed except that the object of the heating step in this method is to produce microparticles which are gel-like in character. The composition is heated at about 60° C for about 45 seconds. The resulting composition then is centrifuged to separate the particles from the vehicle of the product; following this step, about 10% w/v of pectin and about 0.1% of ascorbic acid are mixed into the product now comprised of the microparticles separated from the vehicle. During the steps in which pectin and ascorbic acid are added, the pH of the composition is also adjusted using either HCl or NaOH as required to about 7.4. The product comprises a nasally or rectally administered medically useful composition. The addition of ascorbic acid can be omitted.

### EXAMPLE 84

The procedure of Example 83 is followed except that in place of the heating step, the product is stored at room temperature for about 24 hours and 5% nitroglycerin is used in place of erythromycin.

### EXAMPLE 85

The procedure of Example 84 is followed. The finished product however is applied to the skin. The drug is released from the composition and enters the circulation through the skin.

### EXAMPLE 86

The procedure of Example 84 is followed except that 4000 mcg of vitamin B-12 is used in place of erythromycin.

### EXAMPLE 87

The procedure of Example 84 is followed except that 4 mgs/0.2 ml of leuprolide is used in place of erythromycin.

### EXAMPLE 88

The procedure of Example 87 is followed except that the composition is administered rectally either in the form of an ointment or a suppository.

### EXAMPLE 89

The procedure of Example 86is followed except that the composition is applied to the skin, thereby comprising a transdermal preparation.

### EXAMPLE 90

The procedure of Example 82 is followed with the following exceptions: The heating step consists of heating the composition for 45 seconds at about 50° C and the adjustment of the pH takes place at a different point in the manufacturing process. Following the heating step, the microparticles are separated from the vehicle and 10% pectin and 0.1% ascorbic acid are mixed into the composition comprised of the microparticles. During this step, the pH of the product is adjusted to 7.4 using HCl or NaOH as required. The finished product comprises a nasally or rectally administered medically useful composition.

### EXAMPLE 91

The procedure of Example 90 is followed except that 5 mg/ml of lidocaine is used in place of erythromycin.

### EXAMPLE 92

The procedure of Example 91 is followed except that the composition is applied to the skin, thereby comprising a transdermal preparation.

### EXAMPLE 93

The process of Example 83 is followed except that 10 mgs/ml of ephredrine is used in place of erythromycin.

### EXAMPLE 94

The procedure of Example 93 is followed except that the preparation is applied to the skin thereby comprising a transdermal preparation.

In accordance with another important embodiment of the method used to prepare both oral and parenteral compositions, the drug-containing coacervate compositions are prepared by (a) dissolving a phospholipid in water and adding thereto an alcohol in an amount which will cause the phospholipid-water-alcohol preparation to separate into three immiscible layers wherein the lowest layer is colloid-poor, the middle layer is colloid-rich, and the upper layer is alcohol; (b) separating the middle phase, the nonpolar, colloid-rich phase, from the other two phases; and (c) adding a water-insoluble drug to a colloid-rich phase in such quantity as will produce a solution containing the drug component in the desired dosage for oral, tissue lining absorptive or parenteral administration. The colloid-poor lower phase may be used to solubilize water-soluble compounds. Either the colloid-rich or colloid-poor phases or a combination of both phases may be used to encase the physiologically active compounds. Additionally, the drug delivery compositions of the present invention can be prepared in the form of microencapsulated sustained release formulations (emulsions) of orally, tissue lining absorptive, or parenterally administrable drugs. The microencapsulated particles range in size from less than abut 100 nanometers to about 3 µm (microns) or larger. In the practice of the time-release embodiment of the present invention, the final composition or product may be composed of particles of the same or different sizes, with differing time-release characteristics.

In accordance with the preferred method of this embodiment of the present invention, an alcohol, such as isopropyl alcohol, is added dropwise to a 4% w/v aqueous solution of one or more surface active agents, such as albumin, until a two-phase liquid aqueous system is produced. The two phases are separated. The upper phase is referred to as the colloid-rich phase; the lower phase known as the colloid-poor phase is discarded or stored for later use to prepare additional quantities of the coacervate composition. A 4% w/v quantity of dry lecithin powder is dissolved in the colloid-rich phase creating an albumin-lecithin based colloid-rich phase. The oral or parenteral drug to be used is dissolved in the colloid-rich phase as described with reference to the three-phase embodiment of manufacture. The finished composition of this embodiment of the present invention can be prepared either as an emulsion or as a suspension.

A critical distinction between the coacervate-based blood substitute compositions identified in prior patents and the compositions of this embodiment of the present invention is in the addition of an appropriate alcohol to a composition to form a two-phase coacervate composition with alcohol on top as an upper third layer or phase. The middle coacervate phase then is separated out and a phospholipid, such as lecithin, is added thereto. A second coacervate composition results therefrom and is used to formulate the coacervate compositions of this embodiment for new and unexpected stability. As an option, the middle coacervate phase can be used without the addition of the phospholipid.

In accordance with the principles of the present invention, any appropriate non-toxic coacervate composition can be used in the manufacture of the drug delivery compositions of the present invention. The coacervate-enveloped compositions of the present invention incorporate non-toxic endogenous or exogenous surface-active agents, derivatives thereof and/or combinations of surface-active agents or derivatives.

To achieve the full advantage of the alcohol embodiment of the present invention, the coacervate compositions include a phospholipid, such as lecithin; a protein with surface-active properties, such as albumin; an alcohol such as n-butyl alcohol and water.

Any drug, particularly drugs administered parenterally or absorbed through the tissue lining, drugs which are prepared and administered at an excessive pH, parenteral drugs that require pH adjustment immediately prior to injection and any salt of a parent drug compound which has been neutralized by means of the proper acid-base reaction to yield the parent compound may be used as the drug component in the orally, tissue lining absorbed, or parenterally administrable coacervate based film-enveloped compositions of the present invention.

It should be noted that while the compositions of the present invention stabilize and solubilize oral, tissue lining absorbed and parenteral drugs as described, some dissolution of the drug also occurs in accordance with the principles of the present invention.

Either the colloid-rich phase of the two-phase liquid aqueous coacervate composition, the colloid-poor phase, or a combination of the colloid-rich and the colloid-poor phases of the compositions may be used to produce the outer coacervate based film enveloping the physiologically active compounds as orally, tissue lining absorptive, or parenterally administrable compositions of the present invention. To achieve the full advantage of the present invention, the physiologically active compound(s) are incorporated into the colloid-rich phase of the composition. The colloid-poor phase can be used to incorporate and deliver formulations of water-soluble, polar and semi-polar drugs. Administration of the formulations of this invention may be oral; absorbed through the tissue lining of the mouth, nasal passages, or rectum; intravenous; intramuscular or subcutaneous, as desired.

Drug compositions commonly understood to be water-insoluble, as defined in the U.S. Pharmocopia, now can be solubilized in an aqueous media by use of the present drug delivery compositions. Further, the compositions of the present invention provide a basis for improving the manufacture and efficacy of literally hundreds of orally, tissue absorptive, and parenterally administered drugs.

A preferred two-phase liquid aqueous coacervate composition in accordance with the alcohol embodiment of the present invention is prepared using the following steps. To 100 mls of sterile, pyrogen-free water are added 1-6% w/v of lecithin, derived from egg or other acceptable sources. The mixture is mixed or shaken vigorously until the lecithin is thoroughly dissolved. 6 to 12 mls or more of any acceptable alcohol, particularly a lower alcohol having 1 to about 6 carbon atoms, such as ethyl alcohol, n-butyl alcohol, and the like then is added to the lecithin solution, and the solution then is shaken vigorously for from 10 to 30 seconds and set aside, undisturbed, for from less than 1 to more than 4 hours. Within this period of time, the solution will separate into three phases or layers. If at the end of two hours the phases have not separated out, additional alcohol may be added dropwise until the separation of phases occurs. If desired, the period of undisturbed storage may extend to 24 hours or longer. Longer periods of storage typically result in greater yields of the coacervate phase. At the close of the storage period, it will be observed that three layers have formed. The uppermost layer comprised of n-butyl alcohol is separated from the other phases such as by decanting or through a separating funnel, and set aside since it is now extraneous to the coacervate system. The middle layer comprises approximately 50 mls. of a colloid-rich coacervate phase and the bottom layer comprises approximately 50 mls of a colloid-poor (equilibrium water) phase. The proportions of the bottom and middle phases may vary from 0.5% to 99.5%, based on the volume of both layers.

After separation of the phases is completed, from 1 to 2 grams of a surface active protein such as human serum albumin, is dissolved in the colloid-rich coacervate phase of the solution to form a second coacervate system or composition. The preparation is then stored, preferably under refrigeration at 4-10° C, until visual inspection indicates that the preparation has separated into two phases. The upper layer comprises the colloid-rich phase of the second coacervate composition; the bottom layer comprises the colloid-poor phase of the second coacervate composition. The two phases can be separated, such as by decanting, and a desired dosage of a drug or other physiologically active compound is dissolved in either the colloid-rich phase and/or the colloid-poor phase to provide an injectable, orally, or tissue absorptive administrable composition. If desired, the quantity of drug combined into the coacervate composition may be more or less than a standard dose to provide a modified clinical dose. Additionally, if desired, urea in an amount that may range of 1% to 40% w/v may be added to the coacervate phase, prior to the addition of the drug component to make the coacervate phase less non-polar.

In accordance with another important embodiment of the present invention, a monoglyceride, diglyceride and/or a triglyceride is included in the coacervate composition as a liquid vehicle for dispersing or solubilizing one or more physiologically active compounds therein. The vehicle containing the physiologically active compound dissolved or dispersed therein then is enveloped in one or more exterior layers of a coacervate based film in order to provide new and unexpected stability to the orally, tissue absorptive or parenterally administrable compositions. As used herein, the term "triglycerides" refers to short chain, medium chain, long chain, and hydrogenated triglycerides. In this invention monoglycerides and diglycerides may be used as an option. Combinations of mono, di, and triglycerides also may be used. Medium chain triglycerides are preferred. In an optional process, physiologically acceptable halogenated compounds, particularly halogenated hydrocarbons may be substituted for the glyceride component of the compositions of the present invention. The preferred halogenated chemicals are the perfluorohydrocarbons, such as perfluorodecalin. Suitable other perfluorochemicals include perfluorotributylamine, perfluoro-N,N dimethylcyclohexylmethylamine, perfluorotripropylamine and perfluorotrimethylbicyclo(3.3.1)nonane. Other suitable halogenated hydrocarbons include halothane (2-bromo-2-chloro-1,1,1-trifluoroethane) and methoxyflurane(2,2-dichloro-1,1-difluoroethylmethylester).

All components may be prepared, combined and/or mixed under sterile conditions. The water used in manufacturing the coacervate system should be sterile and pyrogen-free for intravenous preparation.

In accordance with this embodiment, the pharmaceutical component or combinations thereof are dissolved in a glyceride, preferably a medium chain triglyceride, preferably 30% w/v, containing a pharmaceutical component solubilizing agent, such as a hydrogenated oil, oleic acid, or linoleic acid preferably 10% w/v, based on the weight of the triglyceride to substantially increase stability. The triglyceride may be 1% to 80% w/v of the composition and may be a monoglyceride or a diglyceride or mixtures of one or more mono, di, or triglycerides. The solubilizing agent may be added to the glyceride in an amount of about 0% to about 50% by weight of the glyceride, for example, 10% hydrogenated vegetable oil or oleic acid, whenever needed for complete solubilization of physiologically active components. The glyceride component may be composed of short chain (up to C9), medium chain (C9 to C18), long chain (above C18) and/or hydrogenated triglycerides or combinations thereof depending upon the composition to be prepared. Medium chain (C9 to C18) triglycerides are preferred. The quantity of the pharmaceutical component(s) mixed into the triglyceride may range from 0.1% to 15% in excess of conventional clinical doses. The individual dose of the pharmaceutical component(s) of the finished composition is adjusted as necessary to meet clinical requirements.

The preferred method of the triglyceride embodiment of the present invention also provides for preparation of sustained release forms of the microemulsions described above. This process involves the following steps: (1) Mix the desired quantity of the pharmaceutic component(s) into a solution containing that quantity of a medium chain triglyceride containing 10% hydrogenated oil as will comprise 30% w/v in the finished product; the w/v concentration of the triglyceride component may vary from 1% to 80% w/v; 30% w/v is preferred; the quantity of the pharmaceutic component(s) used can range from 0.01% to 15% in excess of established clinical doses; in the finished product, the individual dose is adjusted to clinical requirements. (2) Prepare a second solution comprising 5% w/v of albumin; mix the same pharmaceutic component(s) used in step 1 into the albumin solution in an amount that may range from 25% less than the established clinical dose to 15% in excess of the established clinical dose; 3% w/v of lecithin then is mixed into the product of step 1; this mixture is repeatedly passed through an appropriate colloid mill to product a microemulsion, the particles or droplets of which are one micron or less in size; it is preferred that heat, in a range from 30° C to 70° C, be applied during the microemulsifying step; in an alternative procedure, the heating step may be deferred until the microemulsion has been formed, as described.

Specific examples of the alcohol and glyceride embodiments of the present invention are provided in the following examples 127-160.

### EXAMPLE 95

To 100 mls of sterile, pyrogen-free water is added 4% w/v of egg lecithin. The mixture is stirred vigorously until the lecithin is thoroughly dissolved. 10 to 12 mls of n-butyl alcohol then are added to the lecithin solution, which is then shaken vigorously for from 1 to 2 hours. At the close of the storage period, it is observed that three layers or phases have formed. The middle layer comprising the colloid-rich phase is separated from the other phases by means of a separatory funnel. 1 to 3 grams of human serum albumin then is dissolved in this colloid-rich phase. The solution then is stored at from 4 to 10° C until visual inspection indicates that this solution has separated into two phases and that no increase in the volume of said phases is occurring. The two phases then are separated by means of a separatory funnel. That quantity of diazepam is added to the colloid-rich phase as will result in a final concentration of 5 mg of diazepam per milliliter of coacervate solution. After this step, the colloid-poor (lower) phase is mixed into the colloid-rich phase. The resulting mixture then is emulsified to produce a microemulsion. The pH of the microemulsion then is adjusted, if necessary, to 7.3-7.4 by the dropwise addition of dilute hydrochloric acid or sodium hydroxide. The preparation then can be stored in suitable containers for use orally or by injection.

### EXAMPLE 96

The procedure of Example 95 is followed except that prior to storage or administration, the composition is heated to produce a sustained (time-release) preparation. The preparation described in Example 1 is heated at 70° C for 60 seconds. The resulting microencapsulated droplets are separated from the emulsion, washed thoroughly with sterile water, then dried thoroughly and dispersed in any appropriate vehicle for parenteral or oral use, for example, to provide a composition containing 5 mg of diazepam per milliliter of solution. On completion of this step, the composition may be administered orally, by injection or stored in suitable containers until needed.

### EXAMPLE 97

The procedure of Example 95 is followed except that 10 mls. of a commercial preparation of diazepam is used in place of diazepam (parent compound).

### EXAMPLE 98

The procedure of Example 96 is followed except that 5 mgs of diazepam is used in place of the commercial parenteral preparation of diazepam.

### EXAMPLE 99

The procedure of Example 95 is followed except that 50 mgs of a commercial preparation of phenytoin is used instead of diazepam.

### EXAMPLE 100

The procedure of Example 96 is used except that 50 mgs of a commercial parenteral preparation of phenytoin is used in place of diazepam.

### EXAMPLE 101

The procedure of Example 95 is followed except that 50 mgs of phenytoin, as the parent compound, is used.

### EXAMPLE 102

The procedure of Example 96 is followed except that 50 mgs of phenytoin, as the parent compound, is used in place of diazepam.

### EXAMPLE 103

The procedure of Example 95 is followed except that 7 mls of diazepam are used.

### EXAMPLE 104

The procedure of Example 95 is followed except that 100 mgs of a commercial intramuscular parenteral preparation of tetracycline HCl is used in place of diazepam.

### EXAMPLE 105

The procedure of Example 96 is followed except that 100 mgs of a commercial intramuscular preparation of tetracycline HCl is used in place of diazepam.

### EXAMPLE 106

The procedure of Example 95 is followed except that 100 mgs of a crystalline tetracycline is used in place of diazepam.

### EXAMPLE 107

The procedure of Example 106 is followed except that 250 mgs of a commercial intravenous preparation of tetracycline HCl (neutralized) reconstituted as per the manufacturer's directions is used instead of diazepam.

### EXAMPLE 108

The procedure of Example 95 is followed except that preparation of the composition is completed when the diazepam is dissolved in the colloid-rich phase of the coacervate system. The preparation is then ready for oral or parenteral administration or storage.

### EXAMPLE 109

The procedure of Example 95 is followed except that preparation of the composition is completed when the phenytoin is dissolved in the colloid-rich phase in place of diazepam. The preparation then may be orally or parenterally administered or stored as required.

### EXAMPLE 110

The procedure of Example 95 is followed except that tetracycline is dissolved in the colloid-rich coacervate phase in place of diazepam. The preparation is then ready for oral or parenteral administration or storage.

### EXAMPLE 111

The procedure of Example 95 is used to the point where the colloid-rich phase is separated from the colloid-poor phase. Instead of using the colloid-rich phase to dissolve the drug component, the colloid-poor phase is used to incorporate the drug component. Thus, 5 mls of commercial trimethoprinsulfamethazole infusion is thoroughly mixed into 125 mls of the colloid-poor phase. The preparation then is ready for infusion.

### EXAMPLE 112

The procedure of Example 111 is followed except that after the drug is mixed into the colloid-poor phase, 125 mls of the colloid-rich phase is added and the preparation emulsified. The composition then may be infused.

### EXAMPLE 113

The procedure of Example 96 is followed except that glucagon, in any quantity ranging from 0.5 mg to 1.0 mg, is substituted for the diazepam. If desired, from 25 mgs to 150 mgs of lactose is added to the preparation following the addition of glucagon.

### EXAMPLE 114

### The procedure of Example 96 is followed except that 0.5 mg of glucagon is substituted for the diazepam.

### EXAMPLE 115

The procedure of Example 95 is followed except that Methyldopate HCl is used instead of diazepam and 1% w/v of urea is added prior to the incorporation of the drug component.

### EXAMPLE 116

Melt the solid form of 15% w/v of long chain triglycerides and 15% w/v medium chain triglycerides. Add 250 mg of erythromycin to the melted product and add 5 ml of an aqueous solution containing 5% albumin and 3% lecithin to this mixture to provide about 250 mg. of erythromycin in the colloid-rich phase. Mix the resulting product and permit the composition to slowly cool to room temperature. If the product is to be used intravenously, adjust the pH and isotonicity as described previously. If the finished product is to be administered orally, adjustment of the pH and isotonicity is optional. Process the product through a colloid mill. This will produce a microsuspended composition having solid microparticles comprised of the pharmaceutic component(s) encapsulated by a triglyceride/coacervate film. While the intravenous product should consist of particles one micron or less in size, oral compositions may have a mixture of particle sizes ranging from a nanometer to 10-15 microns.

### EXAMPLE 117

The procedure of Example 116 is followed except that 1000 units/ml per unit dose of heparin is used in place of erythromycin.

### EXAMPLE 118

Example 95 is followed except 10 ml of Factor VIII is substituted for diazepam.

### EXAMPLE 119

Example 95 is followed except 30 ml of Factor IX complex is substituted for diazepam.

### EXAMPLE 120

### Example 95 is followed except 250 mg of atrial peptide 3 is substituted for diazepam.

### EXAMPLE 121

14% by weight of hemoglobin is encapsulated in a liposome product and then the liposome is placed in the coacervate phase of Example 95. The remaining procedure is the same as Example 95.

### EXAMPLE 122

The procedure of Example 95 is followed except that nutritional quantities of L clonine, L argiuine, L aspartic acid, L glutamine, glysine, L histidine, L proline, L servine, and L tryosine also are added.

### EXAMPLE 123

The procedure of Example 95 is followed except that the diazepam component is first enveloped in a liposome. The resulting product then is mixed into the coacervate phase and processed in the manner described in Example 127.

### EXAMPLE 124

The procedure of Example 123 is followed except that 5 mg of heme is used in place of diazepam.

### EXAMPLE 125

The procedure of Example 123 is followed except that 2,500,000 I.U. of urokinase is used in place of diazepam.

### EXAMPLE 126

The procedure of Example 123 is followed except that, that amountof erythromycin as will give 250 mg of erythromycin in the finished product is used in place of diazepam.

### EXAMPLE 127

The procedure of Example 123 is followed except that, that amountof insulin as will result in a finished product containing 40 units of insulin is used in place of diazepam.

### EXAMPLE 128

The procedure of Example 123 is followed except that, that amount of stroma free hemoglobin as will result in a finished product containing 14 percent by weight of stroma free hemoglobin is used in place of diazepam.

In accordance with another important embodiment of the present invention, compositions capable of oral or intravenous administration, can be prepared containing a physiologically-active component that augments the oxygen-transport capability of the body, to treat various anemias, and act as an oxygen-carrying plasma volume expander. Initially, three aqueous solutions are prepared: an aqueous solution including at least 0.1%, e.g. .1 to 5% w/v of a first non-toxic surfactant, such as albumin; optionally an aqueous solution including at least 0.1%, e.g. .1 to 5% w/v of a second surfactant, such as lecithin; and a aqueous solution containing an oxygen-carrying molecule containing iron, such as heme, a hemoprotein and/or a heme-hemoprotein complex. It is preferred that the hemoprotein of the heme-hemoprotein complex be stroma-free hemoglobin.

In accordance with still another important embodiment of the present invention, it has been found that the instability of the physiologically-active iron-containing molecule capable of carrying and releasing oxygen (heme and/or the hemoproteins and/or the heme-hemoprotein complex) can be minimized or substantially completely eliminated by treating a solution (preferably aqueous) containing the iron-containing molecule, prior to coacervation, with a chemical complexing agent capable of forming a stable iron complex, such as carbon monoxide or an inorganic cyanide, particularly cyanide salts or cyanide complexes, such as sodium cyanide or hydrogen cyanide, and thereafter treating the complexed solution, preferably to saturation, with an interactant, capable of interaction with the iron complex, to release the complexing agent from the iron-containing molecule which escapes from the solution in gaseous form. To achieve the full advantage of the present invention, the chemical complexing agent is carbon monoxide and the interactant is oxygen or purified air bubbled through the solution in excess to release the carbon monoxide from the iron-containing molecule and saturate the solution to remove the carbon monoxide from the solution. This process of iron-complexing and then interaction to release the complexing molecule will stabilized the physiologically-active iron-containing solution, either before or after admixture or emulsification with the other components of the composition so long as the process is carried out prior to coacervation. To achieve full advantage of the present invention, an antioxidant, such as ascorbic acid, is added to the final composition to prevent oxidation of the final composition.

In general, the amount of heme and/or hemoprotein, and/or heme-hemoprotein complex present in the composition of the present invention is determined by the clinically indicated required dose for the treatment of a particular dysfunction. If the composition is to be used to augment the oxygen-carrying capacity of the recipient, the amount of heme, hemoprotein and/or heme-hemoprotein complex in the composition of the present invention is the amount necessary to restore oxygen transport to normal levels within the time frame of the treatment regimen. However, if the intended use of the composition of the present invention is to treat anemic states, such as those due to blood loss, the quantity of heme, hemoprotein and/or heme-hemoprotein complex is the amount necessary to restore normal hemoglobin levels of between approximately 5% and approximately 14% within the time frame of the treatment regimen. Likewise, if the intended use of the composition of the present invention is to treat iron deficiency states, an exemplary amount of heme, hemoprotein and/or heme-hemoprotein complex is that amount that introduces approximately 100-150 mg of elemental iron per dose.

In accordance with the preferred embodiment, the aqueous albumin, lecithin and heme, hemoprotein and/or heme-hemoprotein complex solutions then are thoroughly admixed. Alternatively, the powdered forms of albumin, lecithin, and the heme, hemoprotein and/or heme-hemoprotein complex can be admixed, then converted into a solution by adding sufficient water such that the amount of albumin and lecithin each equals approximately 3% w/v in the finished composition, and the heme, hemoprotein or heme-hemoprotein equals from about 5% to about 30% w/v in the finished composition. This mixture then is subjected to a physical or chemical precipitating agent. For example, heat, ranging from about 20° to about 70° C., is applied until the mixture "precipitates" to produce an aqueous two-phase coacervate system. Temperatures below about 20° C., or above about 70° C., can be utilized according to the method of the present invention so long as the time of heat treatment causes the solution to precipitate into a two-phase coacervate solution while preventing excessive heat treatment to the extent of denaturing the composition causing loss of the coacervate. Alternatively, rather than heating, a non-toxic alcohol, such as ethyl alcohol, or an appropriate electrolyte, such as sodium chloride, can be added to the mixture to produce an aqueous, two-phase coacervate system. The coacervate system then is emulsified using conventional techniques. The isotonicity and the pH of the emulsion are adjusted to approximate the values found in the human body, approximately 0.9% saline. The pH is adjusted using an acid, such as hydrochloric acid, or an alkaline substance, such as sodium hydroxide. Isotonicity is adjusted through addition of an appropriate electrolyte such as sodium chloride, or water as required.

This emulsion can be used as a composition for the oral or intravenous introduction of iron into the body. However, the optional addition of a sterol, such as cholesterol, added in a sufficient amount, for example from 1% to 5% w/v based on the total weight of both phases, provides the composition with a degree of surface hardness thereby improving the composition for use as an orally or intravenously administrable composition.

The emulsion also can be processed further, before isotonicity and pH adjustments are made, by using conventional techniques to produce a microemulsion. The microemulsion includes the coacervate-based microemulsified particles and the heme, and/or hemoprotein and/or the heme-hemoprotein complex, each in a form that is compatible with the recticuloendothelial system and microcirculation systems of the human body and is a size that can be absorbed by the intestines. This microemulsion composition can be administered orally or intravenously.

For oral administration, the microemulsion can be dispensed by any of the conventional oral dosage forms, such as liquid microemulsion or soft capsulation, e.g. a syrup or a capsule containing a liquid. In accordance with another important feature of the present invention, the microemulsion can be processed further using either heat or a cross-linking agent to produce a microencapsulated composition having sustained time-release characteristics. Generally, heat, ranging from 25° to 70° C., is preferred, and is applied over varying time periods, such as 2 sec. to 150 sec., to convert the microemulsion into microencapsulated particles having varying degrees of surface-coacervate film hardnesses. The microemulsified coacervate can also be treated at temperatures 70° C., or below 25° C., for a time sufficient to produce a time-release composition capable of releasing the physiologically-active component, for example, up to about 72 hours, after administration of the composition. It has been found that longer heating time increases the film hardness, thereby affecting the time-release characteristics of microencapsulated particles. Therefore, particles of similar or different release rates can be combined in a single dose and can be administered orally or intravenously after being placed in an appropriate vehicle or dosage form.

In accordance with the present invention, coacervate systems also can be produced by using a single surfactant, such as albumin alone, and a precipitating agent. The precipitating agent can be a non-toxic alcohol, such as isopropyl alcohol or n-butyl alcohol, electrolytes, heat, or a combination thereof. Similarly, an alternative coacervate system that can be used in the compositions of the present invention, can be manufactured by preparing an aqueous solution of a surfactant, such as lecithin, and then adding a precipitating agent, such as an alcohol or an electrolyte in sufficient amount for phase separation to to yield a coacervate system. Subsequent processing steps for these alternative coacervates to produce orally or intravenously administrable microemulsified or microencapsulated compositions are identical to the processing steps described above.

Specific examples directed to the coacervate compositions including an iron-containing molecule capable of carrying and releasing oxygen (heme and/or hemoproteins and/or heme-hemoprotein complexes) are provided in the following examples 129-143.

### EXAMPLE 129

An aqueous 3% w/v solution of albumin, an aqueous 3% w/v solution of lecithin and an aqueous solution of heme, containing the dose of heme desired in the end product, are thoroughly admixed after the heme solution was treated with sufficient carbon monoxide to drive off essentially all the oxygen. The mixture then is heated at 50° C. for a sufficient time to produce a two-phase coacervate system. The two-phase coacervate system then is emulsified in a blender, followed by the addition of an antioxidant, such as ascorbic acid, and by adjustment of (1) isotonicity using sodium chloride and/or water and (2) pH using either an acid or a base, such as hydrochloric acid or sodium hydroxide in an amount sufficient to bring the isotonicity and pH values of the emulsion within normal physiological ranges, e.g., 7.2 to 7.5 pH. The emulsion then is converted, by conventional colloid mill techniques to a microemulsion of coacervate-based microemulsified particles, having a diameter of approximately 1 µm (micron) or less. The composition containing the microemulsified particles, containing encapsulated heme, then is adjusted for isotonicity and pH as above, if necessary. The microemulsified particles can be used for oral or intravenous administration to the body. This microemulsified composition then is heated at 60° C. for about 2 to about 150 seconds to produce hardened, microencapsulated particles having a range of sustained time-release characteristics. The microencapsulated particles then are treated with excess oxygen to remove the carbon monoxide, and, optionally, stored under an inert blanket of nitrogen gas. These hardened, microencapsulated particles, when added to a suitable vehicle, such as syrup, also can be used orally, or in a physiological saline solution intravenously. These compositions are useful to augment the oxygen-transport capacity of the recipient and to increase circulatory volume.

### EXAMPLE 130

The procedure of Example 129, except that a complex of heme and stroma-free hemoglobin replaced the heme.

### EXAMPLE 131

The procedure of Example 129, except that an aqueous solution of 3% w/v of casein replaced the aqueous 3% w/v solution of albumin.

### EXAMPLE 132

The procedure of Example 129, except that an aqueous 3% w/v solution of gelatin replaced the aqueous 3% w/v solution of albumin.

### EXAMPLE 133

The procedure of Example 129, however the manufacturing process was terminated after preparation of the coacervate system microemulsion, thereby producing an orally administrable composition useful in the treatment of iron deficiency anemia, and anemias due to blood loss.

### EXAMPLE 134

The procedure of Example 133, except that a heme and stroma-free hemoglobin complex replaced the heme, thereby producing a composition useful in treating anemias due to iron deficiency or blood loss.

### EXAMPLE 135

The procedure of Example 129, except that the preparation of the microencapsulated sustained time-release formulation is omitted.

### EXAMPLE 136

The procedure of Example 129, except that the complex of heme and stroma-free hemoglobin is used, and the manufacturing process is terminated after preparation of the microemulsion.

### EXAMPLE 137

The procedure of Example 129, except that an aqueous 5% w/v solution of albumin is used instead of the aqueous 3% w/v albumin solution.

### EXAMPLE 138

The procedure of Example 129, except that an aqueous 4% w/v solution of lecithin is used instead of the aqueous 3% w/v lecithin solution.

### EXAMPLE 139

The procedure of Example 129, except that the coacervate system is produced by subjecting an aqueous 3% w/v solution of albumin to temperatures of 30-35° C. until formation of a two-phase coacervate system.

### EXAMPLE 140

The procedure of Example 129, except that the coacervate system is prepared by the dropwise addition of ethyl alcohol to to an aqueous 3% w/v solution of lecithin until a two-phase coacervate system is produced.

### EXAMPLE 141

The procedure of Example 129, except that an aqueous 3% w/v solution of casein is used to replace the aqueous 3% w/v albumin solution, and a complex of heme and stroma-free hemoglobin is used to replace the heme.

### EXAMPLE 142

The procedure of Example 129, except the heme molecule is first encapsulated in a lecithin-cased liposome before proceeding to produce the aqueous two-phase coacervate system.

### EXAMPLE 143

The procedure of Example 129, except the heme-hemoglobin complex is first encapsulated in a lecithin-cased liposome before proceeding to produce the aqueous two-phase coacervate system.

According to the perhalogenated embodiment of the method and composition of the present invention, a non-toxic perhalogenated compound, such as a perfluorinated hydrocarbon and/or a perfluorinated amine, can be used with the aqueous coacervate system to provide the compositions of the present invention. The perhalogenated compounds useful in the present invention include perfluorinated and perfluoriodinated aliphatic and alicyclic hydrocarbons, perfluorinated and perfluoriodinated aliphatic and alicyclic ethers, and/or perfluorinated and perfluoriodinated aliphatic and alicyclic amines. It has been found that perfluorodecalin and perfluoroiododecalin are especially useful in the compositions of the present invention, either used alone or in combination with other perfluorinated compounds, such as perfluorotributylamine, perfluorotripropylamine, perfluorotrimethylbicyclo[3.3.1]nonane and/or perfluoro-N,N-dimethylcyclohexylmethylamine.

In accordance with an important feature of the present invention, a method of producing a composition, including a perhalogenated, and preferably a perfluorinated, compound; a two-phase aqueous coacervate system; and a physiologically-active compound is disclosed. According to the method of the present invention, the physiologically-active compound is admixed with the perhalogenated compound, and the resulting mixture then is admixed with an aqueous surfactant solution, such as an aqueous albumin solution and/or an aqueous lecithin solution. Subsequent processing of the complete mixture produces a coacervate system. The resulting coacervate-based composition then is processed further to produce a microemulsion, having particles of 1 µm (micron) or less in diameter. Additionally, the microemulsion can be processed further using heat, or other appropriate precipitating agents, to produce sustained time-release compositions, having a single rate of release or a range of release rates.

The composition of the present invention can be administered orally or parenterally, preferably intravenously. In addition, the composition of the present invention can be lyophilized using conventional techniques to convert the liquid to a powder. The powdered product then can be reconstituted using appropriate physiological fluids. For instance, when the reconstituted composition is intended for intravenous use, isotonicity and pH should be adjusted to normal body values before administration.

In preparing the compositions of the present invention, it is preferred that both phases of the two-phase, aqueous coacervate system be used. However, the non-polar, colloid-rich phase can be separated from the colloid-poor phase, then used to prepare the compositions of the present invention. The compositions of the present invention comprise microemulsified particles containing the aqueous coacervate and the perhalogenated compound. A physiologically-active component can be incorporated into the perhalogenated compound-coacervate system compositions of the present invention to yield a variety of end products that are useful in the medical field. The microemulsified particles are manufactured to be of appropriate size, such that the particles are compatible with the microcirculation system and the reticuloendothelial system of the human body and other mammals. The compositions of the present invention may, if desired, be processed further to produce compositions having time-release characteristics, such as by microencapsulating the particles to provide a time-release hardened coating on the composition.

To prepare the perhalogenated compositions embodiment of the present invention, two solutions are prepared: an aqueous solution, including from 0.01% w/v to a quantity up to 100% w/v dispersion, of albumin; and an aqueous solution including from 0.01% w/v to a quantity up to 100% w/v dispersion of lecithin. In addition, a sufficient amount of a perhalogenated compound, such as perfluorodecalin, is required to produce a composition including from 0.1% w/v to 99% w/v of the perhalogenated compound. To achieve the full advantage of the present invention, the aqueous solutions include from 1% to 5%, and preferably 3%, w/v of albumin and 1% to 5%, and preferably 3% w/v of lecithin. Optionally, powdered albumin and/or powdered lecithin can be mixed with the perhalogenated compound, and this mixture then converted to an aqueous solution containing the required amount of albumin, lecithin and perhalongenated compound. According to the method of the present invention, the compositions can include equal or unequal proportions of albumin solution to lecithin solution, however, equal proportions of the albumin and lecithin solutions are preferred. To achieve the full advantage of the present invention, equal weight amounts of albumin and lecithin are included in the compositions of the present invention.

For example, if the finished product is intended to be used as a barrier to food absorption, approximately equal amounts of an approximately 3% w/v aqueous solution of albumin and an approximately 3% w/v aqueous solution of lecithin, and a quantity of a perhalogenated compound, such as perfluorodecalin, are thoroughly admixed and processed in a colloid mill to yield a mixture including from 30% w/v to 50% w/v, and preferably 40% w/v, of perfluorodecalin. The mixture then is subjected to a physical or chemical precipitating agent. For example, heat, ranging from 20° to 70° C., and preferably ranging from 30° to 50° C, is applied until the mixture "precipitates" to produce a composition comprising the aqueous two-phase coacervate system and the perhalogenated compound. Temperatures below 20° C., or above 70° C., can be utilized according to the method of the present invention so long as the time of heat treatment causes the solution to precipitate into a two-phase coacervate solution while preventing excessive heat treatment to the extent of denaturing the composition causing loss of the coacervate. Alternatively, rather than heating, a non-toxic alcohol, such as ethyl alcohol, or an appropriate electrolyte, such as sodium chloride, can be added to the mixture to produce an aqueous, two-phase coacervate system. The mixture is subjected to the physical or chemical precipitating agent until visual inspection, or inspection by microscopic techniques known to those skilled in the art, shows that the mixture has separated into the two-phase aqueous coacervate system.

The coacervate system-perhalogenated compound composition then is re-emulsified using conventional techniques. The resulting emulsion also can be processed further, by using conventional techniques, such as a colloid mill to produce a microemulsion. The microemulsion includes the coacervate-based microemulsified particles and the perfluorinated compound, each in a form that is compatible with the recticuloendothelial system and microcirculation systems of the human body. This microemulsion composition can be administered orally or intravenously.

For oral administration, the microemulsion can be dispensed by any of the conventional oral dosage forms, such as liquid microemulsion or soft capsulation, e.g. a syrup or a capsule containing a liquid. In accordance with another important feature of the present invention, the microemulsion can be processed further using either heat or a cross-linking agent to produce a microencapsulated composition having sustained time-release characteristics. Generally, heat, ranging from 25° to 70° C., is preferred, and is applied over varying time periods, such as about 2 sec. to about 150 sec., to convert the microemulsion into microencapsulated particles having varying degrees of surface-coacervate film hardnesses. The microemulsified coacervate can also be treated at temperatures above 70° C., or below 25° C., for a time sufficient to produce a time-release composition capable of releasing the physiologically-active component, for example, up to about 72 hours, after administration of the composition. It has been found that longer heating time increases the film hardness, thereby affecting the time-release characteristics of microencapsulated particles. Therefore, particles of similar or different release rates can be combined in a single dose and can be administered orally or intravenously after being placed in an appropriate vehicle or dosage form.

The composition made in accordance with the above method can be utilized in weight reduction programs, whereby after orally administering the food-barrier composition, the perhalogenated compound included in the composition coats the intestinal walls to preclude the absorption of food. Therefore, the patient is not limited in the amount of food consumed because no food is absorbed through the intestinal walls until the perhalogenated compound is expelled from the body. The dosages and frequency of treatment are clinical determinations that are made in regard to each individual patient such that weight loss objectives and nutritional needs are balanced.

It should be noted that other additives, such as flavorants, can be added to the perhalogenated compound-aqueous coacervate composition to enhance patient appeal, without detracting from the efficacy of the composition. According to the method of the present invention, the aqueous coacervate system allows the oral introduction of the non-polar, non-aqueous perhalogenated compound into the patient without the need of additional emulsifying and suspending agents that are normally required to solubilize the perfluorinated compound, but generally detract from product aesthetics and patient appeal.

The aqueous coacervate system, including the perfluorinated compound, can also be used as a resuscitation fluid to quickly introduce an oxygen-carrying molecule into a patient. In the manufacture of a resuscitation fluid, the process of admixing the aqueous albumin and lecithin solutions and the perfluorodecalin is performed as above except that prior to admixing the three liquids heme and/or stroma-free hemoglobin, in an amount ranging from 0.1% to 30% w/v is added to the perfluorodecalin. The amount of perhalogenated compound used in the resuscitation fluid is limited to from 10% w/v to 30% w/v, and preferably to 20% w/v, of the entire composition. At these levels, the amount of perhalogenated compound is too low to act as a food barrier, but is sufficiently large to coat the intestinal wall and bring the heme and/or stroma-free hemoglobin into intimate contact with the intestinal wall for rapid absorption. The perhalogenated compound is not absorbed by the intestinal walls but serves to facilitate transport of the oxygen-carrying heme or stroma-free hemoglobin into the circulatory system.

It is preferred that the amount of the oxygen-carrying molecule included in the resuscitation fluid approximate the amount of hemoglobin percent in whole blood, such as from 5% to 14%. It should be noted, however, that because of the inherent oxygen-carrying capability of the perfluorinated compound, the amount of heme or stroma-free hemoglobin can be below the 14% wt% hemoglobin normally found in whole blood. The heme or hemoglobin used in the present invention may be of endogenous, exogenous, or synthetic sources.

After admixing the albumin and lecithin solutions, the perhalogenated compound, and the heme and/or stroma-free hemoglobin, the osmotic pressure of the resulting mixture is adjusted to the isotonicity of whole blood by the addition of sodium chloride and/or any other suitable electrolyte or water, as required. The pH is adjusted using an acid, such as hydrochloric acid, or an alkaline substance, such as sodium hydroxide. The isotonicity and the pH of the emulsion are adjusted to approximate the values found in the human body, approximately 0.9% saline, and a pH of approximately 7.3 to 7.45.

After the pH and isotonicity adjustments, the mixture is processed as previously described for the food-barrier composition to produce a microemulsion having a particle size that is compatible with the microcirculation and the reticuloendothelial system, such as 1 µm (micron) or less in diameter. The product then can be administered intravenously as a resuscitative fluid.

Similarly, a product useful as an oxygencarrying contrast medium, such as those used in angioscopy, can be manufactured according to the method of the present invention. The oxygen-carrying contrast medium can be prepared by the method described above for the manufacture of a resuscitation fluid, however, a perfluoroiodinated compound, such as perfluoroiododecalin or iodinated perfluorodecalin, is used in place of perfluorodecalin.

If the perfluorinated compound-coacervate system composition is to be used as a drug delivery system, perfluorodecalin, the albumin solution and the lecithin solution are prepared as described above. Before the solutions are admixed, however, the non-polar to semi-polar physiologically-active and/or pharmacological compound or compounds are admixed into the perfluorodecalin in a sufficient amount to yield the desired, or required, dose in the finished product. If the solubility of the physiologically-active and/or pharmacological compound or compounds in the perfluorinated compound is exceeded, the physiologically-active and/or the pharmacological compound or compounds can be dispersed and suspended in the perfluorinated compound to achieve the required, or desired, clinical dosage.

The physiologically-active and/or pharmacological components can include any drug, hormone, biological, enzyme, antibiotic, peptide or other useful medical composition that is soluble in the perfluorinated compound to a sufficient degree to obtain a clinical dose. If salts of the parent drug compounds or other water-soluble pharmaceuticals also are included in the product, these compounds are dissolved in the aqueous albumin or the aqueous lecithin solutions before admixing and processing to produce the coacervate system. After admixing, the mixture is processed in the same manner as described previously to prepare the food barrier composition.

In those instances where the product is to be administered intravenously, the pH and isotonicity of the composition are adjusted, if necessary, to approximate that of whole blood. The product then is suitable for either oral or intravenous administration, as the particle size of the microemulsion, 1 micron or less in diameter, allows absorption through the intestinal wall and is compatible with the microcirculation system and the reticuloendothelial system. For oral compositions, the amount of the perhalogenated compound is maintained at between 10% w/v to 30% w/v, and preferably 20% w/v of the finished drug composition to facilitate absorption of the drug into the circulatory system. For parenterally administered compositions, the amount of perhalogenated compound can be incorporated in amounts varying from 0.1% w/v to 99% w/v of the finished drug composition, in order to maximize the solubility and stability of the physiologically-active and/or pharmacological component.

The method of the present invention also can be utilized to incorporate a local or general anesthetic into the coacervate system-perhalogenated compound composition. The anesthetic agent, either a gas, liquid, liquified gas, or mixture thereof, first is dissolved in the perfluorinated compound in the amount necessary to produce the desired anesthetic dose in the final product. As described previously, the albumin solution, lecithin solution and perfluorinated compound are admixed, followed by heating the mixture at temperatures ranging from approximately 20° to approximately 70° C for a sufficient time to produce a two-phase coacervate system, as shown by visual or microscopic techniques.

The two-phase coacervate system then is processed to yield a microemulsion, having a particle size that is compatible with the microcirculation and the reticuloendothelial system. The pH of the microemulsion is adjusted to that of whole blood using an acid, such as hydrochloric acid or a base such as sodium hydroxide. The resulting product is suitable for intravenous and/or oral administration, allowing for more control in the amount of anesthetic delivered and in the depth of the anesthesia.

Any of the oral or intravenously administered products can be prepared in a sustained time-release form by treating the composition with an organic aldehyde, such as glutaraldehyde, or heat. The method provides a composition having a single or multiple rate of release. The process yields microemulsified particles encapsulated by a coacervate-based film that can be of any desired degree of particle surface film hardness, ranging from liquid to gel-like to rigid.

According to this method, a portion, or all, of the microemulsified composition is heated, generally in a range of from about 25° to about 70° C, and preferably in the range of 30° to about 40° C over a varying time period, such as 2 sec. to about 150 sec., to convert the microemulsion into microencapsulated particles having varying degrees of coacervate film hardness. The microemulsified coacervate can also be treated at temperatures above 70° C., or below 25° C., for a time sufficient to produce a time-release composition capable of releasing the physiologically-active component, for example, up to about 72 hours, after administration of the composition. It has been found that longer heating time increases the film hardness, thereby affecting the time-release characteristics of microencapsulated particles. Therefore, particles of similar or different release rates can be combined in a single dose and can be administered orally or intravenously after being placed in an appropriate vehicle or dosage form.

In accordance with the present invention, coacervate systems also can be produced by using a single surfactant, such as albumin alone, and a precipitating agent. The precipitating agent can be a non-toxic alcohol, such as isopropyl alcohol or n-butyl alcohol; electrolytes; heat; or a combination thereof. Similarly, an alternative coacervate system that is useful in the compositions of the present invention, can be manufactured by preparing an aqueous solution of a surfactant, such as lecithin, and then adding a precipitating agent, such as an alcohol or an electrolyte, in sufficient amount for phase separation to yield a coacervate system. Subsequent processing steps for these alternative coacervates to produce orally or intravenously administrable microemulsified or microencapsulated compositions are identical to the processing steps described above.

Specific examples of the perhalogenated composition embodiment of the present invention are provided in the following examples 144-180.

### EXAMPLE 144

Approximately equal volumes of an aqueous 3% w/v solution of albumin and an aqueous 3% w/v solution of lecithin, and a sufficient amount of perfluorodecalin to provide 40% w/v of perfluorodecalin in the final solution are thoroughly admixed. The mixture then is heated at 70° C. for a sufficient time, such as from about 3 seconds to about 2 minutes, to produce a two-phase coacervate system. The mixture is examined continuously by visual or microscopic techniques throughout the heating period in order to terminate the application of heat as soon as the mixture separates into a two-phase aqueous coacervate system. The coacervate system, including the perfluorodecalin, then is processed in a colloid mill to produce a microemulsion. The microemulsion then can be used as an orally-administrable food absorption barrier composition.

### EXAMPLE 145

The procedure of Example 144, except that the application of heat is replaced by the dropwise addition of ethyl alcohol until a visual inspection shows that the mixture has separated into the two-phase aqueous coacervate system.

### EXAMPLE 146

The procedure of Example 144, except that the application of heat is replaced by the addition of sodium chloride until a visual inspection shows that the mixture has separated into the two-phase aqueous coacervate system.

### EXAMPLE 147

The procedure of Example 144, except that perfluorotributylamine is used in place of perfluorodecalin to yield a food absorption barrier composition including 30% w/v or perfluorotributylamine.

### EXAMPLE 148

The procedure of Example 144, except that the same volume of an aqueous 5% w/v albumin solution is used.

### EXAMPLE 149

The procedure of Example 144, except that the same volume of an aqueous 5% w/v lecithin solution is used.

### EXAMPLE 150

The procedure of Example 144, except that the mixture is heated at 40° C.

### EXAMPLE 151

The procedure of Example 144, except that an aqueous solution of heme is admixed into the perfluorodecalin before the albumin solution, lecithin solution and perfluorodecalin are thoroughly admixed.

The amount of 20% w/v heme solution added to the mixture is sufficient to provide a final composition including approximately 7% w/v of heme. The perfluorodecalin is present at approximately 20% w/v of the final composition. The isotonicity and pH of the mixture then are adjusted using sodium chloride and/or water, and either an acid or a base, such as hydrochloric acid or sodium hydroxide, in an amount sufficient to bring the isotonicity and pH values of the emulsion within normal physiological ranges, e.g., 7.2 to 7.5 pH and 0.9% saline. The mixture then is heated at approximately 70° C until a visual inspection indicates that the mixture has separated into the two-phase aqueous coacervate system. The coacervate system, including the heme, then is emulisifed to produce a microemulsion having a particle size of 1 micron or less in diameter. The composition then can be used as an intravenously administrable resuscitation fluid.

### EXAMPLE 152

The procedure of Example 151, except that an aqueous solution of 20% w/v stroma-free hemoglobin instead of heme is admixed into the aqueous 3% w/v albumin solution to provide a mixture including 7% stroma-free hemoglobin.

### EXAMPLE 153

The procedure of Example 151, except that an aqueous solution of a heme and stroma-free hemoglobin complex instead of heme is admixed into the aqueous 3% w/v lecithin solution to provide a mixture including 7% w/v heme and stroma-free hemoglobin complex.

### EXAMPLE 154

The procedure of Example 152, except that polymerized stroma-free hemoglobin is used in place of heme.

### EXAMPLE 155

The procedure of Example 152, except that pyridoxilated polymerized, stroma-free hemoglobin is used in place of heme.

### EXAMPLE 156

The procedure of Example 151, except that the composition includes 30% w/v of perfluorinated trimethylbicyclo[3.3.1]nonane in place of the 20% perfluorodecalin.

### EXAMPLE 157

The procedure of Example 151, except that the composition includes 20% w/v of perfluorotributylamine in place of the 20% perfluorodecalin.

### EXAMPLE 158

The procedure of Example 151, except that the composition includes 12% w/v of perfluorodecalin and 8% w/v of perfluorotributylamine in place of the 20% perfluorodecalin.

### EXAMPLE 159

The procedure of Example 151, except that insulin is used in place of heme to provide a composition including 40 units/cc of insulin.

### EXAMPLE 160

The procedure of Example 151, except that the anti-coagulant, sodium heparin, is used in place of heme to provide a composition including 1000 USP units/ml of sodium heparin.

### EXAMPLE 161

The procedure of Example 144, except (1) methotrexate, a cancer treatment drug, is added to the perfluorodecalin before the perfluorodecalin is admixed with the aqueous lecithin and aqueous albumin solutions, and (2) the pH of the albumin-lecithin-perfluorodecalin-methotrexate mixture is adjusted to approximately 7.4, by adding either hydrochloric acid or sodium hydroxide, before the mixture is processed to produce a microemulsion to provide a composition including 25 mg/ml of methotrexate.

### EXAMPLE 162

The procedure of Example 161, except (1) the antibiotic, gentamycin, is used in place of methotrexate, and (2) the pH adjustment is made after the mixture is processed to produce a microemulsion to provide a composition including one mg/ml of gentamycin.

### EXAMPLE 163

The procedure of Example 161, except (1) the antibiotic, cefazolin, is used in place of methotrexate, and (2) the pH adjustment is made before and after the mixture is processed to produce a microemulsion to provide a composition including 125 mg/ml of cefazolin.

### EXAMPLE 164

The procedure of Example 161, except that the antibiotic cephapirin is used in place of methotrexate to provide a composition including 250 mg/ml of cephapirin.

### EXAMPLE 165

The procedure of Example 161, except that the antibiotic thiopental is used in place of methotrexate.

### EXAMPLE 166

The procedure of Example 161, except (1) the anesthetic halothane is used in place of methotrexane, and (2) the halothane is bubbled into the perfluorodecalin to provide a composition including 20% w/v of halothane.

### EXAMPLE 167

The procedure of Example 161, except that the anesthetic fentanyl is used in place of methotrexate to provide a composition including 0.05 mg/ml of fentanyl.

### EXAMPLE 168

The procedure of Example 161, except that the anesthetic sufentanil is used in place of methotrexate to provide a composition including 50 ug/ml of sufentanil.

### EXAMPLE 169

The procedure of Example 161, except that the muscle relaxant succinylcholine is used in place of methotrexate to provide a composition including 20 mg/ml of succinylcholine.

### EXAMPLE 170

The procedure of Example 161, except that calcitonin, used in the treatment of osteoporosis, is used in place of methotrexate to provide a composition including 20 I.U./ml of calcitonin.

### EXAMPLE 171

The procedure of Example 161, except that the anesthetic ketamine is used in place of methotrexate to provide a composition including 10 mg/ml of ketamine.

### EXAMPLE 172

The procedure of Example 161, except that the anesthetic fluorocil is used in place of methotrexate to provide a composition including 50 mg/ml of fluorocil.

### EXAMPLE 173

The procedure of Example 161, except that the labor inducer oxytocin is used in place of methotrexate to provide a composition including 10 USP units/ml of oxytocin.

### EXAMPLE 174

The procedure of Example 151, except that monensin is used in place of heme.

### EXAMPLE 175

The procedure of Example 161, except that monensin is used in addition to methotrexate to provide a composition including 25 mg/ml of methotrexate.

### EXAMPLE 176

The procedure of Example 144, except that iodine is added to the perfluorodecalin prior to the admixing the albumin solution, lecithin solution and perfluorodecalin solution to provide a contrast medium containing 1% w/v of iodine.

### EXAMPLE 177

The procedure of Example 144, except that vitamin A, vitamin D, and vitamin E are added to the perfluorodecalin prior to admixing the perfluorodecalin, albumin solution and lecithin solution. After admixing, the mixture is heated at 70° C until the mixture separates into a two-phase coacervate system. The two phases are separated, and thiamine hydrochloride, d-biotin, riboflavin, niacinamide, pyridoxine hydrochloride, dexpanthenol, ascorbic acid, folic acid, and cyanocobalamin is added to the polar phase. The phases are recombined and processed to produce a microemulsion having a particle size of 1 micron or less in diameter to provide a composition including 3300 USP units/ml of vitamin A, 200 USP units/ml vitamin D, 10 USP units/ml vitamin E, 3 mg/ml thiamine hydrochloride, 60 mg/ml d-biotin, 3.5 mg/ml riboflavin, 40 mg/ml niacinamide, 4 mg/ml pyridoxine hydrochloride, 15 mg/ml dexpanthenol, 100 mg/ml ascorbic acid, 400 ug/ml folic acid, and 5 ug/ml cyanocobalamin.

### EXAMPLE 178

The procedure of Example 161, except that the anti-asthmatic aminophylin is used in place of methotrexate to provide a composition including 25 mg/ml of aminophylin.

### EXAMPLE 179

The procedure of Example 153, except that the microemulsion is divided into four equal portions. The first portion is left untreated, the second portion is heated at 70° C for 10 seconds, the third portion is heated at 70° C for 40 seconds and the fourth portion is heated at 70°C for 70 seconds to produce a microencapsulated composition having sustained time release capabilities.

### EXAMPLE 180

The procedure of Example 144, except that the commercial product, perfluoroiodide, is used in place of perfluorodecalin to provide a contrast medium including 40% w/v of perfluoroiodide.

In one embodiment a method of maintaining tissue of an organ of a mammal viable, outside of the mammal's body comprising immersing the tissue or organ in the composition as claimed herein to maintain the viability of the tissue or organ until said tissue or organ is attached to a mammal.

In another embodiment a method of preparing a perhalogenated composition includes mixing an aqueous solution of water, a surface active agent, and a perhalogenated compound under conditions to form a two-phase aqueous coacervate system and contacting the perhalogenated physiologically-active compound with one or both of the two-phases to envelope the physiologically-active compound, bound in particles, within a coacervate-based aqueous film; said particles having a particle size of less than 1000 µm (microns).

## Claims

1. A method of preparing a composition useful as a system to introduce and transport medically useful compositions in the body of mammals by oral, tissue absorptive, inhalation, or parenteral administration; the method being characterised in that it comprises forming a core material comprising core particles of a material having a desired particle size; forming a mixture of one or more surface active agents, water and one or more physiologically-active compounds and emulsifying the mixture to produce a two phase coacervate composition comprising a coacervate phase and an equilibrium water phase, the physiologically-active compound being contained in one or both of said phases; forming the core material particles with one or both of the coacervate phases or contacting the core material with one or both of the coacervate phases to embed the physiologically-active compound within a coacervate-based core material matrix or to encapsulate the core material particles within an encapsulating coacervate-based film containing the physiologically-active compound.

2. A method as claimed in claim 1 further characterised by emulsifying and microemulsifying the core material with an emulsifying agent and water under conditions to form coacervate particles in the matrix useful for oral administration having a particle size less than 10 µm (microns), preferably less than 1 µm (micron).

3. A method as claimed in either claim 1 or claim 2, characterised in that the surface active agent is a polymerized phospholipid, preferably polymerized lecithin, cephalin, isolecithin, sphingomyelin, phosphalidyl serine, phosphatidic acid, phosphatidyl inositol, phosphatidyl choline, or mixtures thereof.

4. A method as claimed in any one of the preceding claims, characterised in that the coacervate mixture includes two surface active agents, at least one of which is a polymer having a weight average molecular weight of 300,000 or less, the two surface active agents being preferably a phospholid and a surface active protein, for example lecithin and polymerized albumin.

5. A method as claimed in any one of the preceding claims, further characterised by drying the composition to form a powder capable of being reconstituted by the addition of a physiologically acceptable liquid.

6. A method as claimed in any one of the preceding claims, characterised in that the or each surface active agent is added in monomeric form and a polymerization initiator is added to said composition, said polymerization initiator being capable of and present in an amount sufficient to polymerize the surface active agent during processing of said coacervate mixture.

7. A method as claimed in any one of the preceding claims, characterised in that the core material is formed from a coacervate-based matrix and the matrix is encapsulated with a coacervate-based film.

8. A method as claimed in any one of the preceding claims, characterised in that the physiologically-active compound comprises insulin, an atrial peptide, or a perhalogenated compound, for example a perfluorinated hydrocarbon, perfluoroiodinated hydrocarbon, perfluorinated amine, perfluoroiodinated amine, perfluorinated ether, perfluoroiodinated ether, perfluorinated hydrocarbon-iodine complex, or mixtures thereof, preferably perfluorodecalin, perfluoroiododecalin, perfluorotributylamine, perfluorotripropylamine, perfluoro-N,N-dimethylcyclohexylmethylamine, perfluorotrimethyl-bicyclo[3.3.1]nonane, iodinated perfluorodecalin or perfluoroiodide.

9. A method as claimed in any one of the preceding claims, characterised in that the physiologically active compound in one or both of the two coacervate phases is bound as particles in a coacervate matrix and within a coacervate-based aqueous film; and further including the steps of separating the particles; and adding a hydrocolloid to the particles in an amount of 2-20% w/v based on the weight of the coacervate mixture to form a gel-like surface film surrounding the physiologically active compound.

10. A method as claimed in any one of the preceding claims, characterised by adding an anti-oxidant to the coacervate mixture during preparation.

11. A method as claimed in any one of the preceding claims, characterised in that it includes the step of adding a physiologically acceptable alcohol to the coacervate mixture to form a three layer liquid comprising a lower polar layer, a middle layer, and an upper non-polar layer; separating the middle layer from the other two layers; adding a second surface active agent to the separated middle layer to form a second two-phase liquid comprising a lower polar layer and an upper non-polar layer; and adding a physiologically-active compound to one or both of said layers formed from said second two-phase liquid.

12. A method as claimed in claim 11, characterised in that the second two-phase liquid is separated into a lower polar layer and an upper non-polar layer and a water-insoluble compound is added to the upper non-polar layer.

13. A method as claimed in claim 11, characterised in that the physiologically-active compound is water-soluble, and is contained within the lower polar layer of said second two-phase liquid.

14. A method as claimed in any one of the preceding claims, characterised in that the core particles are encapsulated by a coacervate-based film; and in that the encapsulated particles are contacted with a second coacervate composition to form an additional coacervate-derived film on the particles.

15. A method as claimed in claim 14, characterised in that the second coacervate composition contains 0.1 to 50% by weight of an additional physiologically-active compound, based on the total weight of the second coacervate composition.

16. A composition prepared by a method as claimed in any one of the preceding claims.

17. A composition as claimed in claim 16, characterised in that the physiologically-active compound is contained within a liposome core material enveloped within an aqueous coacervate-based film to prevent the liposome from unravelling prematurely.

18. A composition as claimed in claim 17, characterised in that the liposome containing the physiologically-active compound is formed prior to encasing the liposome within the coacervate-based film, and wherein the coacervate-based film comprises an aqueous colloid-rich phase, or an aqueous colloid-poor phase, or a combination of both phases.

19. A composition as claimed in any one of claims 16 to 18, characterised in that the coacervate-based film comprises an aqueous colloid-rich phase, or an aqueous colloid-poor phase, or a combination thereof from the coacervate mixture.

20. A composition as claimed in any one of claims 16 to 19, for introduction into the bloodstream of a mammal, characterised in that it further comprises glycerol esters selected from the group consisting of a monoglyceride, a diglyceride, a triglyceride, and mixtures thereof; said physiologically-active compound being dissolved or dispersed in the glyceride.

21. A composition as claimed in claim 20, characterised in that the glyceride is a mono or diglyceride having 4 to 24 carbon atoms, preferably 8 to 18 carbon atoms.

22. A composition as claimed in either claims 20 or 21, characterised in that it includes an effective amount of solubilizing agent capable of increasing the solubility of the physiologically-active compound in the glyceride, said agent being preferably a hydrogenated oil or an unsaturated oil.

23. A composition as claimed in any one of claims 20 to 22, characterised in that the glyceride contains 1% by weight to 50% by weight of a hydrogenated oil or an unsaturated oil, preferably in an amount of 5% by weight to 15% by weight of the glyceride.

24. An orally ingestible composition as claimed in any one of claims 16 to 23.

25. A composition as claimed in claim 24 for increasing the circulatory volume or introducing iron and/or oxygen into the circulatory system of a mammal characterised in that the physiologically-active compound comprises an iron containing molecule capable of carrying and releasing oxygen or an oxygen solvent.

26. A composition as claimed in claim 25, characterised in that the iron-containing molecule capable of carrying and releasing oxygen is a heme, a hemoprotein, a heme-hemoprotein complex, pyridoxilated polymerized hemoglobin, polymerized hemoglobin; or a mixture thereof.

27. A composition as claimed in claim 24, for controlling the caloric intake of a mammal characterised in that the composition includes a perhalogenated compound in an amount of from 30% w/v to 50% w/v based on the total weight of the composition.

28. A composition as claimed in claim 24 for increasing the oxygen content in the circulatory system of a mammal characterised in that the physiologically-active compound is a combination of a perhalogenated compound and a compound selected from the group consisting of an iron-containing molecule capable of carrying and releasing oxygen and an oxygen solvent; said aqueous coacervate system comprising an aqueous colloid-rich phase, an aqueous colloid-poor phase, or a combination thereof; wherein the coacervate phase includes the perhalogenated compound, and either an iron-containing molecule capable of carrying and releasing oxygen or an oxygen solvent, or a combination thereof.

29. A composition as claimed in claim 28, characterised in that the iron-containing molecule capable of carrying and releasing oxygen is selected from the group consisting of heme; a hemoprotein, a heme-hemoprotein complex; pyridoxylated hemoglobin; pyridoxylated polymerized hemoglobin and mixtures thereof.

30. A contrast medium for introduction into the circulatory system of a mammal comprising an orally ingestiable or injectible composition as claimed in any one of claims 16 to 23.

31. An anaesthetic composition as claimed in any one of claims 16 to 23, characterised in that the composition includes an anaesthetic added to the composition during preparation.

32. An injectible composition comprising a composition as claimed in any one of claims 16 to 23.

33. A composition as claimed in any one of claim 16 to 23 for increasing the circulatory volume or introducing iron and/or oxygen into the circulatory system of a mammal by establishing fluid communication between the mammal's circulatory system and the composition.

## Patentansprüche

1. Verfahren zum Herstellen einer Zusammensetzung, die als System zum Einbringen und Transportieren von medizinisch brauchbaren Zusammensetzungen in den Körper von Säugetieren durch orale, Gewebeabsorptions-, Inhalations- oder parenterale Verabreichung brauchbar ist; wobei das Verfahren **dadurch gekennzeichnet** ist, daß es das Bilden eines Kernmaterials, welches Kernpartikel aus einem Material mit einer gewünschten Partikelgröße umfaßt; das Bilden eines Gemisches aus einem oder mehreren oberflächenaktiven Mitteln, Wasser und einer oder mehreren physiologisch wirksamen Verbindungen und das Emulgieren des Gemisches zum Herstellen einer Zweiphasenkoazervatzusammensetzung, die eine Koazervatphase und eine Gleichgewichtswasserphase umfaßt, wobei die physiologisch wirksame Verbindung in einer oder beiden Phasen enthalten ist; das Bilden der Kernmaterialpartikel mit einer oder beiden Koazervatphasen oder das Inkontaktbringen des Kernmaterials mit einer oder beiden Koazervatphasen, um die physiologisch wirksame Verbindung in einer Kernmaterialmatrix auf Koazervatbasis einzubetten oder um die Kernmaterialpartikel in einem einkapselnden Film auf Koazervatbasis einzukapseln, welcher die physiologisch wirksame Verbindung enthält, umfaßt.

2. Verfahren nach Anspruch 1, welches ferner **gekennzeichnet** ist durch Emulgieren und Mikroemulgieren des Kernmaterials mit einem Emulgiermittel und Wasser unter Bedingungen zum Bilden von Koazervatpartikeln in der Matrix, welche zur oralen Verabreichung brauchbar sind und eine Partikelgröße von weniger als 10 µm, vorzugsweise weniger als 1µm haben.

3. Verfahren nach Anspruch 1 oder Anspruch 2, **dadurch gekennzeichnet**, daß das oberflächenaktive Mittel ein polymerisiertes Phospholipid, vorzugsweise polymerisiertes Lecithin, Cephalin, Isolecithin, Sphingomyelin, Phosphatidylserin, Phosphatidsäure, Phosphatidylinositol, Phosphatidylcholin oder Gemische davon ist.

4. Verfahren nach einem der vorhergehenden Ansprüche, **dadurch gekennzeichnet**, daß das Koazervatgemisch zwei oberflächenaktive Mittel einschließt, von denen mindestens eines ein Polymer mit einem gewichtsdurchschnittlichen Molekulargewicht von 300 000 oder weniger ist, wobei die zwei oberflächenaktiven Mittel vorzugsweise ein Phospholipid und ein oberflächenaktives Protein, z.B. Lecithin und polymerisiertes Albumin sind.

5. Verfahren nach einem der vorhergehenden Ansprüche, ferner **gekennzeichnet durch** Trocknen der Zusammensetzung, um ein Pulver zu bilden, welches durch die Zugabe einer physiologisch annehmbaren Flüssigkeit zur ursprünglichen Konzentration gelöst (rekonstituiert) werden kann.

6. Verfahren nach einem der vorhergehenden Ansprüche, **dadurch gekennzeichnet**, daß das oder jedes oberflächenaktive Mittel in monomerer Form zugegeben wird und ein Polymerisationsinitiator der Zusammensetzung zugegeben wird, wobei der Polymerisationsinitiator die Fähigkeit hat und in einer ausreichenden Menge vorhanden ist, um das oberflächenaktive Mittel während der Verarbeitung des Koazervatgemisches zu polymerisieren.

7. Verfahren nach einem der vorhergehenden Ansprüche, **dadurch gekennzeichnet**, daß das Kernmaterial aus einer Matrix auf Koazervatbasis gebildet wird und die Matrix mit einem Film auf Koazervatbasis eingekapselt wird.

8. Verfahren nach einem der vorhergehenden Ansprüche, **dadurch gekennzeichnet**, daß die physiologisch wirksame Verbindung Insulin, ein Herzvorhofpeptid oder eine perhalogenierte Verbindung, z.B. einen perfluorierten Kohlenwasserstoff, perfluoriodierten Kohlenwasserstoff, perfluoriertes Amin, perfluoriodiertes Amin, perfluorierten Ether, perfluoriodierten Ether, perfluorierten Kohlenwasserstoff-Iod-Komplex oder Gemische davon, vorzugsweise Perfluordecalin, Perfluorioddecalin, Perfluortributylamin, Perfluortripropylamin, Perfluor-N,N-dimethylcyclohexylmethylamin, Perfluortrimethyl-bicyclo[3.3.1] nonan, iodiertes Perfluordecalin oder Perfluoriodid umfaßt.

9. Verfahren nach einem der vorhergehenden Ansprüche, **dadurch gekennzeichnet**, daß die physiologisch wirksame Verbindung in einer oder beiden der zwei Koazervatphasen als Partikel in einer Koazervatmatrix und in einem wässrigen Film auf Koazervatbasis gebunden ist; und welches ferner die Schritte des Trennens der Partikel; und des Zugebens eines Hydrokolloids zu den Partikeln in einer Menge von 2 - 20% Gew/Vol., bezogen auf das Gewicht des Koazervatgemisches, zum Bilden eines gelartigen Oberflächenfilms, welcher die physiologisch wirksame Verbindung umgibt, einschließt.

10. Verfahren nach einem der vorhergehenden Ansprüche, **gekennzeichnet durch** Zugeben eines Antioxidationsmittels zu dem Koazervatgemisch während der Herstellung.

11. Verfahren nach einem der vorhergehenden Ansprüche, **dadurch gekennzeichnet**, daß es den Schritt des Zugebens eines physiologisch annehmbaren Alkohols zu dem Koazervatgemisch zum Bilden einer 3-Schichten-Flüssigkeit, welche eine untere polare Schicht, eine mittlere Schicht und eine obere unpolare Schicht umfaßt; das Abtrennen der mittleren Schicht von den anderen beiden Schichten; das Zugeben eines zweiten oberflächenaktiven Mittels zu der abgetrennten mittleren Schicht zum Bilden einer zweiten Zweiphasenflüssigkeit, welche eine untere polare Schicht und eine obere unpolare Schicht umfaßt; und das Zugeben einer physiologisch wirksamen Verbindung zu einer oder beiden Schichten, die aus der zweiten Zweiphasenflüssigkeit gebildet sind, einschließt.

12. Verfahren nach Anspruch 11, **dadurch gekennzeichnet**, daß die zweite Zweiphasenflüssigkeit in eine untere polare Schicht und eine obere unpolare Schicht getrennt wird und eine wasserunlösliche Verbindung zu der oberen unpolaren Schicht zugegeben wird.

13. Verfahren nach Anspruch 11, **dadurch gekennzeichnet**, daß die physiologisch wirksame Verbindung wasserlöslich ist und in der unteren polaren Schicht der zweiten Zweiphasenflüssigkeit enthalten ist.

14. Verfahren nach einem der vorhergehenden Ansprüche, **dadurch gekennzeichnet**, daß die Kernpartikel durch einen Film auf Koazervatbasis eingekapselt sind; und dadurch daß die eingekapselten Partikel mit einer zweiten Koazervatzusammensetzung in Kontakt gebracht werden, um einen zusätzlichen aus Koazervat erhaltenen Film auf den Partikeln zu bilden.

15. Verfahren nach Anspruch 14, **dadurch gekennzeichnet,** daß die zweite Koazervatzusammensetzung 0,1 bis 50 Gew.-% einer zusätzlichen physiologisch wirksamen Verbindung enthält, bezogen auf das Gesamtgewicht der zweiten Koazervatzusammensetzung.

16. Zusammensetzung, die durch ein Verfahren nach einem der vorhergehenden Ansprüche hergestellt ist.

17. Zusammensetzung nach Anspruch 16, **dadurch gekennzeichnet**, daß die physiologisch wirksame Verbindung in einem Liposomen-Kernmaterial enthalten ist, welches in einen wässrigen Film auf Koazervatbasis eingehüllt ist, um zu verhindern, daß sich das Liposom vorzeitig auflöst.

18. Zusammensetzung nach Anspruch 17, **dadurch gekennzeichnet,** daß das Liposom, welches die physiologisch wirksame Verbindung enthält, vor dem Einhüllen des Liposoms in den Film auf Koazervatbasis gebildet wird, und worin der Film auf Koazervatbasis eine wässrige kolloidreiche Phase oder eine wässrige kolloidarme Phase oder eine Kombination aus beiden Phasen umfaßt.

19. Zusammensetzung nach einem der Ansprüche 16 bis 18, **dadurch gekennzeichnet**, daß der Film auf Koazervatbasis eine wässrige kolloidreiche Phase oder eine wässrige kolloidarme Phase oder eine Kombination davon aus dem Koazervatgemisch umfaßt.

20. Zusammensetzung nach einem der Ansprüche 16 bis 19 zum Einbringen in den Blutstrom eines Säugetiers, **dadurch gekennzeichnet**, daß sie ferner Glycerinester, die ausgewählt sind aus der Gruppe bestehend aus einem Monoglycerid, einem Diglycerid, einem Triglycerid und Gemischen davon, umfaßt; wobei die physiologisch wirksame Verbindung in dem Glycerid gelöst oder dispergiert ist.

21. Zusammensetzung nach Anspruch 20, **dadurch gekennzeichnet**, daß das Glycerid ein Mono- oder Diglycerid mit 4 bis 24 Kohlenstoffatomen, vorzugsweise 8 bis 18 Kohlenstoffatomen, ist.

22. Zusammensetzung nach Anspruch 20 oder 21, **dadurch gekennzeichnet**, daß sie eine wirksame Menge eines löslichmachenden Mittels einschließt, welches in der Lage ist, die Löslichkeit der physiologisch wirksamen Verbindung in dem Glycerid zu erhöhen, wobei das Mittel vorzugsweise ein gehärtetes Öl oder ein ungesättigtes Öl ist.

23. Zusammensetzung nach einem der Ansprüche 20 bis 22, **dadurch gekennzeichnet**, daß das Glycerid 1 Gew.-% bis 50 Gew.-% eines gehärteten Öls oder eines ungesättigten Öls, vorzugsweise in einer Menge von 5 Gew.-% bis 15 Gew.-% des Glycerids enthält.

24. Oral einnehmbare Zusammensetzung nach einem der Ansprüche 16 bis 23.

25. Zusammensetzung nach Anspruch 24 zum Erhöhen des Kreislaufvolumens oder zum Einbringen von Eisen und/oder Sauerstoff in das Kreislaufsystem eines Säugetiers, **dadurch gekennzeichnet**, daß die physiologisch wirksame Verbindung ein eisenhaltiges Molekül, welches Sauerstoff transportieren und freisetzen kann, oder ein Sauerstofflösungsmittel umfaßt.

26. Zusammensetzung nach Anspruch 25, **dadurch gekennzeichnet**, daß das eisenhaltige Molekül, welches Sauerstoff transportieren und freisetzen kann, ein Häm, ein Hämoprotein, ein Häm-Hämoprotein-Komplex, pyridoxyliertes, polymerisiertes Hämoglobin, polymerisiertes Hämoglobin oder ein Gemisch davon ist.

27. Zusammensetzung nach Anspruch 24 zum Regeln der Kalorienaufnahme eines Säugetiers, **dadurch gekennzeichnet**, daß die Zusammensetzung eine perhalogenierte Verbindung in einer Menge von 30% Gew./Vol. bis 50% Gew./Vol., bezogen auf das Gesamtgewicht der Zusammensetzung, einschließt.

28. Zusammensetzung nach Anspruch 24 zum Erhöhen des Sauerstoffgehalts in dem Kreislaufsystem eines Säugetiers, **dadurch gekennzeichnet**, daß die physiologisch wirksame Verbindung eine Kombination aus einer perhalogenierten Verbindung und einer Verbindung ist, welche ausgewählt ist aus der Gruppe bestehend aus einem eisenhaltigen Molekül, welches Sauerstoff transportieren und freisetzen kann, und einem Sauerstofflösungsmittel; wobei das wässrige Koazervatsystem eine wässrige kolloidreiche Phase, eine wässrige kolloidarme Phase oder eine Kombination davon umfaßt; worin die Koazervatphase die perhalogenierte Verbindung und entweder ein eisenhaltiges Molekül, welches Sauerstoff transportieren und freisetzen kann oder ein Sauerstofflösungsmittel oder eine Kombination davon einschließt.

29. Zusammensetzung nach Anspruch 28, **dadurch gekennzeichnet**, daß das eisenhaltige Molekül, welches Sauerstoff transportieren und freisetzen kann, ausgewählt ist aus der Gruppe bestehend aus Häm; einem Hämoprotein, einem Häm-Hämoprotein-Komplex; pyridoxyliertem Hämoglobin; pyridoxyliertem polymerisierten Hämoglobin und Gemischen davon.

30. Kontrastmedium zur Einbringung in das Kreislaufsystem eines Säugetiers, umfassend eine oral aufnehmbare oder injizierbare Zusammensetzung nach einem der Ansprüche 16 bis 23.

31. Eine anästhetische Zusammensetzung nach einem der Ansprüche 16 bis 23, **dadurch gekennzeichnet,** daß die Zusammensetzung ein Anästhetikum einschließt, welches der Zusammensetzung bei der Herstellung zugegeben wird.

32. Injizierbare Zusammensetzung, umfassend eine Zusammensetzung nach einem der Ansprüche 16 bis 23.

33. Zusammensetzung nach einem der Ansprüche 16 bis 23 zum Erhöhen des Kreislaufvolumens oder zum Einbringen von Eisen und/oder Sauerstoff in das Kreislaufsystem eines Säugetiers durch Herstellen einer Flüssigkeitsverbindung zwischen dem Kreislaufsystem des Säugetiers und der Zusammensetzung.

## Revendications

1. Procédé de préparation d'une composition utilisable comme système pour introduire et transporter des compositions utiles du point de vue médical dans le corps des mammifères par administration orale, par absorption tissulaire, inhalation ou parentérale, le procédé étant caractérisé en ce qu'il comprend la formation d'un matériau de noyau comprenant des particules de noyau d'un matériau ayant une taille de particules voulue, la formation d'un mélange d'un ou plusieurs agents tensioactifs, d'eau et d'un ou plusieurs composés physiologiquement actifs et l'émulsification du mélange pour produire une composition de coacervat à deux phases comprenant une phase de coacervat et une phase aqueuse d'équilibre, le composé physiologiquement actif étant contenu dans l'une desdites phases ou dans les deux phases, la formation des particules de matériau de noyau avec l'une des phases de coacervat ou avec les deux phases de coacervat ou la mise en contact du matériau de noyau avec l'une des phases de coacervat ou avec les deux phases de coacervat pour introduire le composé physiologiquement actif dans une matrice de matériau de noyau à base de coacervat ou pour encapsuler les particules de matériau de noyau dans un film à base de coacervat d'encapsulation contenant le composé physiologiquement actif.

2. Procédé selon la revendication 1, caractérisé en outre par l'émulsification et la microémulsification du matériau de noyau avec un agent émulsifiant et de l'eau dans des conditions permettant la formation de particules de coacervat dans la matrice utilisables pour une administration orale, ayant une taille de particules inférieure à 10 µm (micromètres), de préférence inférieure à 1 µm (micromètre)

3. Procédé selon la revendication 1 ou la revendication 2, caractérisé en ce que l'agent tensioactif est un phospholipide polymérisé, de préférence une lécithine polymérisée, la céphaline, l'isolécithine, la sphingomyéline, la phosphalidylsérine, l'acide phosphatidique, le phosphatidylinositol, la phosphatidyl choline ou leurs mélanges.

4. Procédé selon l'une quelconque des revendications précédentes, caractérisé en ce que le mélange de coacervat comprend deux agents tensioactifs dont l'un au moins est un polymère ayant une masse moléculaire moyenne en nombre de 300 000 ou moins, les deux agents tensioactifs étant de préférence un phospholipide et une protéine tensioactive, par exemple une lécithine et une albumine polymérisée.

5. Procédé selon l'une quelconque des revendications précédentes, caractérisé en outre par le séchage de la composition pour former une poudre pouvant être reconstituée par addition d'un liquide physiologiquement acceptable.

6. Procédé selon l'une quelconque des revendications précédentes, caractérisé en ce que le ou chaque agent tensioactif est ajouté sous forme monomère et un amorceur de polymérisation est ajouté à ladite composition, ledit amorceur de polymérisation étant capable de, et étant présent en une quantité suffisante pour, polymériser l'agent tensioactif au cours du traitement dudit mélange de coacervat.

7. Procédé selon l'une quelconque des revendications précédentes, caractérisé en ce que le matériau de noyau est formé à partir d'une matrice à base de coacervat et la matrice est encapsulée avec un film à base de coacervat.

8. Procédé selon l'une quelconque des revendications précédentes, caractérisé en ce que le composé physiologiquement actif comprend l'insuline, un peptide atrial ou un composé perhalogéné, par exemple un hydrocarbure perfluoré, un hydrocarbure perfluoroiodé, une amine perfluorée, une amine perfluoroiodée, un éther perfluoré, un éther perfluoroiodé, un complexe hydrocarbure perfluoréiodé, ou leurs mélanges, de préférence la perfluorodécaline, la perfluoroiododécaline, la perfluorotributylamine, la perfluorotripropylamine, la perfluoro-N,N-diméthylcyclohexylméthylamine, le perfluorotriméthyl-bicyclo[3.3.1]nonane, la perfluorodécaline iodée ou le perfluoroiodure.

9. Procédé selon l'une quelconque des revendications précédentes, caractérisé en ce que le composé physiologiquement actif dans l'une des deux phases de coacervat ou dans les deux phases de coacervat est fixé sous forme de particules dans une matrice de coacervat et à l'intérieur d'un film aqueux à base de coacervat, et en ce qu'il comprend en outre les étapes de séparation des particules et d'addition d'un hydrocolloïde aux particules en une quantité de 2 à 20 % en poids/volume par rapport au poids du mélange de coacervat pour former un film superficiel analogue à un gel entourant le composé physiologiquement actif.

10. Procédé selon l'une quelconque des revendications précédentes, caractérisé par l'addition d'un antioxydant au mélange de coacervat pendant la préparation.

11. Procédé selon l'une quelconque des revendications précédentes, caractérisé en ce qu'il comprend l'étape consistant à ajouter un alcool physiologiquement acceptable au mélange de coacervat pour former un liquide à trois couches comprenant une couche polaire inférieure, une couche intermédiaire et une couche non polaire supérieure, séparer la couche intermédiaire des deux autres couches, ajouter un second agent tensioactif à la couche intermédiaire séparée pour former un second liquide à deux phases comprenant une couche polaire inférieure et une couche non polaire supérieure et ajouter un composé physiologiquement actif à l'une desdites couches ou aux deux couches formées à partir dudit second liquide à deux phases.

12. Procédé selon la revendication 11, caractérisé en ce que le second liquide à deux phases est séparé en une couche polaire inférieure et en une couche non polaire supérieure et un composé insoluble dans l'eau est ajouté à la couche non polaire supérieure.

13. Procédé selon la revendication 11, caractérisé en ce que le composé physiologiquement actif est soluble dans l'eau et est contenu dans la couche polaire inférieure dudit second liquide à deux phases.

14. Procédé selon l'une quelconque des revendications précédentes, caractérisé en ce que les particules de noyau sont encapsulées par un film à base de coacervat et en ce que les particules encapsulées sont mises en contact avec une seconde composition de coacervat pour former un film dérivé de coacervat supplémentaire sur les particules.

15. Procédé selon la revendication 14, caractérisé en ce que la seconde composition de coacervat contient 0,1 à 50 % en poids d'un composé physiologiquement actif supplémentaire, par rapport au poids total de la seconde composition de coacervat.

16. Composition préparée par un procédé selon l'une quelconque des revendications précédentes.

17. Composition selon la revendication 16, caractérisée en ce que le composé physiologiquement actif est contenu dans un matériau de noyau liposomique enveloppé à l'intérieur d'un film à base de coacervat aqueux pour empêcher le liposome de se désagréger prématurément.

18. Composition selon la revendication 17, caractérisée en ce que le liposome contenant le composé physiologiquement actif est formé avant l'inclusion du liposome dans le film à base de coacervat, et dans laquelle le film à base de coacervat comprend une phase riche en colloïde aqueux ou une phase pauvre en colloïde aqueux ou une combinaison de ces deux phases.

19. Composition selon l'une quelconque des revendications 16 à 18, caractérisée en ce que le film à base de coacervat comprend une phase riche en colloïde aqueux ou une phase pauvre en colloïde aqueux ou une combinaison de ces phases provenant du mélange de coacervat.

20. Composition selon l'une quelconque des revendications 16 à 19, destinée à être introduite dans le courant sanguin d'un mammifère, caractérisée en ce qu'elle comprend en outre des esters du glycérol choisis dans le groupe formé par un monoglycéride, un diglycéride, un triglycéride et leurs mélanges, ledit composé physiologiquement actif étant dissous ou dispersé dans le glycéride.

21. Composition selon la revendication 20, caractérisée en ce que le glycéride est un mono ou diglycéride ayant 4 à 24 atomes de carbone, de préférence 8 à 18 atomes de carbone.

22. Composition selon la revendication 20 ou 21, caractérisée en ce qu'elle comprend une quantité efficace d'un agent solubilisant capable d'augmenter la solubilité du composé physiologiquement actif dans le glycéride, ledit agent étant de préférence une huile hydrogénée ou une huile insaturée.

23. Composition selon l'une quelconque des revendications 20 à 22, caractérisée en ce que le glycéride contient 1 % en poids à 50 % en poids d'une huile hydrogénée ou d'une huile insaturée, de préférence en une quantité de 5 % en poids à 15 % en poids du glycéride.

24. Composition pouvant être ingérée oralement selon l'une quelconque des revendications 16 à 23.

25. Composition selon la revendication 24, pour augmenter le volume du sang circulant ou introduire du fer et/ou de l'oxygène dans le système circulatoire d'un mammifère, caractérisée en ce que le composé physiologiquement actif comprend une molécule contenant du fer capable de transporter et de libérer de l'oxygène ou un solvant de l'oxygène.

26. Composition selon la revendication 25, caractérisée en ce que la molécule contenant du fer capable de transporter et de libérer de l'oxygène est un hème, une hémoprotéine, un complexe hème-hémoprotéine, une hémoglobine polymérisée pyridoxylée, une hémoglobine polymérisée ou un de leurs mélanges.

27. Composition selon la revendication 24, pour contrôler l'apport calorique d'un mammifère, caractérisée en ce que la composition comprend un composé perhalogéné en une quantité de 30 % en poids/volume à 50 % en poids/volume par rapport au poids total de la composition.

28. Composition selon la revendication 24, pour augmenter la teneur en oxygène du système circulatoire d'un mammifère, caractérisée en ce que le composé physiologiquement actif est une combinaison d'un composé perhalogéné et d'un composé choisi dans le groupe formé par une molécule contenant du fer capable de transporter et de libérer de l'oxygène et un solvant de l'oxygène, ledit système de coacervat aqueux comprenant une phase riche en colloïde aqueux, une phase pauvre en colloïde aqueux ou une combinaison de celles-ci, dans laquelle la phase de coacervat comprend le composé perhalogéné et une molécule contenant du fer capable de transporter et de libérer de l'oxygène ou un solvant de l'oxygène, ou une combinaison de ceux-ci.

29. Composition selon la revendication 28, caractérisée en ce que la molécule contenant du fer capable de transporter et de libérer de l'oxygène est choisie dans le groupe formé par un hème, une hémoprotéine, un complexe hème-hémoprotéine, une hémoglobine pyridoxylée, une hémoglobine pyridoxylée polymérisée et leurs mélanges.

30. Produit de contraste destiné à être introduit dans le système circulatoire d'un mammifère, comprenant une composition pouvant être ingérée oralement ou injectable selon l'une quelconque des revendications 16 à 23.

31. Composition anesthésique selon l'une quelconque des revendications 16 à 23, caractérisée en ce que la composition comprend un anesthésique ajouté à la composition pendant sa préparation.

32. Composition injectable comprenant une composition selon l'une quelconque des revendications 16 à 23.

33. Composition selon l'une quelconque des revendications 16 à 23, pour augmenter le volume du sang circulant ou pour introduire du fer et/ou de l'oxygène dans le système circulatoire d'un mammifère par établissement d'une communication par fluide entre le système circulatoire du mammifère et la composition.
